# EUROPEAN PATENT SPECIFICATION

(11) **EP 4 263 502 B1**
(45) Date of publication and mention of the grant of the patent: **25.12.2024**
(21) Application number: 21839626.5
(22) Date of filing: 20.12.2021
(51) Int. Cl.: C07D 207/267, C07C 237/52, C07D 401/12, A61P 31/14, A61K 31/4015, A61K 31/4439, A61K 31/165, A61K 38/00, C07K 5/02

(54) **SARS-COV-2 MPRO INHIBITOR COMPOUNDS**
SARS-COV-2-MPRO-INHIBITORVERBINDUNGEN
COMPOSÉS INHIBITEURS DE MPRO DU SARS-COV-2

(30) Priority: 18.12.2020 GB 202020190; 24.05.2021 GB 202107385
(43) Date of publication of application: 25.10.2023
(73) Proprietor: Nxera Pharma UK Limited, Cambridge, Cambridgeshire CB21 6DG (GB)
(72) Inventor: CONGREVE, Miles Stuart, Cambridge Cambridgeshire CB21 6DG (GB); CHRISTOPHER, John Andrew, Cambridge Cambridgeshire CB21 6DG (GB); PICKWORTH, Mark, Cambridge Cambridgeshire CB21 6DG (GB); DE GRAAF, Chris, Cambridge Cambridgeshire CB21 6DG (GB); HIGUERUELO, Alicia Perez, Cambridge Cambridgeshire CB21 6DG (GB); MASON, Jonathan Stephen, Cambridge Cambridgeshire CB21 6DG (GB); KULKARNI, Santosh S., Bangalore 560 099 (IN)
(74) Representative: Gill Jennings & Every LLP
(86) International application number: PCT/GB2021/053374
(87) International publication number: WO 2022/129953

(56) References cited:
- WO-A1-2004/093860
- WO-A1-2005/113580
- WO-A1-2006/042478
- WO-A1-2020/030143
- WO-A2-2006/061714
- WO-A2-2018/042343
- WO-A2-99/24460
- US-A- 5 763 576
- ZHANG LINLIN ET AL: "[alpha]-Ketoamides as Broad-Spectrum Inhibitors of Coronavirus and Enterovirus Replication: Structure-Based Design, Synthesis, and Activity Assessment", JOURNAL OF MEDICINAL CHEMISTRY, vol. 63, no. 9, 11 February 2020 (2020-02-11), US, pages 4562 - 4578, XP055806728, ISSN: 0022-2623, Retrieved from the Internet <URL:https://pubs.acs.org/doi/pdf/10.1021/acs.jmedchem.9b01828> DOI: 10.1021/acs.jmedchem.9b01828
- NIE QUANDENG ET AL: "3D-quantitative structure-activity relationship study for the design of novel enterovirus A71 3C protease inhibitors", CHEMICAL BIOLOGY & DRUG DESIGN, vol. 92, no. 4, 26 June 2018 (2018-06-26), Hoboken, USA, pages 1750 - 1762, XP055832524, ISSN: 1747-0277, Retrieved from the Internet <URL:https://api.wiley.com/onlinelibrary/tdm/v1/articles/10.1111%2Fcbdd.13344> DOI: 10.1111/cbdd.13344
- PACIFICO SALVATORE ET AL: "Synthesis and Biological Activity of Peptide [alpha]-Ketoamide Derivatives as Proteasome Inhibitors", ACS MEDICINAL CHEMISTRY LETTERS, vol. 10, no. 7, 11 July 2019 (2019-07-11), US, pages 1086 - 1092, XP055886635, ISSN: 1948-5875, Retrieved from the Internet <URL:https://pubs.acs.org/doi/pdf/10.1021/acsmedchemlett.9b00233> DOI: 10.1021/acsmedchemlett.9b00233
- GRUM-TOKARS ET AL: "Evaluating the 3C-like protease activity of SARS-Coronavirus: Recommendations for standardized assays for drug discovery", VIRUS RESEARCH, AMSTERDAM, NL, vol. 133, no. 1, 11 March 2008 (2008-03-11), pages 63 - 73, XP022520313, ISSN: 0168-1702, DOI: 10.1016/J.VIRUSRES.2007.02.015
- ZHOU QIONGQIONG ANGELA ET AL: "Potential Therapeutic Agents and Associated Bioassay Data for COVID-19 and Related Human Coronavirus Infections", ACS PHARMACOLOGY & TRANSLATIONAL SCIENCE, vol. 3, no. 5, 9 October 2020 (2020-10-09), pages 813 - 834, XP055874568, ISSN: 2575-9108, Retrieved from the Internet <URL:https://pubs.acs.org/doi/pdf/10.1021/acsptsci.0c00074> DOI: 10.1021/acsptsci.0c00074
- WANG YAXIN ET AL: "Inhibition of enterovirus 71 replication by an [alpha]-hydroxy-nitrile derivative NK-1.9k", ANTIVIRAL RESEARCH, ELSEVIER BV, NL, vol. 141, 5 January 2017 (2017-01-05), pages 91 - 100, XP029949620, ISSN: 0166-3542, DOI: 10.1016/J.ANTIVIRAL.2017.01.002
- ZHANG LINLIN ET AL: "-Ketoamides as Broad-Spectrum Inhibitors of Coronavirus and Enterovirus Replication: Structure-Based Design, Synthesis, and Activity Assessment", JOURNAL OF MEDICINAL CHEMISTRY, vol. 63, no. 9, 11 February 2020 (2020-02-11), US, pages 4562 - 4578, XP055806728, ISSN: 0022-2623, Retrieved from the Internet <URL:https://pubs.acs.org/doi/pdf/10.1021/acs.jmedchem.9b01828> DOI: 10.1021/acs.jmedchem.9b01828
- FU LIFENG ET AL: "Both Boceprevir and GC376 efficaciously inhibit SARS-CoV-2 by targeting its main protease", NATURE COMMUNICATIONS, vol. 11, no. 1, 1 December 2020 (2020-12-01), XP055886766, Retrieved from the Internet <URL:https://www.nature.com/articles/s41467-020-18233-x.pdf> DOI: 10.1038/s41467-020-18233-x
- THANIGAIMALAI PILLAIYAR ET AL: "Design, synthesis, and biological evaluation of novel dipeptide-type SARS-CoV 3CL protease inhibitors: Structure-activity relationship s", EUROPEAN JOURNAL OF MEDICINAL CHEMISTRY, ELSEVIER, AMSTERDAM, NL, vol. 65, 20 May 2013 (2013-05-20), pages 436 - 447, XP028677373, ISSN: 0223-5234, DOI: 10.1016/J.EJMECH.2013.05.005
- ZHANG LINLIN ET AL: "Crystal structure of SARS-CoV-2 main protease provides a basis for design of improved -ketoamide inhibitors", SCIENCE, vol. 368, no. 6489, 20 March 2020 (2020-03-20), US, pages 409 - 412, XP055814007, ISSN: 0036-8075, Retrieved from the Internet <URL:https://science.sciencemag.org/content/sci/368/6489/409.full.pdf> DOI: 10.1126/science.abb3405
- JOSHI RAKESH S. ET AL: "Discovery of potential multi-target-directed ligands by targeting host-specific SARS-CoV-2 structurally conserved main protease", JOURNAL OF BIOMOLECULAR STRUCTURE & DYNAMICS, 24 April 2020 (2020-04-24), US, pages 1 - 16, XP055874570, ISSN: 0739-1102, Retrieved from the Internet <URL:https://www.ncbi.nlm.nih.gov/pmc/articles/PMC7212545/pdf/TBSD_A_1760137.pdf> DOI: 10.1080/07391102.2020.1760137

## Description

This application relates to novel compounds and their use as SARS-CoV-2 Main Protease (Mpro) inhibitors. Compounds described herein may be useful in the treatment of SARS-CoV-2 and related viruses and disorders associated with SARS-CoV-2: Mpro. The application is also directed to pharmaceutical compositions comprising these compounds and the manufacture and use of these compounds and compositions in the treatment of SARS-CoV-2 and related viruses and disorders associated with SARS-CoV-2: Mpro. The compounds and compositions may be useful in preventing death or complications arising due to chronic underlying conditions or comorbidities in patients infected with SARS-CoV-2 and related viruses.

### BACKGROUND OF THE INVENTION

Coronaviruses have long existed in nature and have made zoonotic transmission to humans, generally causing mild respiratory illnesses such as the common cold upon infection. However, in the last two decades outbreaks of novel human coronavirus infections that cause severe respiratory illness have presented a major global health concern. This includes the severe acute respiratory syndrome coronavirus (SARS-CoV) outbreak in 2002-2004, the Middle East respiratory syndrome coronavirus (MERS-CoV) outbreak in 2012-2015 and severe acute respiratory syndrome coronavirus 2 (SARS-CoV-2), the most recently emerged strain of coronavirus, that was identified in Wuhan, China, in 2019 and is the aetiological agent responsible for the 2019-2020 viral pneumonia outbreak of coronavirus disease 2019 (COVID-19). Despite the tragic and widespread effects of these sudden occurrences and the periodic emergence of novel human coronaviruses increasing the potential for future outbreaks, we do not yet have validated antiviral treatments targeting coronavirus infections.

SARS-CoV-2 packages a large RNA genome of ~30kb, two-thirds of which encodes for the two polyproteins pp1a and pp1b (Hegyi et al. Journal of General Virology 83 (3): 595-99). These polyproteins are processed into 16 non-structural proteins (nsps) that are liberated from the long polypeptide chains by two viral cysteine proteases, the papain-like protease (nsp3) and the 3C-like protease (nsp5). The latter species, also referred to as the main protease (Mpro), cleaves the viral polyproteins at eleven sites to generate twelve non-structural proteins (nsp5-16). Included in these nsps are those involved in the replication and transcription machinery such as the the RNA-dependent RNA polymerase (nsp12) and helicase (nsp13). The essential role Mpro plays in viral replication has been demonstrated in mutagenesis experiments (Kim et al. Virology 208 (1): 1-8; Stobart et al. Journal of Virology 86 (9): 4801-10), which makes it an attractive target for the design of inhibitors to treat coronavirus infection.

Furthermore, there are no human proteases with similar cleavage specificity and therefore selective inhibitors of Mpro are highly likely to be non-toxic (Anand et al. 2003. Science 300 (5626): 1763-67).

The use of protease inhibitors for the treatment of viral diseases is well precedented (Bacon et al. The New England Journal of Medicine 364 (13): 1207-17) and the similarity of the SARS-CoV-2 Mpro active site to other viral proteases has driven efforts to identify clinically approved drugs that could be repurposed for the treatment of COVID-19 (Riva et al. Nature, 586: 113-119). Screening of a selection of 18 viral protease inhibitors designed for the treatment of human immunodeficiency virus (HIV) and Hepatitis C virus (HCV) identified the anti-HCV drug boceprevir and the pre-clinical inhibitor against feline infectious peritonitis virus (FIPV) GC376 as inhibitors of SARS-CoV-2 Mpro (Fu et al. Nature Communications 11 (1): 4417). While GC376 showed a more potent inhibition efficacy of recombinant protease activity (IC₅₀ = 0.15 µM) than boceprevir (IC₅₀ = 8 µM), GC376 has shown side effects in trials performed in cats raising potential safety concerns (Pedersen et al. Journal of Feline Medicine and Surgery 20 (4): 378-92). Boceprevir was also identified as an inhibitor of SARS-CoV-2 Mpro alongside telaprevir in a different study, albeit both drugs inhibited SARS-CoV-2 Mpro with IC₅₀ values of >1 µM) (Anson et al. 2020. doi:10.21203/rs.3.rs-26344/v1). In addition to SARS-CoV-2 Mpro, the inhibitory efficacy of boceprevir and telaprevir was also assessed at Mpro proteases from eight other coronaviruses including SARS, MERS, HKU1, HKU4, HKU5, NL63, FIPV and IBV. Within this selection boceprevir was able to inhibit all coronavirus proteases tested except NL63 and a similarly broad spectrum of activity was shown for telaprevir with inhibitory activity shown at SARS, HKU4, HKU5, NL63 and IBV. While the antiviral activity of these drugs at SARS-CoV-2 Mpro is not sufficient for clinical development, their ability to inhibit a broad range of proteases highlights the potential for the design of broad-spectrum antiviral drugs able to treat not only SARS-CoV-2 infection but also other human coronaviruses and potentially novel coronaviruses that could emerge in the future.

The sequence similarly between SARS-CoV and SARS-CoV-2 Mpro active sites was also exploited in the identification of the SARS-CoV-2 Mpro inhibitor PF-07304814, a phosphate prodrug of PF-00835231 which was originally designed for the treatment of SARS-CoV (Boras et al. BioRxiv, 2020.09.12.293498). PF-00835231 inhibited SARS-CoV-2 Mpro with a Kᵢ of 0.27 nM and displayed broad inhibitory activity against ten further coronavirus strains with Kᵢ values of 0.03-4 nM. This translated into ~1 µM activity in cell-based live virus assays. The activity of PF-00835231 in combination with remdesivir, a nucleoside RNA-dependent RNA polymerase inhibitor, was also evaluated as antiviral agents that target different aspects of the viral replication process can yield synergistic effects in combination. Indeed, PF-00835231 and remdesivir displayed either synergistic or additive effects in a cell-based antiviral assay, which suggests that the combination of Mpro inhibitors with antivirals with other modes of actions could show clinical benefit.

In 2020 the crystal structure of SARS-CoV-2 Mpro in complex with N3 (a Michael acceptor inhibitor) was published (Jin et al. Nature 582 (7811): 289-93), thereby enabling virtual screening and structure-based drug design (SBDD) for inhibitors of SARS-CoV-2 Mpro. Such SBDD efforts included the design of peptidomimetic α-ketoamides as broad-spectrum inhibitors of coronaviruses and enteroviruses with the two most promising inhibitors showing 0.71-12.27 µM IC₅₀ values in recombinant inhibition assays for proteases from enteroviruses EV-A71 and CVB3 as well as coronaviruses SARS-CoV and NL63 (Zhang et al. 2020. Journal of Medicinal Chemistry 63 (9): 4562-4578). The activity observed in the recombinant protease assays broadly matched antiviral activity in cell-based live virus assays with IC₅₀ values within 10-fold in both systems, suggesting that good activity in the protease inhibition assay is a good indicator of antiviral activity.

Currently, there are no targeted therapeutic agents for the treatment of COVID-19, and effective treatment options remain very limited. Despite much ongoing research activity and numerous clinical trials in progress, only remdesivir and favipiravir have been approved in selected countries for limited use to treat SARS-CoV-2 infection but show only modest effects (Zhou et al. ACS Pharmacology & Translational Science 3 (5): 813-834). There exists a need for targeted therapeutic agents for the treatment of SARS-CoV-2 infection and for the reasons outlined above SARS-CoV-2 Mpro represents an attractive drug target for SARS-CoV-2. The compounds disclosed herein are shown to be inhibitors of SARS-CoV-2 Mpro and therefore represent potential candidates for the treatment of coronavirus infection and associated disorders including but not limited to COVID-19.

WO 2020/030143 describes ketoamide compounds and their use in inhibiting coronavirus or Ebola virus, and in treating or preventing related conditions.

Zhang Linlin et al. Journal of Medicinal Chemistry, 11 Feb 2020, Vol. 63, No. 9, Pages 4562-4578 describes various α-Ketoamides as Broad-Spectrum Inhibitors of Coronavirus and Enterovirus Replication.

WO 99/24460 describes N-terminal substituted dipeptide nitrites and their use as inhibitors of cysteine cathepsins.

### THE INVENTION

The present invention provides compounds having activity as SARS-CoV-2: Mpro inhibitors.

Provided is a compound of Formula (1'): 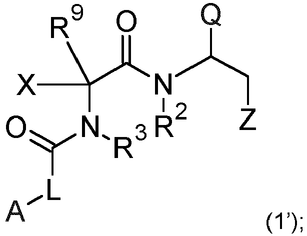 or a salt thereof, wherein;
A is selected from: 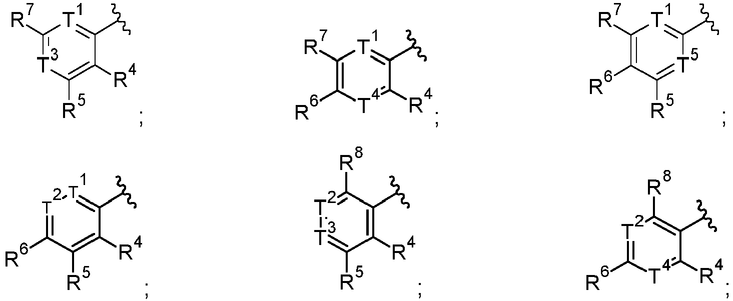
Q is CN or a group of formula: 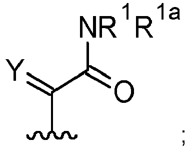
X is a C₁₋₆ saturated or unsaturated hydrocarbon group optionally substituted with 1 to 6 fluorine atoms or X is joined with R⁹ to form a C₃₋₆ cycloalkyl ring which is optionally substituted with 1 to 3 fluorine atoms or 1 to 3 C₁₋₃ alkyl groups;
Y is O or NOR¹⁶;
T¹ is CR⁸ or N;
T² is CR⁷ or N;
T³ is CR⁶ or N;
T⁴ is CR⁵ or N;
T⁵ is CR⁴ or N;
Z is a 5- or 6-membered heterocyclic ring optionally substituted with oxo or 1 to 6 fluorine atoms, or Z is C₃₋₆ cycloalkyl optionally substituted with 1 to 6 fluorine atoms, or Z is - (CH₂)ₚCONHR¹³, or Z is -(CH₂)ₚCO₂R¹³;
L is -CR¹¹=CR¹²-, -CHR¹¹-CHR¹²- or -O-CHR¹¹-;
R¹ and R^{1a} are independently H or a C₁₋₆ saturated hydrocarbon group optionally substituted with 1 to 6 fluorine atoms, or R¹ and R^{1a} are linked together to form a 3 to 6-membered saturated ring optionally containing an additional heteroatom;
R² and R³ are independently H or C₁₋₃ alkyl optionally substituted with 1 to 6 fluorine atoms; R⁴, R⁵, R⁶, R⁷ and R⁸ are independently H, halo, CN, CO₂R¹⁴, OR¹⁴, SO₂R¹⁴, SONHR¹⁴, OSO₂R¹⁴, PO(R¹⁴)₂, SF₅ or C₁₋₃ alkyl optionally substituted with 1 to 6 fluorine atoms;
R⁹ is H or is joined with X to form a C₃₋₆ cycloalkyl ring which is optionally substituted with 1 to 3 fluorine atoms or 1 to 3 C₁₋₃ alkyl groups;
R¹¹ and R¹² are independently H, -(CH₂)ₘCO₂R¹⁵ or C₁₋₃ alkyl optionally substituted with 1 to 6 fluorine atoms; or R¹¹ and R¹² are joined to form a cyclopropyl ring;
R¹³, R¹⁴ and R¹⁵ are independently H or C₁₋₃ alkyl optionally substituted with 1 to 6 fluorine atoms;
R¹⁶ is H or C₁₋₃ alkyl optionally substituted with 1 to 6 fluorine atoms;
p and m are independently 0-3;
wherein when R¹ and R^{1a} are not both H, L is:

   -CHR¹¹-CHR¹²-;

   -CR¹¹=CR¹²- where R³, R⁴, R⁵, R⁶, R⁷ and R⁸ are not all H;
   or -O-CHR¹¹- where R³ is not H, or where two or more of R⁴, R⁵, R⁶, R⁷ and R⁸ are not H.

Compounds of the present invention may be used as SARS-CoV-2: Mpro inhibitors. Compounds of the present invention may be used in the treatment of SARS-CoV-2 and related viruses or a disease or disorder associated with SARS-CoV-2: Mpro. Compounds of the present invention may be useful in preventing death or complications arising due to chronic underlying conditions or comorbidities in patients infected with SARS-CoV-2 and related viruses. Such chronic underlying conditions or comorbidities may include for example hypertension, obesity, chronic lung conditions (TB, asthma and cystic fibrosis), diabetes and cardiovascular conditions (coronary heart disease, congenital heart disease and heart failure). Compounds of the present invention may be used in the manufacture of medicaments. The compounds or medicaments may be for use in treating, preventing, ameliorating, controlling or reducing the risk of SARS-CoV-2 and related viruses and diseases or disorders in which SARS-CoV-2: Mpro is involved. The compounds or medicaments may be for use in treating, preventing, ameliorating, controlling or reducing the risk of chronic underlying conditions or comorbidities in patients infected with SARS-CoV-2 and related viruses.

Compounds of the present invention may be for use as a single agent or in combination with one or more additional pharmaceutical agents. Compounds of the present invention may be useful in the treatment of SARS-CoV-2 and related viruses or conditions or symptoms related thereto.

### DETAILED DESCRIPTION OF THE INVENTION

The invention relates to novel compounds. The invention also relates to the use of novel compounds as inhibitors of SARS-CoV-2: Mpro. The invention further relates to the use of novel compounds in the manufacture of medicaments for use as SARS-CoV-2: Mpro inhibitors. The invention further relates to compounds, compositions and medicaments that may be useful in the treatment of SARS-CoV-2 and related viruses or conditions or symptoms related thereto.

Provided is a compound of Formula (1'): 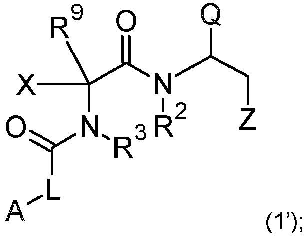 or a salt thereof, wherein;
A is selected from: 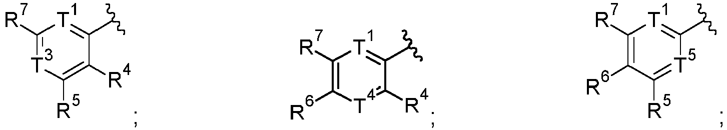 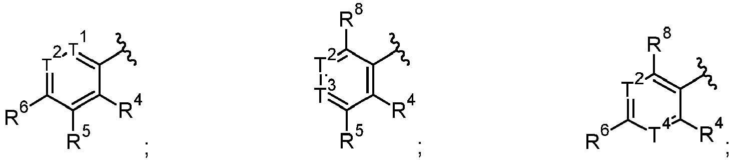
Q is CN or a group of formula: 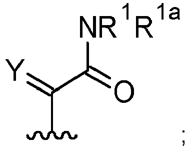
X is a C₁₋₆ saturated or unsaturated hydrocarbon group optionally substituted with 1 to 6 fluorine atoms or X is joined with R⁹ to form a C₃₋₆ cycloalkyl ring which is optionally substituted with 1 to 3 fluorine atoms or 1 to 3 C₁₋₃ alkyl groups;
Y is O or NOR¹⁶;
T¹ is CR⁸ or N;
T² is CR⁷ or N;
T³ is CR⁶ or N;
T⁴ is CR⁵ or N;
T⁵ is CR⁴ or N;
Z is a 5- or 6-membered heterocyclic ring optionally substituted with oxo or 1 to 6 fluorine atoms, or Z is C₃₋₆ cycloalkyl optionally substituted with 1 to 6 fluorine atoms, or Z is - (CH₂)ₚCONHR¹³, or Z is -(CH₂)ₚCO₂R¹³;
L is -CR¹¹=CR¹²-, -CHR¹¹-CHR¹²- or -O-CHR¹¹-;
R¹ and R^{1a} are independently H or a C₁₋₆ saturated hydrocarbon group optionally substituted with 1 to 6 fluorine atoms, or R¹ and R^{1a} are linked together to form a 3 to 6-membered saturated ring optionally containing an additional heteroatom;
R² and R³ are independently H or C₁₋₃ alkyl optionally substituted with 1 to 6 fluorine atoms;
R⁴, R⁵, R⁶, R⁷ and R⁸ are independently H, halo, CN, CO₂R¹⁴, OR¹⁴, SO₂R¹⁴, SONHR¹⁴, OSO₂R¹⁴, PO(R¹⁴)₂, SF₅ or C₁₋₃ alkyl optionally substituted with 1 to 6 fluorine atoms;
R⁹ is H or is joined with X to form a C₃₋₆ cycloalkyl ring which is optionally substituted with 1 to 3 fluorine atoms or 1 to 3 C₁₋₃ alkyl groups;
R¹¹ and R¹² are independently H, -(CH₂)ₘCO₂R¹⁵ or C₁₋₃ alkyl optionally substituted with 1 to 6 fluorine atoms; or R¹¹ and R¹² are joined to form a cyclopropyl ring;
R¹³, R¹⁴ and R¹⁵ are independently H or C₁₋₃ alkyl optionally substituted with 1 to 6 fluorine atoms;
R¹⁶ is H or C₁₋₃ alkyl optionally substituted with 1 to 6 fluorine atoms;
p and m are independently 0-3;
wherein when R¹ and R^{1a} are not both H, L is:

   -CHR¹¹-CHR¹²-;

   -CR¹¹=CR¹²- where R³, R⁴, R⁵, R⁶, R⁷ and R⁸ are not all H;
   or -O-CHR¹¹- where R³ is not H, or where two or more of R⁴, R⁵, R⁶, R⁷ and R⁸ are not H.

Also provided is a compound of Formula (1b): 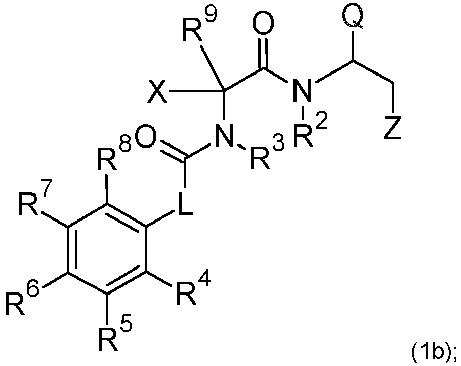 or a salt thereof, wherein;
Q is CN or a group of formula: 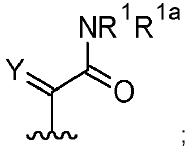
X is a C₁₋₆ saturated or unsaturated hydrocarbon group optionally substituted with 1 to 6 fluorine atoms or X is joined with R⁹ to form a C₃₋₆ cycloalkyl ring which is optionally substituted with 1 to 3 fluorine atoms or 1 to 3 C₁₋₃ alkyl groups;
Y is O or NOR¹⁶;
Z is a 5- or 6-membered heterocyclic ring optionally substituted with oxo or 1 to 6 fluorine atoms, or Z is C₃₋₆ cycloalkyl optionally substituted with 1 to 6 fluorine atoms, or Z is - (CH₂)ₚCONHR¹³, or Z is -(CH₂)ₚCO₂R¹³;
L is -CR¹¹=CR¹²-, -CHR¹¹-CHR¹²- or -O-CHR¹¹-;
R¹ and R^{1a} are independently H or a C₁₋₆ saturated hydrocarbon group optionally substituted with 1 to 6 fluorine atoms, or R¹ and R^{1a} are linked together to form a 3 to 6-membered saturated ring optionally containing an additional heteroatom;
R² and R³ are independently H or C₁₋₃ alkyl optionally substituted with 1 to 6 fluorine atoms;
R⁴, R⁵, R⁶, R⁷ and R⁸ are independently H, halo, CO₂R¹⁴ or C₁₋₃ alkyl optionally substituted with 1 to 6 fluorine atoms;
R⁹ is H or is joined with X to form a C₃₋₆ cycloalkyl ring which is optionally substituted with 1 to 3 fluorine atoms or 1 to 3 C₁₋₃ alkyl groups;
R¹¹ and R¹² are independently H, -(CH₂)ₘCO₂R¹⁵ or C₁₋₃ alkyl optionally substituted with 1 to 6 fluorine atoms;
R¹³, R¹⁴ and R¹⁵ are independently H or C₁₋₃ alkyl optionally substituted with 1 to 6 fluorine atoms;
R¹⁶ is H or C₁₋₃ alkyl optionally substituted with 1 to 6 fluorine atoms;
p and m are independently 0-3;
wherein when R¹ and R^{1a} are not both H, L is:

   -CHR¹¹-CHR¹²-;

   -CR¹¹=CR¹²- where R³, R⁴, R⁵, R⁶, R⁷ and R⁸ are not all H;
   or -O-CHR¹¹- where R³ is not H, or where two or more of R⁴, R⁵, R⁶, R⁷ and R⁸ are not H.

Also provided is a compound of Formula (1c): 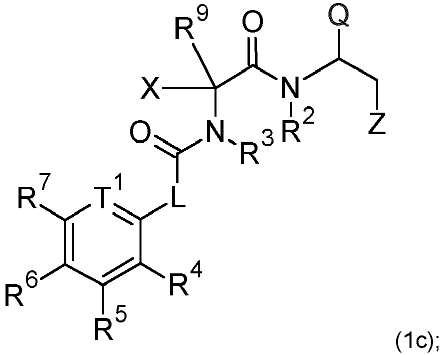 or a salt thereof, wherein Z, Q, X, L, T¹, R², R³, R⁴, R⁵, R⁶, R⁷ and R⁹ are as defined herein.

Also provided is a compound of Formula (1): 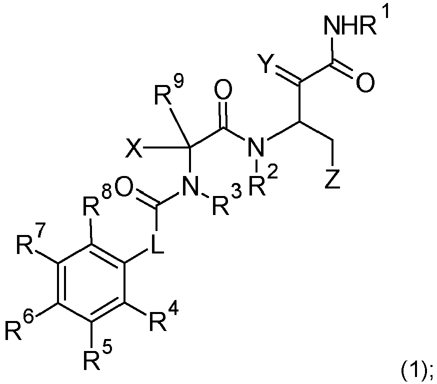 or a salt thereof, wherein;
X is a C₁₋₆ saturated or unsaturated hydrocarbon group optionally substituted with 1 to 6 fluorine atoms or X is joined with R⁹ to form a C₃₋₆ cycloalkyl ring which is optionally substituted with 1 to 3 fluorine atoms or 1 to 3 C₁₋₃ alkyl groups;
Y is O or NOR¹⁶;
Z is a 5- or 6-membered heterocyclic ring optionally substituted with oxo or 1 to 6 fluorine atoms, or Z is C₃₋₆ cycloalkyl optionally substituted with 1 to 6 fluorine atoms, or Z is - (CH₂)ₚCONHR¹³, or Z is -(CH₂)ₚCO₂R¹³;
L is -CR¹¹=CR¹²-, -CHR¹¹-CHR¹²- or -O-CHR¹¹-;
R¹ is H or a C₁₋₆ saturated hydrocarbon group optionally substituted with 1 to 6 fluorine atoms; R² and R³ are independently H or C₁₋₃ alkyl optionally substituted with 1 to 6 fluorine atoms; R⁴, R⁵, R⁶, R⁷ and R⁸ are independently H, halo, CO₂R¹⁴ or C₁₋₃ alkyl optionally substituted with 1 to 6 fluorine atoms;
R⁹ is H or is joined with X to form a C₃₋₆ cycloalkyl ring which is optionally substituted with 1 to 3 fluorine atoms or 1 to 3 C₁₋₃ alkyl groups;
R¹¹ and R¹² are independently H, -(CH₂)ₘCO₂R¹⁵ or C₁₋₃ alkyl optionally substituted with 1 to 6 fluorine atoms;
R¹³, R¹⁴ and R¹⁵ are independently H or C₁₋₃ alkyl optionally substituted with 1 to 6 fluorine atoms;
R¹⁶ is H or C₁₋₃ alkyl optionally substituted with 1 to 6 fluorine atoms;
p and m are independently 0-3;
wherein when R¹ is a C₁₋₆ saturated hydrocarbon group optionally substituted with 1 to 6 fluorine atoms, L is:

   -CHR¹¹-CHR¹²-;

   -CR¹¹=CR¹²- where R³, R⁴, R⁵, R⁶, R⁷ and R⁸ are not all H;
   or -O-CHR¹¹- where R³ is not H, or where two or more of R⁴, R⁵, R⁶, R⁷ and R⁸ are not H.

Provided is a compound of Formula (1i): 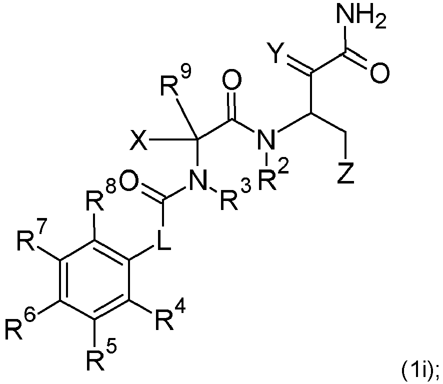 or a salt thereof, wherein;
X is a C₁₋₈ saturated or unsaturated hydrocarbon group optionally substituted with 1 to 6 fluorine atoms or X is joined with R⁹ to form a C₃₋₆ cycloalkyl ring which is optionally substituted with 1 to 3 fluorine atoms or 1 to 3 C₁₋₃ alkyl groups;
Y is O or NOR¹⁶;
Z is a 5- or 6-membered heterocyclic ring optionally substituted with oxo or 1 to 6 fluorine atoms, or Z is C₃₋₆ cycloalkyl optionally substituted with 1 to 6 fluorine atoms, or Z is - (CH₂)ₚCONHR¹³, or Z is -(CH₂)ₚCO₂R¹³;
L is -CR¹¹=CR¹²-, -CHR¹¹-CHR¹²- or -O-CHR¹¹-;
R² and R³ are independently H or C₁₋₃ alkyl optionally substituted with 1 to 6 fluorine atoms;
R⁴, R⁵, R⁶, R⁷ and R⁸ are independently H, halo, CO₂R¹⁴ or C₁₋₃ alkyl optionally substituted with 1 to 6 fluorine atoms;
R⁹ is H or is joined with X to form a C₃₋₆ cycloalkyl ring which is optionally substituted with 1 to 3 fluorine atoms or 1 to 3 C₁₋₃ alkyl groups;
R¹¹ and R¹² are independently H, -(CH₂)ₘCO₂R¹⁵ or C₁₋₃ alkyl optionally substituted with 1 to 6 fluorine atoms;
R¹³, R¹⁴ and R¹⁵ are independently H or C₁₋₃ alkyl optionally substituted with 1 to 6 fluorine atoms;
R¹⁶ is H or C₁₋₃ alkyl optionally substituted with 1 to 6 fluorine atoms;
p and m are independently 0-3.

Also provided are compounds of Formula (1'a), (1'ai) and (1'aii): 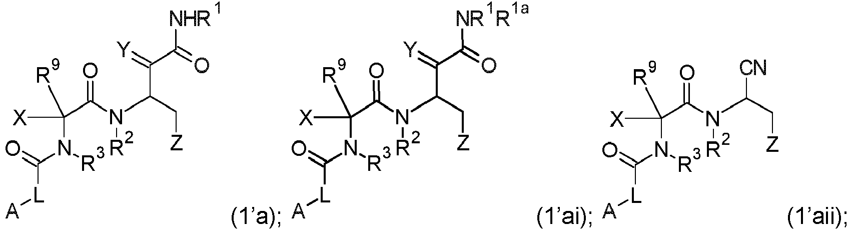 and salts thereof, wherein A, X, Z, Y, L, R¹, R^{1a}, R², R³ and R⁹ are as defined herein.

Also provided are compounds of Formula (1'aa), (1'aai) and (1'aaii): 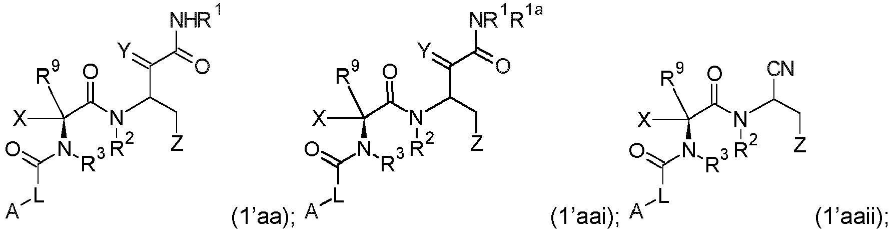 and salts thereof, wherein A, X, Z, Y, L, R¹, R^{1a}, R², R³ and R⁹ are as defined herein.

Also provided are compounds of Formula (1ca), (1cai) and (1caii): 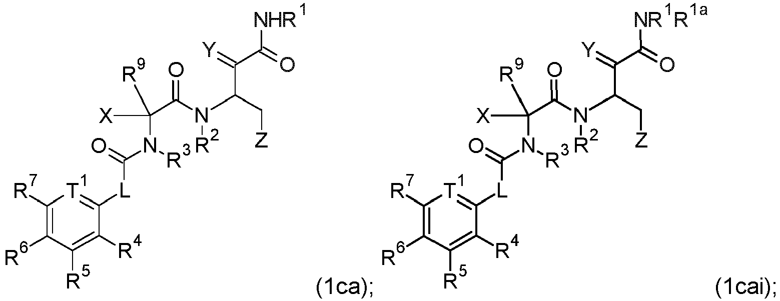 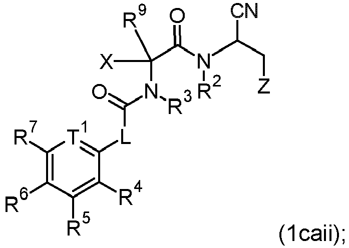 and salts thereof, wherein X, Z, Y, L, T¹, R¹, R^{1a}, R², R³, R⁴, R⁵, R⁶, R⁷ and R⁹ are as defined herein.

Also provided are compounds of Formula (1caa), (1caai) and (1caaii): 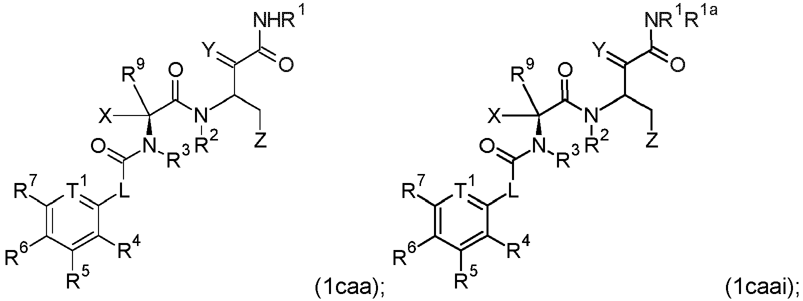 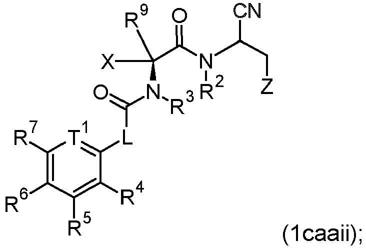 and salts thereof, wherein X, Z, Y, L, T¹, R¹, R^{1a}, R², R³, R⁴, R⁵, R⁶, R⁷ and R⁹ are as defined herein.

Also provided are compounds of Formula (1a), (1ai) and (1aii): 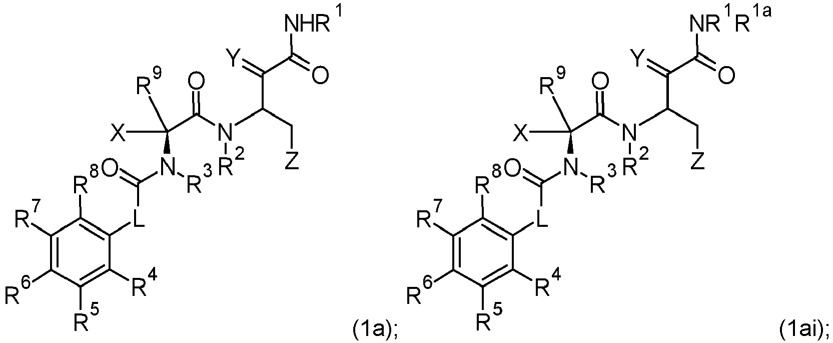 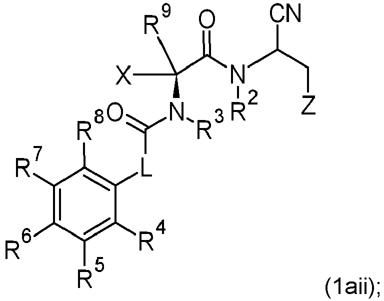 and salts thereof, wherein X, Z, Y, L, R¹, R^{1a}, R², R³, R⁴, R⁵, R⁶, R⁷, R⁸ and R⁹ are as defined herein.

Also provided are compounds of Formula (2a), (2ai) and (2aii): 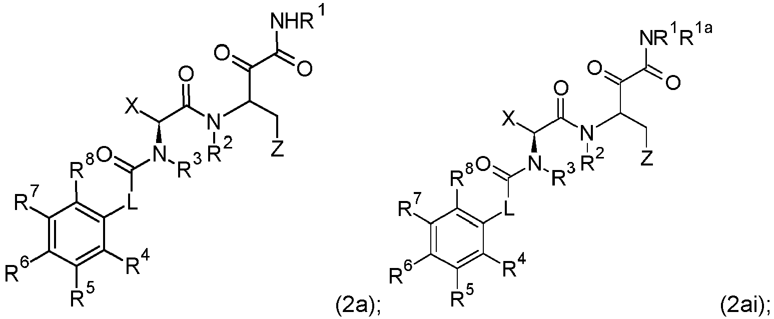 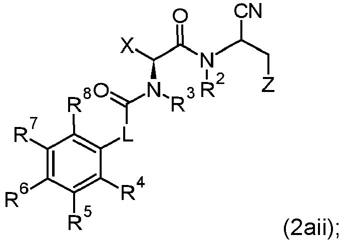 and salts thereof, wherein X, Z, L, R¹, R^{1a}, R², R³, R⁴, R⁵, R⁶, R⁷ and R⁸ are as defined herein.

Also provided are compounds of Formula (3a), (3ai) and (3aii): 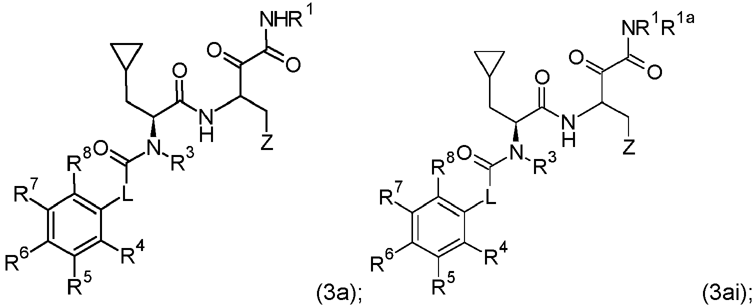 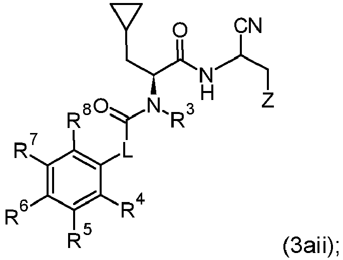 and salts thereof, wherein Z, L, R¹, R^{1a}, R³, R⁴, R⁵, R⁶, R⁷ and R⁸ are as defined herein.

Also provided are compounds of Formula (3b), (3bi) and (3bii): 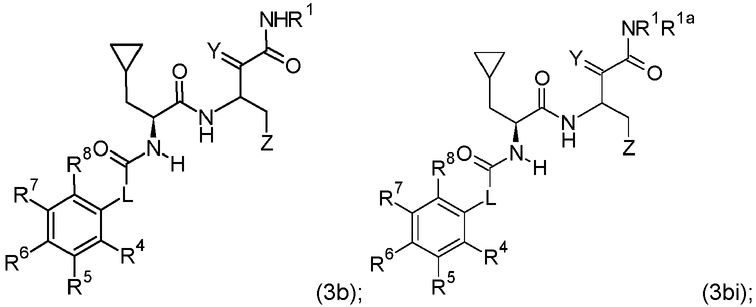 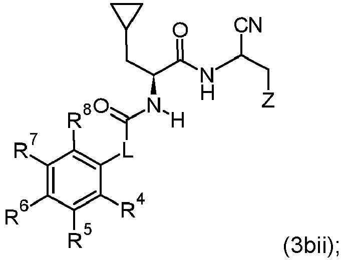 and salts thereof, wherein
Y is O or NOH;
Z is a 5- or 6-membered heterocyclic ring optionally substituted with oxo or 1 to 6 fluorine atoms, or Z is C₃₋₆ cycloalkyl optionally substituted with 1 to 6 fluorine atoms;
L is -CH=CH-, -CH₂-CH₂- or -O-CH₂-;
R¹ and R^{1a} are independently H or a C₁₋₄ saturated hydrocarbon group optionally substituted with 1 to 6 fluorine atoms;
R⁴, R⁵, R⁶, R⁷ and R⁸ are independently H or halo.

Also provided are compounds of Formula (3c), (3ci) and (3cii): 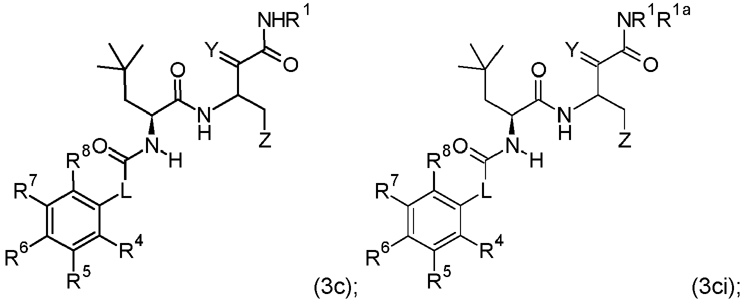 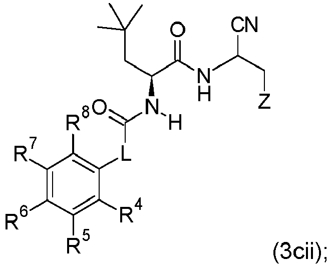 and salts thereof, wherein
Y is O or NOH;
Z is a 5- or 6-membered heterocyclic ring optionally substituted with oxo or 1 to 6 fluorine atoms, or Z is C₃₋₆ cycloalkyl optionally substituted with 1 to 6 fluorine atoms;
L is -CH=CH-, -CH₂-CH₂- or -O-CH₂-;
R¹ and R^{1a} are independently H or a C₁₋₄ saturated hydrocarbon group optionally substituted with 1 to 6 fluorine atoms;
R⁴, R⁵, R⁶, R⁷ and R⁸ are independently H or halo.

Also provided are compounds of Formula (4a), (4ai) and (4aii): 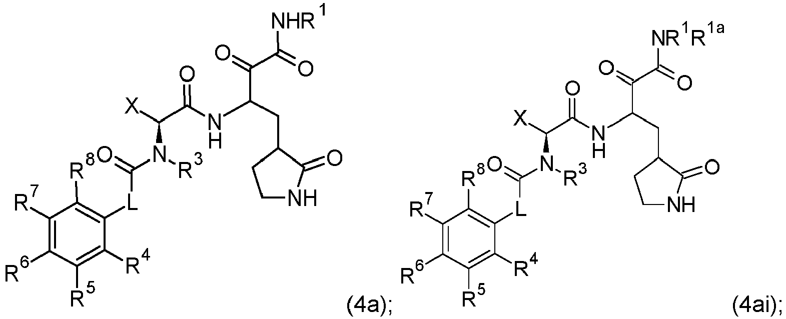 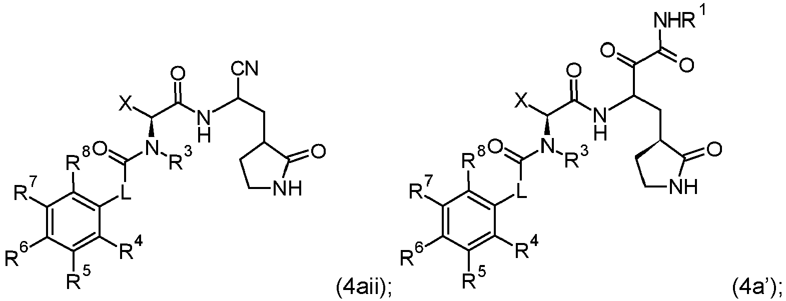 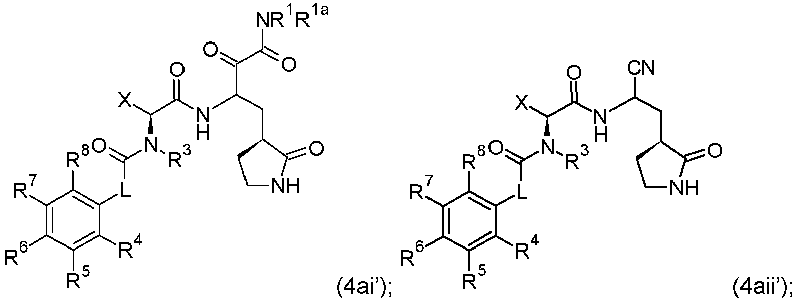 and salts thereof, wherein X, L, R¹, R^{1a}, R³, R⁴, R⁵, R⁶, R⁷ and R⁸ are as defined herein.

Also provided are compounds of Formula (5a) and (5ai): 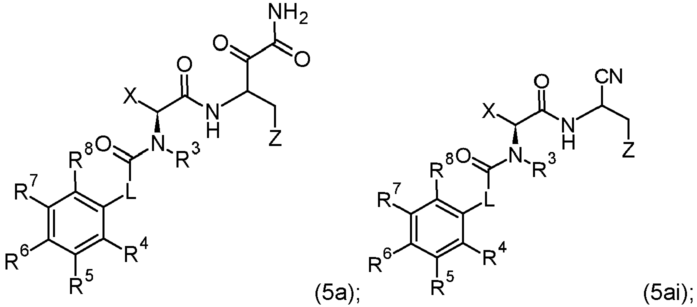 and salts thereof, wherein X, Z, L, R³, R⁴, R⁵, R⁶, R⁷ and R⁸ are as defined herein.

Also provided are compounds of Formula (6a) and (6ai): 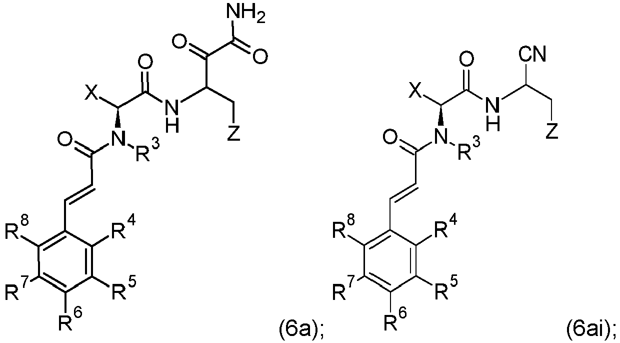 and salts thereof, wherein X, Z, R³, R⁴, R⁵, R⁶, R⁷ and R⁸ are as defined herein.

Also provided are compounds of Formula (7a) and (7ai): 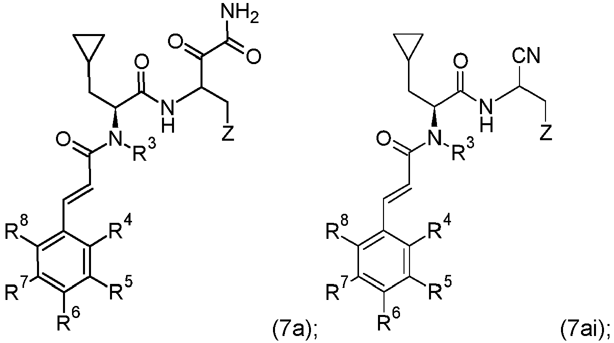 and salts thereof, wherein Z, R³, R⁴, R⁵, R⁶, R⁷ and R⁸ are as defined herein.

Also provided are compounds of Formula (8a) and (8ai): 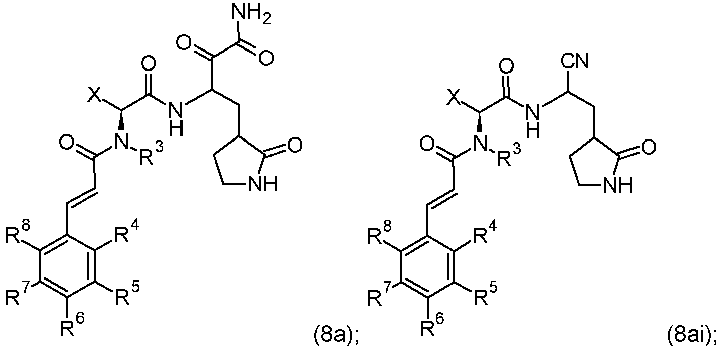 and salts thereof, wherein X, R³, R⁴, R⁵, R⁶, R⁷ and R⁸ are as defined herein.

Also provided are compounds of Formula (9a), (9ai) and (9b): 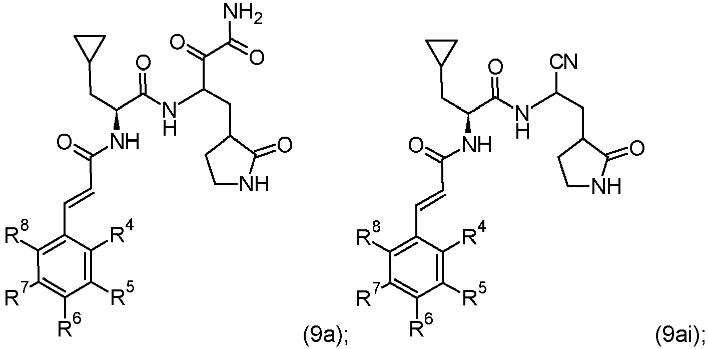 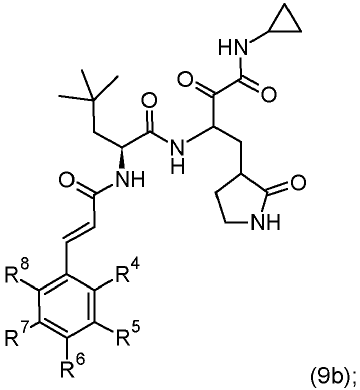 and salts thereof, wherein R⁴, R⁵, R⁶, R⁷ and R⁸ are as defined herein.

Also provided are compounds of Formula (2av) - (9bv): 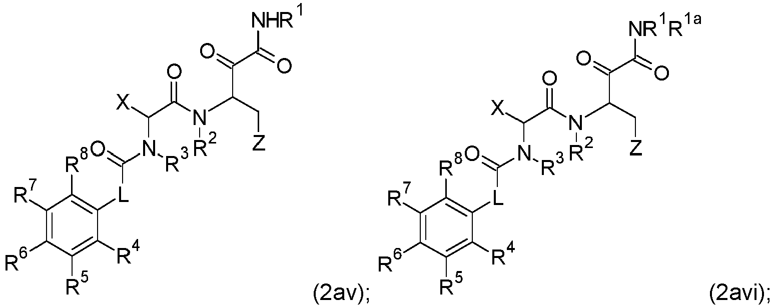 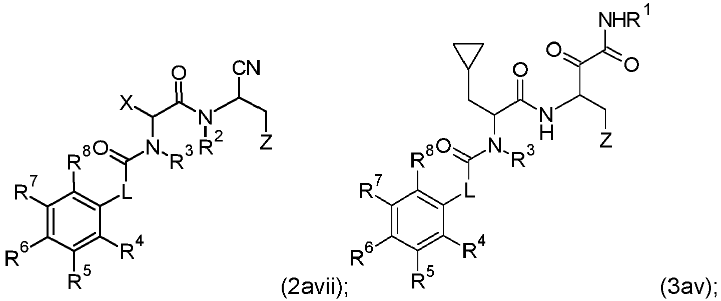 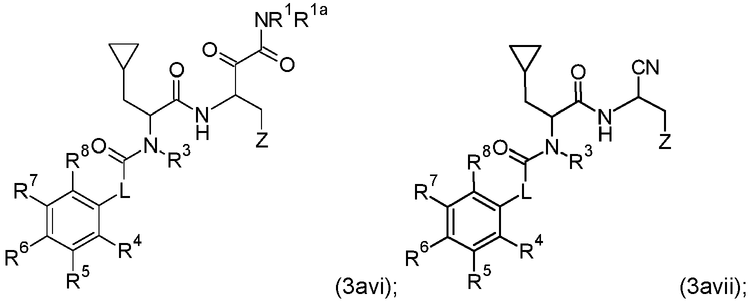 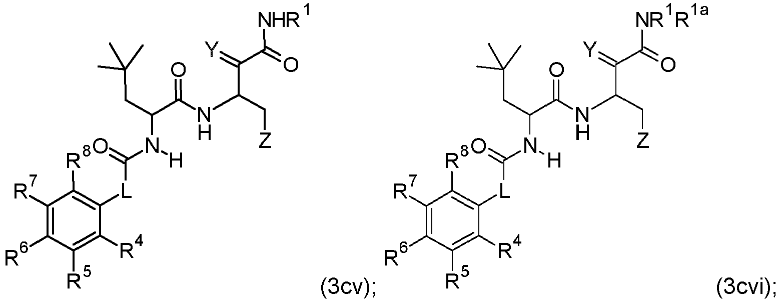 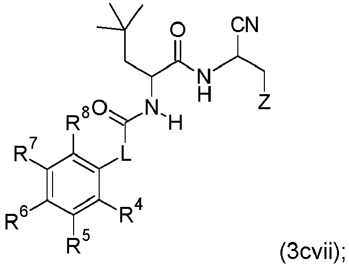 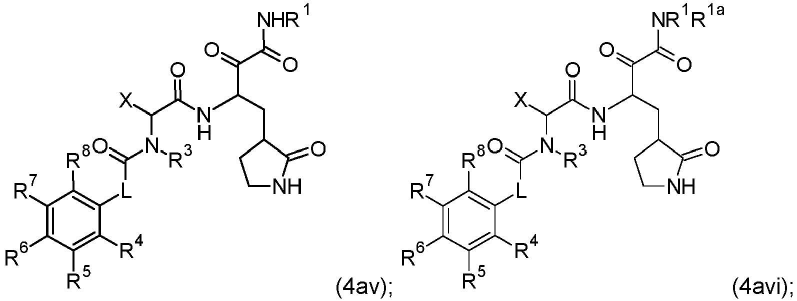 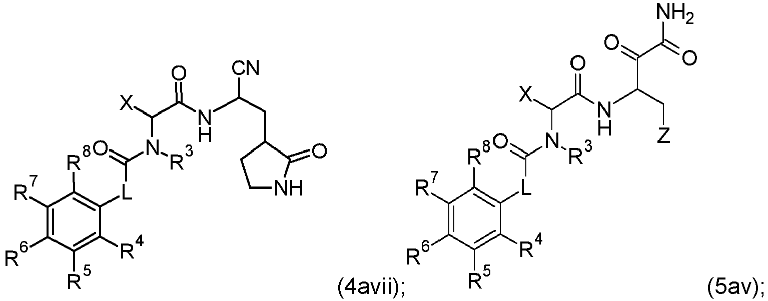 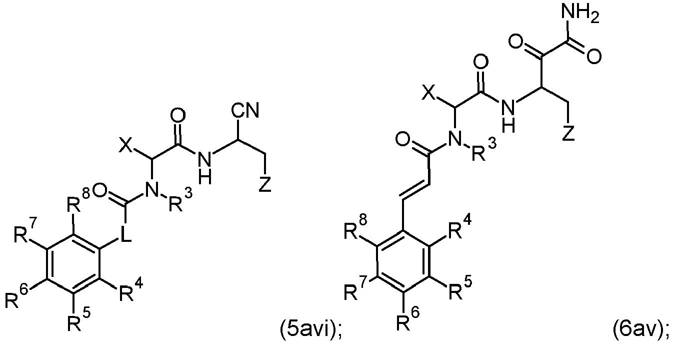 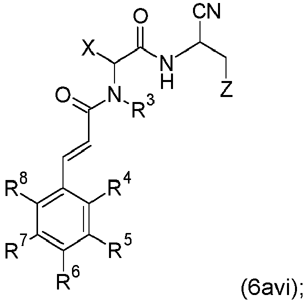 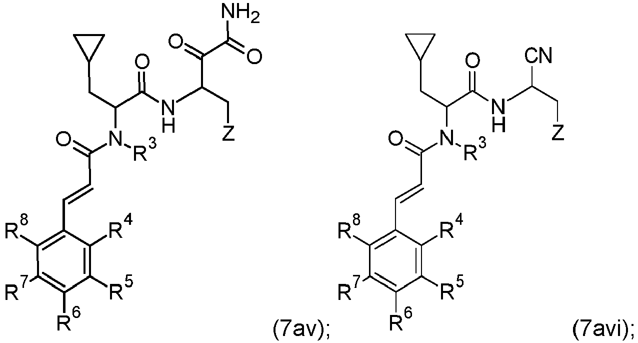 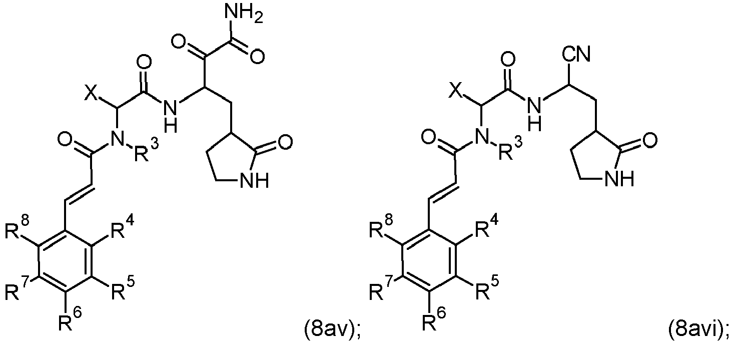 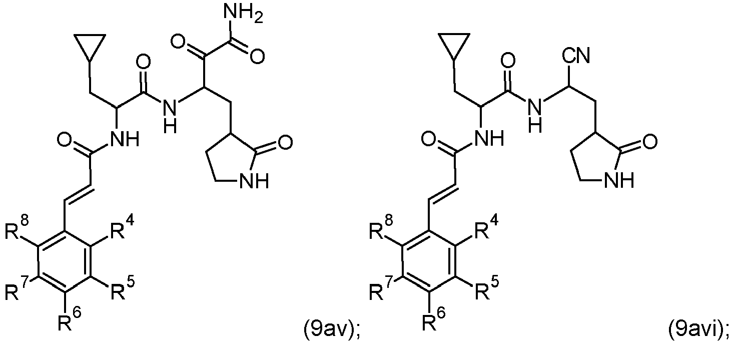 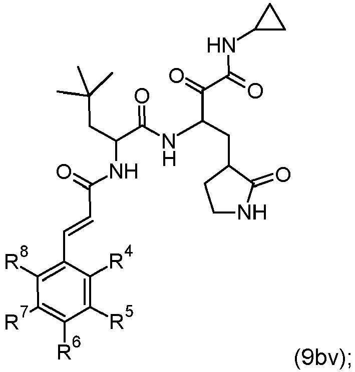 and salts thereof, wherein X, Z, Y, L, R¹, R², R³, R⁴, R⁵, R⁶, R⁷ and R⁸ are as defined herein.

In the compounds herein, Q can be CN. Q can be a group of formula: 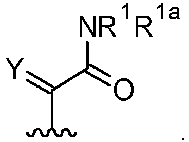
Q can be selected form the group consisting of: 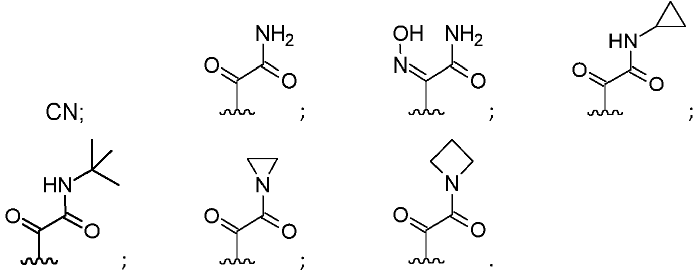
Q can be 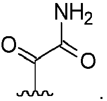 Q can be

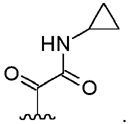

In the compounds herein, A can be selected from: 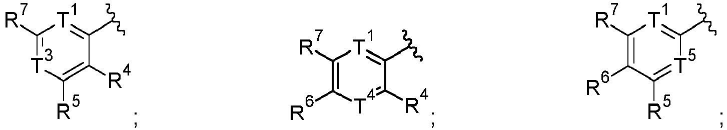 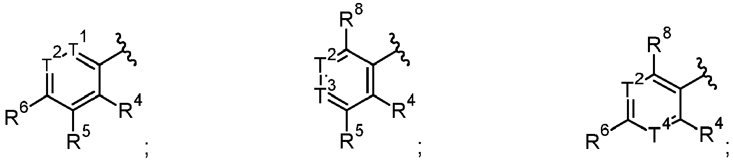 wherein, T¹, T², T³, T⁴, T⁵, R⁴, R⁵, R⁶, R⁷ and R⁸ are as defined herein.

A can be selected from: 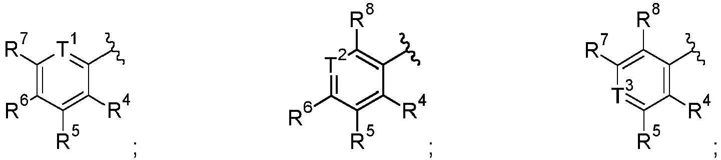 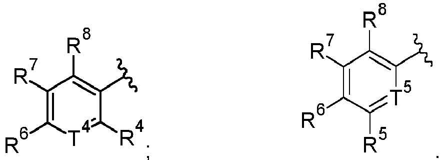 wherein, T¹, T², T³, T⁴, T⁵, R⁴, R⁵, R⁶, R⁷ and R⁸ are as defined herein.

A can be selected from:

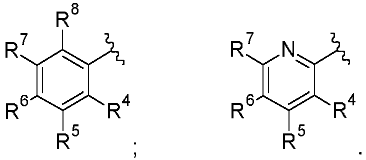

In the compounds herein, T¹ can be CR⁸ or N. T¹ can be CR⁸. T¹ can be N.

In the compounds herein, T² can be CR⁷ or N. T² can be CR⁷. T² can be N.

In the compounds herein, T³ can be CR⁶ or N. T³ can be CR⁶. T³ can be N.

In the compounds herein, T⁴ can be CR⁵ or N. T⁴ can be CR⁵. T⁴ can be N.

In the compounds herein, T⁵ can be CR⁴ or N. T⁵ can be CR⁴. T⁵ can be N.

In the compounds herein, X is a C₁₋₆ saturated or unsaturated hydrocarbon group optionally substituted with 1 to 6 fluorine atoms or X is joined with R⁹ to form a C₃₋₆ cycloalkyl ring which is optionally substituted with 1 to 3 fluorine atoms or 1 to 3 C₁₋₃ alkyl groups. X can be a C₁₋₆ saturated or unsaturated hydrocarbon group optionally substituted with 1 to 6 fluorine atoms. X can be C₁₋₆ alkyl optionally substituted with 1 to 6 fluorine atoms, branched C₁₋₆ alkyl optionally substituted with 1 to 6 fluorine atoms, C₃₋₆ cycloalkyl optionally substituted with 1 to 6 fluorine atoms, C₃₋₆ cycloalkenyl optionally substituted with 1 to 6 fluorine atoms, -CH₂-C₃₋₅ cycloalkyl optionally substituted with 1 to 6 fluorine atoms or -CH₂-C₃₋₅ cycloalkenyl optionally substituted with 1 to 6 fluorine atoms. X can be C₁₋₆ alkyl or branched C₁₋₆ alkyl. X can be selected from the group consisting of -CH₂CHF₂, isobutyl, neopentyl, -CH₂-cyclobutyl, cyclobutylmethyl, -CH₂-cyclopropyl, cyclopropylmethyl, -CH₂-difluorocyclobutyl, -CH₂-bicyclo[1.1.1]pentanyl, -CH₂-cyclopentenyl. X can be -CH₂-cyclopropyl. X can be cyclopropyl methyl.

X can be joined with R⁹ to form a C₃₋₆ cycloalkyl ring which is optionally substituted with 1 to 3 fluorine atoms or 1 to 3 C₁₋₃ alkyl groups. X can be joined with R⁹ to form a cyclobutyl ring which is optionally substituted with 1 to 3 fluorine atoms or 1 to 3 C₁₋₃ alkyl groups. X can be joined with R⁹ to form a cyclobutyl ring which is optionally substituted with 1 to 3 methyl groups. X can be joined with R⁹ to form

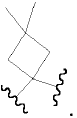

X can be selected from the group consisting of:

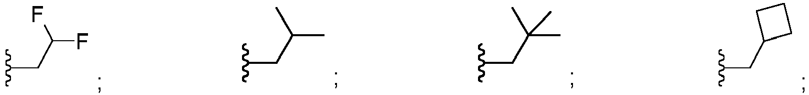

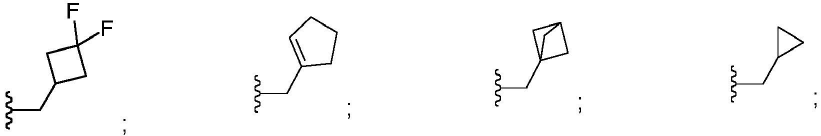

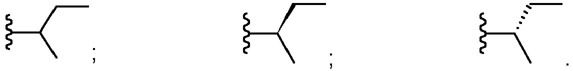

X can be 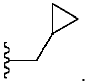 X can be

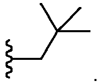

In the compounds herein, R⁹ is H or is joined with X to form a C₃₋₆ cycloalkyl ring which is optionally substituted with 1 to 3 fluorine atoms or 1 to 3 C₁₋₃ alkyl groups. R⁹ can be H. R⁹ can be joined with X to form a cyclobutyl ring which is optionally substituted with 1 to 3 fluorine atoms or 1 to 3 C₁₋₃ alkyl groups. R⁹ can be joined with X to form a cyclobutyl ring which is optionally substituted with 1 to 3 methyl groups. R⁹ can be joined with X to form

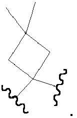

In the compounds herein, Z can be a 5- or 6-membered heterocyclic ring optionally substituted with oxo or 1 to 6 fluorine atoms, Z can be C₃₋₆ cycloalkyl optionally substituted with 1 to 6 fluorine atoms, Z can be -(CH₂)ₚCONHR¹³, Z can be -(CH₂)ₚCO₂R¹³. Z can be a pyrrolidine ring optionally substituted with oxo or 1 to 6 fluorine atoms. Z can be a pyrrolidone ring optionally substituted with 1 to 6 fluorine atoms. Z can be a 2-pyrrolidone ring optionally substituted with 1 to 6 fluorine atoms. Z can be a 2-pyrrolidone ring. Z can be selected from the group consisting of pyrrolidone, cyclopentane, cyclopropane, pyridine, -CH₂CONH₂ and pyrazole. Z can be pyrrolidone. Z can be 2-pyrrolidone.

Z can be selected from the group consisting of:

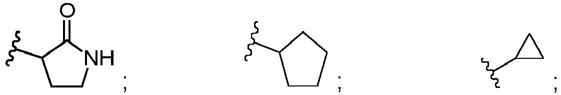

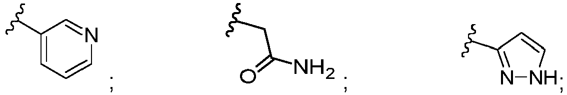

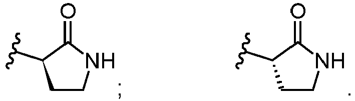

Z can be 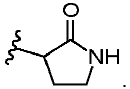 Z can be

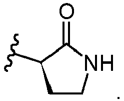

In the compounds herein p can be 0-3. p can be 0, 1, 2 or 3. p can be 0. p can be 1. p can be 2. p can be 3.

In the compounds herein, R¹³, R¹⁴ and R¹⁵ can independently be H or C₁₋₃ alkyl optionally substituted with 1 to 6 fluorine atoms.

In the compounds herein R¹³ can be H or C₁₋₃ alkyl optionally substituted with 1 to 6 fluorine atoms. R¹³ can be H or methyl. R¹³ can be H.

In the compounds herein R¹⁴ can be H or C₁₋₃ alkyl optionally substituted with 1 to 6 fluorine atoms. R¹⁴ can be H or methyl. R¹⁴ can be H.

In the compounds herein R¹⁵ can be H or C₁₋₃ alkyl optionally substituted with 1 to 6 fluorine atoms. R¹⁵ can be H or methyl. R¹⁵ can be H.

In the compounds herein, Y can be O or NOR¹⁶. Y can be O. Y can be NOR¹⁶. Y can be NOH.

In the compounds herein, R¹⁶ is H or C₁₋₃ alkyl optionally substituted with 1 to 6 fluorine atoms. R¹⁶ can be H.

In the compounds herein, L is a linker group selected from -CR¹¹=CR¹²-, -CHR¹¹-CHR¹²- and -O-CHR¹¹-. L can be -CH=CH-, -CH₂CH₂-, -CH₂-CH(CH₂CH₃)-, -CH₂-CH(CH₂CO₂H)-, -CH₂-CH(CH₃)-, -CH₂-CH(CF₃)-, -OCH₂-, -OCH(CH₃)- or 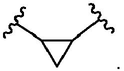 L can be -CH=CH-, -CH₂CH₂-, -CH₂-CH(CH₂CH₃)-, -CH₂-CH(CH₂CO₂H)- or -OCH₂-. L can be -CHR¹¹-CHR¹²-. When R¹ and R^{1a} are both H, L can be -CR¹¹=CR¹²-. When R¹ and R^{1a} are not both H, L can be -CR¹¹=CR¹²-where R³, R⁴, R⁵, R⁶, R⁷ and R⁸ are not all H. When R¹ and R^{1a} are both H, L can be is -O-CHR¹¹-. When R¹ and R^{1a} are not both H, L can be -O-CHR¹¹- where R³ is not H, or where two or more of R⁴, R⁵, R⁶, R⁷ and R⁸ are not H.

L can be selected from the group consisting of:

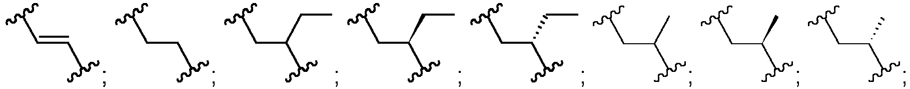

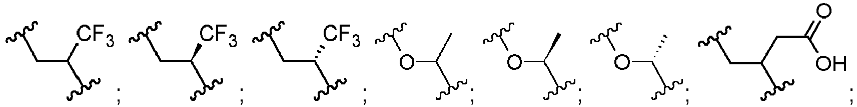

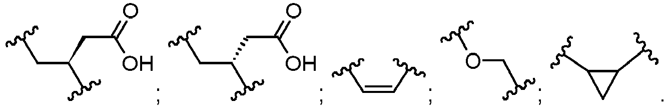

Unless stated otherwise L groups as defined herein can take either possible orientation with respect to their points of attachment to the remainder of the molecule. For example where L is defined as -OCH₂- included are both Examples 82 and 91 and where L is defined as -CH₂-CH(CF₃)- included are both Examples 86 and 88 (Table 1).

In the compounds herein R¹¹ and R¹² can be independently H, -(CH₂)ₘCO₂R¹⁵ or C₁₋₃ alkyl optionally substituted with 1 to 6 fluorine atoms; or R¹¹ and R¹² can be joined to form a cyclopropyl ring. R¹¹ and R¹² can be independently H, -(CH₂)ₘCO₂R¹⁵ or C₁₋₃ alkyl optionally substituted with 1 to 6 fluorine atoms; or R¹¹ and R¹² can be joined to form a cyclopropyl ring. R¹¹ and R¹² can independently be H, -(CH₂)ₘCO₂R¹⁵ or C₁₋₃ alkyl optionally substituted with 1 to 6 fluorine atoms. R¹¹ and R¹² can independently be H, methyl, ethyl or -CH₂CO₂H. R¹¹ can be H, -(CH₂)ₘCO₂R'^{S} or C₁₋₃ alkyl optionally substituted with 1 to 6 fluorine atoms. R¹¹ can be H, methyl, ethyl or-CH₂CO₂H. R¹² can be H, -(CH₂)ₘCO₂R¹⁵ or C₁₋₃ alkyl optionally substituted with 1 to 6 fluorine atoms. R¹² can be H, methyl, ethyl or -CH₂CO₂H. R¹¹ can be joined to R¹² to form a cyclopropyl ring. R¹² can be joined to R¹¹ to form a cyclopropyl ring. R¹¹ can be joined to R¹² to form a cyclopropyl ring. R¹² can be joined to R¹¹ to form a cyclopropyl ring.

In the compounds herein m can be 0-3. m can be 0, 1, 2 or 3. m can be 0. m can be 1. m can be 2. m can be 3. p and m can independently be 0-3.

In the compounds herein, R¹ and R^{1a} can independently be H or a C₁₋₆ saturated hydrocarbon group optionally substituted with 1 to 6 fluorine atoms, or R¹ and R^{1a} can be linked together to form a 3 to 6-membered saturated ring optionally containing an additional heteroatom. R¹ and R^{1a} can independently be H, C₁₋₆ alkyl optionally substituted with 1 to 6 fluorine atoms, branched C₁₋₆ alkyl optionally substituted with 1 to 6 fluorine atoms, C₃₋₆ cycloalkyl optionally substituted with 1 to 6 fluorine atoms or -CH₂-C₃₋₆ cycloalkyl optionally substituted with 1 to 6 fluorine atoms. R¹ and R^{1a} can independently be C₁₋₆ alkyl, branched C₁₋₆ alkyl, C₃₋₆ cycloalkyl or -CH₂-C₃₋₆ cycloalkyl. R¹ and R^{1a} can independently be selected from H methyl, ethyl, isopropyl, cyclopropyl, t-butyl, isobutyl, cyclopropylmethyl and -CH₂-cyclopropyl. R¹ and R^{1a} can both be H. R¹ can be joined to R^{1a} to form a 3 to 6-membered saturated ring optionally containing an additional heteroatom. R¹ can be joined to R^{1a} to form a 3 to 6-membered saturated ring. R¹ can be joined to R^{1a} to form an aziridine ring. R¹ can be joined to R^{1a} to form an azetidine ring. R¹ can be joined to R^{1a} to form a pyrrolidine ring. R¹ can be joined to R^{1a} to form a piperidine ring.

In the compounds herein, R¹ can be H or a C₁₋₆ saturated hydrocarbon group optionally substituted with 1 to 6 fluorine atoms, or R¹ and R^{1a} can be linked together to form a 3 to 6-membered saturated ring optionally containing an additional heteroatom. R¹ can be C₁₋₆ alkyl optionally substituted with 1 to 6 fluorine atoms, branched C₁₋₆ alkyl optionally substituted with 1 to 6 fluorine atoms, C₃₋₆ cycloalkyl optionally substituted with 1 to 6 fluorine atoms or -CH₂-C₃₋₆ cycloalkyl optionally substituted with 1 to 6 fluorine atoms. R¹ can be C₁₋₆ alkyl, branched C₁₋₆ alkyl, C₃₋₆ cycloalkyl or -CH₂-C₃₋₆ cycloalkyl. R¹ can be selected from H, methyl, ethyl, isopropyl, cyclopropyl, t-butyl, isobutyl, cyclopropylmethyl and -CH₂-cyclopropyl. R¹ can be H. R¹ can be cyclopropyl. R¹ can be joined to R^{1a} to form a 3 to 6-membered saturated ring optionally containing an additional heteroatom. R¹ can be joined to R^{1a} to form a 3 to 6-membered saturated ring. R¹ can be joined to R^{1a} to form an aziridine ring. R¹ can be joined to R^{1a} to form an azetidine ring. R¹ can be joined to R^{1a} to form a pyrrolidine ring. R¹ can be joined to R^{1a} to form a piperidine ring.

In the compounds herein, R^{1a} can be H or a C₁₋₆ saturated hydrocarbon group optionally substituted with 1 to 6 fluorine atoms, or R¹ and R^{1a} can be linked together to form a 3 to 6-membered saturated ring optionally containing an additional heteroatom. R^{1a} can be C₁₋₆ alkyl optionally substituted with 1 to 6 fluorine atoms, branched C₁₋₆ alkyl optionally substituted with 1 to 6 fluorine atoms, C₃₋₆ cycloalkyl optionally substituted with 1 to 6 fluorine atoms or -CH₂-C₃₋₆cycloalkyl optionally substituted with 1 to 6 fluorine atoms. R^{1a} can be C₁₋₆ alkyl, branched C₁₋₆ alkyl, C₃₋₆ cycloalkyl or -CH₂-C₃₋₆ cycloalkyl. R^{1a} can be selected from H, methyl, ethyl, isopropyl, cyclopropyl, *t*-butyl, isobutyl, cyclopropylmethyl and -CH₂-cyclopropyl. R^{1a} can be H. R^{1a} can be joined to R¹ to form a 3 to 6-membered saturated ring optionally containing an additional heteroatom. R^{1a} can be joined to R¹ to form a 3 to 6-membered saturated ring. R^{1a} can be joined to R¹ to form an aziridine ring. R^{1a} can be joined to R¹ to form an azetidine ring. R^{1a} can be joined to R¹ to form a pyrrolidine ring. R^{1a} can be joined to R¹ to form a piperidine ring.

Where R¹ and R^{1a} are linked together to form a 3 to 6-membered saturated ring optionally containing an additional heteroatom, the optional additional heteroatom may be selected from N, O, S and oxidised forms thereof.

R¹ and R^{1a} can independently be H or selected from the group consisting of: 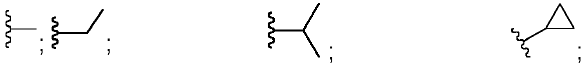 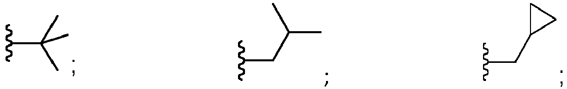 or R¹ and R^{1a} can be joined to form a ring such that the group NR¹R^{1a} is:

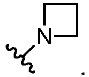

R¹ can be H or can be selected from the group consisting of:

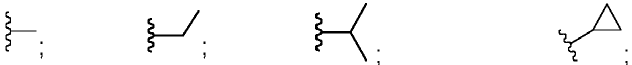

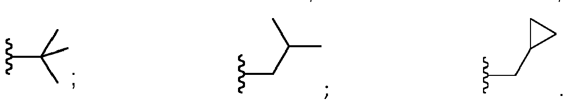

R^{1a} can be H or can be selected from the group consisting of:

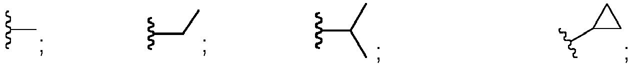

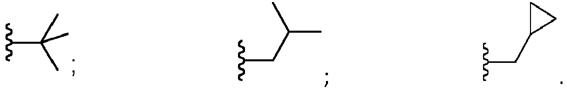

In the compounds herein R² and R³ are independently H or C₁₋₃ alkyl optionally substituted with 1 to 6 fluorine atoms. R² and R³ can independently be H, methyl or CH₂CF₃. R² and R³ can be H. R¹, R² and R³ can be H.

In the compounds herein, R² can be H or C₁₋₃ alkyl optionally substituted with 1 to 6 fluorine atoms. R² can be H. R¹ and R² can both be H.

In the compounds herein, R³ can be H or C₁₋₃ alkyl optionally substituted with 1 to 6 fluorine atoms. R³ can be H, methyl or CH₂CF₃. R³ can be H.

In the compounds herein, R⁴, R⁵, R⁶, R⁷ and R⁸ are independently H, halo, CN, CO₂R¹⁴, OR¹⁴, SO₂R¹⁴, SONHR¹⁴, OSO₂R¹⁴, PO(R¹⁴)₂, SF₅ or C₁₋₃ alkyl optionally substituted with 1 to 6 fluorine atoms. R⁴, R⁵, R⁶, R⁷ and R⁸ can be independently selected from H, halo, CO₂R¹⁴ or C₁₋₃ alkyl optionally substituted with 1 to 6 fluorine atoms. R⁴, R⁵, R⁶, R⁷, and R⁸ can be independently selected from H, CN, Cl, F, CF₂H, OCH₃, OCF₃, OCF₂H, SO₂CH₃, OSO₂CH₃, PO(CH₃)₂, SF₅ and CO₂H. R⁴, R⁵, R⁶, R⁷, and R⁸ can be independently selected from H, Cl, F, CF₂H and CO₂H. R⁴, R⁵, R⁶, R⁷ and R⁸ can be independently selected from H, Cl, F and CO₂H. R⁴, R⁵, R⁶, R⁷ and R⁸ can be H. R⁴ can be F, R⁵ can be H, R⁶ can be Cl and R⁷ and R⁸ can be H.

R⁴ can be selected from H, halo, CN, CO₂R¹⁴, OR¹⁴, SO₂R¹⁴, SONHR¹⁴, OSO₂R¹⁴, PO(R¹⁴)₂, SF₅ or C₁₋₃ alkyl optionally substituted with 1 to 6 fluorine atoms. R⁴ can be selected from H, Cl, F, CF₃, CF₂H and CO₂H. R⁴ can be H.

R⁵ can be selected from H, halo, CN, CO₂R¹⁴, OR¹⁴, SO₂R¹⁴, SONHR¹⁴, OSO₂R¹⁴, PO(R¹⁴)₂, SF₅ or C₁₋₃ alkyl optionally substituted with 1 to 6 fluorine atoms. R⁵ can be selected from H, CN, Cl, F, CF₂H, OCH₃, OCF₃, OCF₂H, SO₂CH₃, OSO₂CH₃, PO(CH₃)₂, SF₅ and CO₂H. R⁵ can be selected from H, Cl, F, CF₃, CF₂H and CO₂H. R⁵ can be H.

R⁶ can be selected from H, halo, CN, CO₂R¹⁴, OR¹⁴, SO₂R¹⁴, SONHR¹⁴, OSO₂R¹⁴, PO(R¹⁴)₂, SF₅ or C₁₋₃ alkyl optionally substituted with 1 to 6 fluorine atoms. R⁶ can be selected from H, CN, Cl, F, CF₂H, OCH₃, OCF₃, OCF₂H, SO₂CH₃, OSO₂CH₃, PO(CH₃)₂, SF₅ and CO₂H. R⁶ can be selected from H, Cl, F, CF₃, CF₂H and CO₂H. R⁶ can be H.

R⁷ can be selected from H, halo, CN, CO₂R¹⁴, OR¹⁴, SO₂R¹⁴, SONHR¹⁴, OSO₂R¹⁴, PO(R¹⁴)₂, SF₅ or C₁₋₃ alkyl optionally substituted with 1 to 6 fluorine atoms. R⁷ can be selected from H, CN, Cl, F, CF₂H, OCH₃, OCF₃, OCF₂H, SO₂CH₃, OSO₂CH₃, PO(CH₃)₂, SF₅ and CO₂H. R⁷ can be selected from H, Cl, F, CF₃, CF₂H and CO₂H. R⁷ can be H.

R⁸ can be selected from H, halo, CN, CO₂R¹⁴, OR¹⁴, SO₂R¹⁴, SONHR¹⁴, OSO₂R¹⁴, PO(R¹⁴)₂, SF₅ or C₁₋₃ alkyl optionally substituted with 1 to 6 fluorine atoms. R⁸ can be selected from H, CN, Cl, F, CF₂H, OCH₃, OCF₃, OCF₂H, SO₂CH₃, OSO₂CH₃, PO(CH₃)₂, SF₅ and CO₂H. R⁸ can be selected from H, Cl, F, CF₃, CF₂H and CO₂H. R⁸ can be H.

In the compounds herein, the moiety A can be selected from the group consisting of:

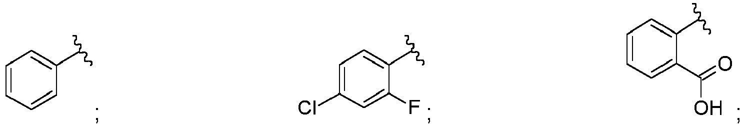

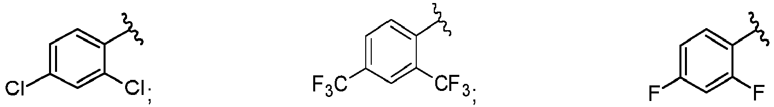

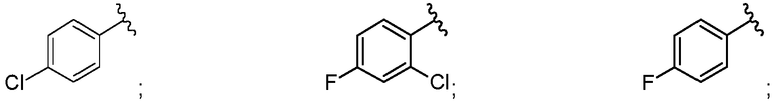

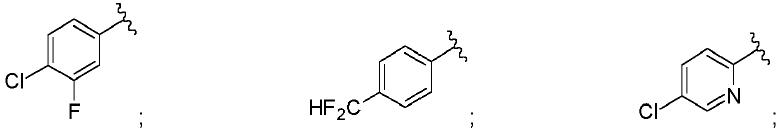

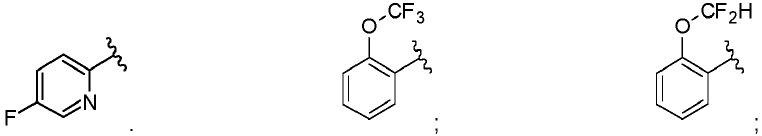

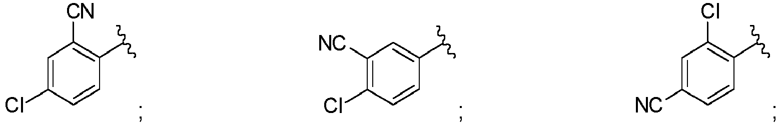

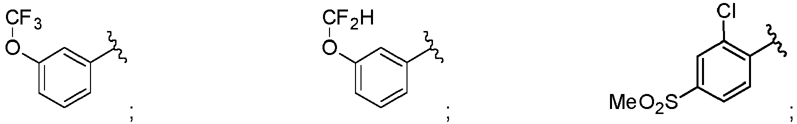

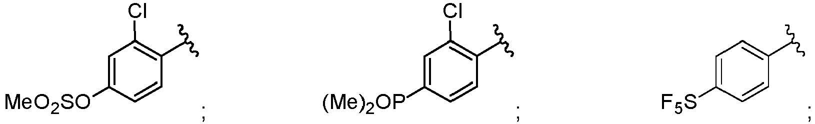

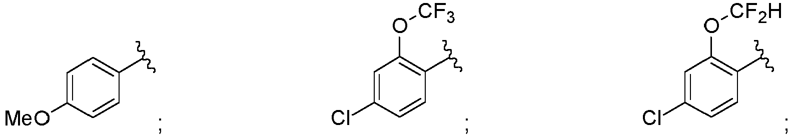

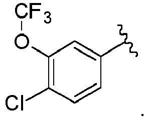

In the compounds herein, the moiety: 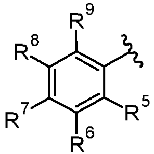 can be selected from the group consisting of:

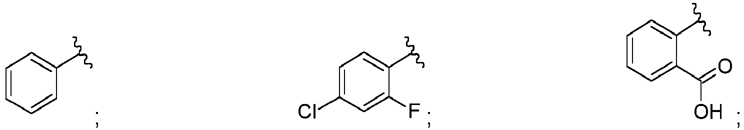

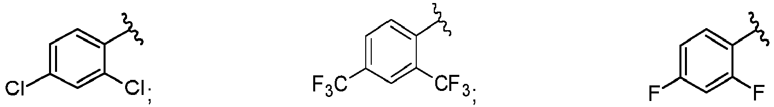

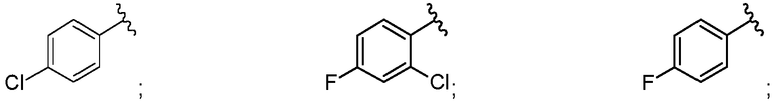

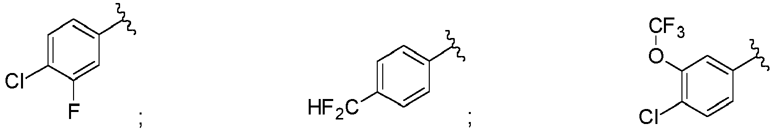

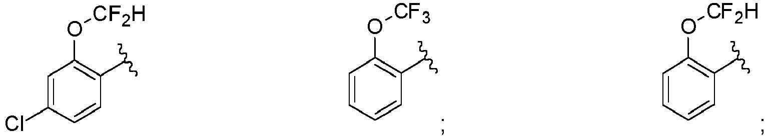

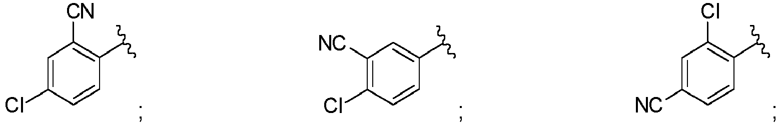

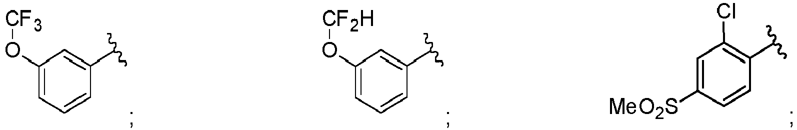

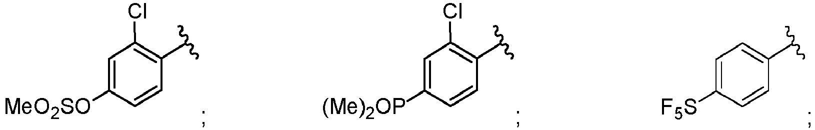

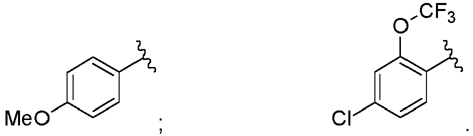

In the compounds herein when R¹ and R^{1a} are not both H, L is:

-CHR¹¹-CHR¹²-;

-CR¹¹=CR¹²- where R³, R⁴, R⁵, R⁶, R⁷ and R⁸ are not all H;
or -O-CHR¹¹- where R³ is not H, or where two or more of R⁴, R⁵, R⁶, R⁷ and R⁸ are not H.

For compounds where L is -CHR¹¹-CHR¹²-, R¹ and R^{1a} can independently be H, a C₁₋₆ saturated hydrocarbon group optionally substituted with 1 to 6 fluorine atoms, or R¹ and R^{1a} can be linked together to form a 3 to 6-membered saturated ring optionally containing an additional heteroatom.

For compounds where L is -CHR¹¹-CHR¹²-, R¹ and R^{1a} can both be H.

For compounds where L is -CHR¹¹-CHR¹²-, R¹ and R^{1a} can be a C₁₋₆ saturated hydrocarbon group optionally substituted with 1 to 6 fluorine atoms, or R¹ and R^{1a} can be linked together to form a 3 to 6-membered saturated ring optionally containing an additional heteroatom.

For compounds where L is -CR¹¹=CR¹²-, R¹ and R^{1a} can independently be H, a C₁₋₆ saturated hydrocarbon group optionally substituted with 1 to 6 fluorine atoms, or R¹ and R^{1a} can be linked together to form a 3 to 6-membered saturated ring optionally containing an additional heteroatom, provided that R³, R⁴, R⁵, R⁶, R⁷ and R⁸ are not all H.

For compounds where L is -CR¹¹=CR¹²-, R¹ and R^{1a} can both be H.

For compounds where L is -CR¹¹=CR¹²-, R¹ and R^{1a} can independently be a C₁₋₆ saturated hydrocarbon group optionally substituted with 1 to 6 fluorine atoms, or R¹ and R^{1a} can be linked together to form a 3 to 6-membered saturated ring optionally containing an additional heteroatom, provided that R³, R⁴, R⁵, R⁶, R⁷ and R⁸ are not all H.

For compounds where L is -O-CHR¹¹-, R¹ and R^{1a} can independently be H, a C₁₋₆ saturated hydrocarbon group optionally substituted with 1 to 6 fluorine atoms, or R¹ and R^{1a} can be linked together to form a 3 to 6-membered saturated ring optionally containing an additional heteroatom, provided that R³ is not H, or provided that two or more of R⁴, R⁵, R⁶, R⁷ and R⁸ are not H.

For compounds where L is -O-CHR¹¹-, R¹ and R^{1a} can both be H.

For compounds where L is -O-CHR¹¹-, R¹ and R^{1a} can independently be a C₁₋₆ saturated hydrocarbon group optionally substituted with 1 to 6 fluorine atoms, or R¹ and R^{1a} can be linked together to form a 3 to 6-membered saturated ring optionally containing an additional heteroatom, provided that R³ is not H, or provided that two or more of R⁴, R⁵, R⁶, R⁷ and R⁸ are not H.

The compound can be selected from any one of Examples 1 to 93 as shown in Table 1 or a salt thereof.

The compound can be selected from the group consisting of:
3-((S)-2-((E)-3-(4-chloro-2-fluorophenyl)acrylamido)-3-cyclopropylpropanamido)-2-oxo-4-((S)-2-oxopyrrolidin-3-yl)butanamide;
3-((*S*)-2-cinnamamido-3-cyclopropylpropanamido)-2-oxo-4-((*S*)-2-oxopyrrolidin-3-yl)butanamide;
benzyl ((2*S*)-1-((4-amino-3,4-dioxo-1-((*S*)-2-oxopyrrolidin-3-yl)butan-2-yl)amino)-3-cyclopropyl-1-oxopropan-2-yl)carbamate;
3-((*S*)-2-((*E*)-3-(4-chloro-2-fluorophenyl)acrylamido)-3-cyclopropylpropanamido)-2-(hydroxyimino)-4-((*S*)-2-oxopyrrolidin-3-yl)butanamide;
*N*-(*tert*-butyl)-3-((*S*)-2-((*E*)-3-(4-chloro-2-fluorophenyl)acrylamido)-3-cyclopropylpropanamido)-2-oxo-4-((*S*)-2-oxopyrrolidin-3-yl)butanamide;
3-((*S*)-2-((*E*)-3-(4-chloro-2-fluorophenyl)acrylamido)-3-cyclopropylpropanamido)-4-cyclopropyl-2-oxobutanamide;
3-((*S*)-2-((*E*)-3-(4-chloro-2-fluorophenyl)acrylamido)-3-cyclopropylpropanamido)-4-cyclopentyl-2-oxobutanamide;
3-((*S*)-3-cyclopropyl-2-(3-phenylpropanamido)propanamido)-2-oxo-4-((*S*)-2-oxopyrrolidin-3-yl)butanamide;
(2*S*)-*N*-(1-cyano-2-((*S*)-2-oxopyrrolidin-3-yl)ethyl)-2-((*E*)-3-(2,4-dichlorophenyl)acrylamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*E*)-3-(2,4-dichlorophenyl)acrylamido)-4,4-dimethylpentanamide;
(2*S*)-2-((*E*)-3-(4-chloro-2-fluorophenyl)acrylamido)-*N*-(4-(cyclopropylamino)-3,4-dioxo-1-((*S*)-2-oxopyrrolidin-3-yl)butan-2-yl)-4,4-dimethylpentanamide;
(2*S*)-*N*-(4-(cyclopropylamino)-3,4-dioxo-1-((*S*)-2-oxopyrrolidin-3-yl)butan-2-yl)-4,4-dimethyl-2-((*S*)-3-phenylbutanamido)pentanamide;
(2*S*)-*N*-(4-(cyclopropylamino)-3,4-dioxo-1-((*S*)-2-oxopyrrolidin-3-yl)butan-2-yl)-4,4-dimethyl-2-((*R*)-3-phenylbutanamido)pentanamide;
(2S)-*N*-(4-(cyclopropylamino)-3,4-dioxo-1-((*S*)-2-oxopyrrolidin-3-yl)butan-2-yl)-4,4-dimethyl-2-((*S*)-3-phenylpentanamido)pentanamide;
(2*S*)-*N*-(4-(cyclopropylamino)-3,4-dioxo-1-((*S*)-2-oxopyrrolidin-3-yl)butan-2-yl)-4,4-dimethyl-2-((*R*)-3-phenylpentanamido)pentanamide;
(2*S*)-*N*-(4-(cyclopropylamino)-3,4-dioxo-1-((*S*)-2-oxopyrrolidin-3-yl)butan-2-yl)-4,4-dimethyl-2-((*S*)-4,4,4-trifluoro-3-phenylbutanamido)pentanamide;
(2*S*)-*N*-(4-(cyclopropylamino)-3,4-dioxo-1-((*S*)-2-oxopyrrolidin-3-yl)butan-2-yl)-4,4-dimethyl-2-((*R*)-4,4,4-trifluoro-3-phenylbutanamido)pentanamide
(2*S*)-*N*-(4-amino-3,4-dioxo-1-((*S*)-2-oxopyrrolidin-3-yl)butan-2-yl)-2-((*E*)-3-(4-chloro-2-fluorophenyl)acrylamido)-4-methylpentanamide;
3-((*S*)-2-((*E*)-3-(4-chloro-2-fluorophenyl)acrylamido)-3-cyclopropylpropanamido)-*N*-ethyl-2-oxo-4-((*S*)-2-oxopyrrolidin-3-yl)butanamide;
3-((*S*)-3-cyclopropyl-2-((*E*)-3-(2,4-dichlorophenyl)acrylamido)propanamido)-2-oxo-4-((*S*)-2-oxopyrrolidin-3-yl)butanamide;
3-((*S*)-2-((*E*)-3-(4-chloro-2-fluorophenyl)acrylamido)-3-cyclopropylpropanamido)-*N-*cyclopropyl-2-oxo-4-((*S*)-2-oxopyrrolidin-3-yl)butanamide;
(2*S*)-*N*-(4-amino-3,4-dioxo-1-((*S*)-2-oxopyrrolidin-3-yl)butan-2-yl)-2-((*E*)-3-(4-chloro-2-fluorophenyl)acrylamido)-4,4-dimethylpentanamide;
(2*S*)-2-((*E*)-3-(4-chloro-2-fluorophenyl)acrylamido)-*N*-(4-(ethylamino)-3,4-dioxo-1-((*S*)-2-oxopyrrolidin-3-yl)butan-2-yl)-4,4-dimethylpentanamide;
3-((*S*)-3-cyclopropyl-2-((*E*)-3-(2,4-dichlorophenyl)acrylamido)propanamido)-*N*-ethyl-2-oxo-4-((*S*)-2-oxopyrrolidin-3-yl)butanamide;
*N*-cyclopropyl-3-((*S*)-3-cyclopropyl-2-((*E*)-3-(2,4-dichlorophenyl)acrylamido)propanamido)-2-oxo-4-((*S*)-2-oxopyrrolidin-3-yl)butanamide;
(2*S*)-2-((*E*)-3-(2,4-dichlorophenyl)acrylamido)-*N*-(4-(ethylamino)-3,4-dioxo-1-((*S*)-2-oxopyrrolidin-3-yl)butan-2-yl)-4,4-dimethylpentanamide;
(2*S*)-*N*-(4-amino-3,4-dioxo-1-((*S*)-2-oxopyrrolidin-3-yl)butan-2-yl)-2-((*E*)-3-(2,4-dichlorophenyl)acrylamido)-4,4-dimethylpentanamide;
3-((*S*)-2-((*E*)-3-(4-chloro-2-fluorophenyl)acrylamido)-3-cyclopropylpropanamido)-*N-*cyclopropyl-2-oxo-4-((*S*)-2-oxopyrrolidin-3-yl)butanamide;
benzyl ((2*S*)-3-cyclopropyl-1-((4-(cyclopropylamino)-3,4-dioxo-1-((*S*)-2-oxopyrrolidin-3-yl)butan-2-yl)amino)-1-oxopropan-2-yl)carbamate;
*N*-cyclopropyl-3-((*S*)-3-cyclopropyl-2-(3-(2,4-dichlorophenyl)propanamido)propanamido)-2-oxo-4-((*S*)-2-oxopyrrolidin-3-yl)butanamide;
3-((*S*)-2-((*E*)-3-(4-chloro-2-fluorophenyl)acrylamido)-3-cyclobutylpropanamido)-*N-*cyclopropyl-2-oxo-4-((*S*)-2-oxopyrrolidin-3-yl)butanamide;
(3*S*)-*N*-((2*S*)-3-cyclopropyl-1-((4-(cyclopropylamino)-3,4-dioxo-1-((*S*)-2-oxopyrrolidin-3-yl)butan-2-yl)amino)-1-oxopropan-2-yl)-3-phenylpentanamide;
(3*R*)-*N*-((2*S*)-3-cyclopropyl-1-((4-(cyclopropylamino)-3,4-dioxo-1-((*S*)-2-oxopyrrolidin-3-yl)butan-2-yl)amino)-1-oxopropan-2-yl)-3-phenylpentanamide;
(2*S*)-*N*-(4-(azetidin-1-yl)-3,4-dioxo-1-((S)-2-oxopyrrolidin-3-yl)butan-2-yl)-2-((*E*)-3-(2,4-dichlorophenyl)acrylamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*E*)-3-(2,4-difluorophenyl)acrylamido)-4,4-dimethylpentanamide;
(2*S*)-2-((*E*)-3-(4-chlorophenyl)acrylamido)-*N*-(4-(cyclopropylamino)-3,4-dioxo-1-((*S*)-2-oxopyrrolidin-3-yl)butan-2-yl)-4,4-dimethylpentanamide;
(2*S*)-2-((*E*)-3-(2-chloro-4-fluorophenyl)acrylamido)-*N*-(4-(cyclopropylamino)-3,4-dioxo-1-((*S*)-2-oxopyrrolidin-3-yl)butan-2-yl)-4,4-dimethylpentanamide;
(2*S*)-*N*-(4-(cyclopropylamino)-3,4-dioxo-1-((*S*)-2-oxopyrrolidin-3-yl)butan-2-yl)-2-((*E*)-3-(4-fluorophenyl)acrylamido)-4,4-dimethylpentanamide;
(2*S*)-2-((*E*)-3-(4-chloro-3-fluorophenyl)acrylamido)-*N*-(4-(cyclopropylamino)-3,4-dioxo-1-((*S*)-2-oxopyrrolidin-3-yl)butan-2-yl)-4,4-dimethylpentanamide;
(2*S*)-2-((*E*)-3-(5-chloropyridin-2-yl)acrylamido)-*N*-(4-(cyclopropylamino)-3,4-dioxo-1-((*S*)-2-oxopyrrolidin-3-yl)butan-2-yl)-4,4-dimethylpentanamide;
(2*S*)-*N*-(4-(cyclopropylamino)-3,4-dioxo-1-((*S*)-2-oxopyrrolidin-3-yl)butan-2-yl)-2-((*E*)-3-(5-fluoropyridin-2-yl)acrylamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*E*)-3-(4-(difluoromethyl)phenyl)acrylamido)-4,4-dimethylpentanamide;
(1*R*,2*R*)-*N*-((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)-2-phenylcyclopropane-1-carboxamide;
(1*S*,2*S*)-*N*-((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)-2-phenylcyclopropane-1-carboxamide;
(2*S*)-*N*-(4-(cyclopropylamino)-3,4-dioxo-1-((*S*)-2-oxopyrrolidin-3-yl)butan-2-yl)-2-((*S*)-3-(2,4-difluorophenyl)pentanamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*R*)-3-(2,4-difluorophenyl)pentanamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*S*)-3-(2,4-dichlorophenyl)pentanamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*R*)-3-(2,4-dichlorophenyl)pentanamido)-4,4-dimethylpentanamide;
(2*S*)-2-((*S*)-3-(4-chloro-2-fluorophenyl)pentanamido)-*N*-(4-(cyclopropylamino)-3,4-dioxo-1-((*S*)-2-oxopyrrolidin-3-yl)butan-2-yl)-4,4-dimethylpentanamide;
(2*S*)-2-((*R*)-3-(4-chloro-2-fluorophenyl)pentanamido)-*N*-(4-(cyclopropylamino)-3,4-dioxo-1-((*S*)-2-oxopyrrolidin-3-yl)butan-2-yl)-4,4-dimethylpentanamide;
(2*S*)-2-((*S*)-3-(4-chlorophenyl)pentanamido)-*N*-(4-(cyclopropylamino)-3,4-dioxo-1-((*S*)-2-oxopyrrolidin-3-yl)butan-2-yl)-4,4-dimethylpentanamide;
(2*S*)-2-((*R*)-3-(4-chlorophenyl)pentanamido)-*N*-(4-(cyclopropylamino)-3,4-dioxo-1-((*S*)-2-oxopyrrolidin-3-yl)butan-2-yl)-4,4-dimethylpentanamide;
(2*S*)-*N*-(4-(cyclopropylamino)-3,4-dioxo-1-((*S*)-2-oxopyrrolidin-3-yl)butan-2-yl)-2-((*S*)-3-(4-fluorophenyl)pentanamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*R*)-3-(4-fluorophenyl)pentanamido)-4,4-dimethylpentanamide;
(2*S*)-*N*-(4-(azetidin-1-yl)-3,4-dioxo-1-((*S*)-2-oxopyrrolidin-3-yl)butan-2-yl)-2-((*E*)-3-(4-chloro-2-fluorophenyl)acrylamido)-4,4-dimethylpentanamide;
(2*S*)-*N*-(4-(cyclopropylamino)-3,4-dioxo-1-((*S*)-2-oxopyrrolidin-3-yl)butan-2-yl)-4,4-dimethyl-2-((*R*)-3-(2-(trifluoromethoxy)phenyl)pentanamido)pentanamide;
(2*S*)-*N*-(4-(cyclopropylamino)-3,4-dioxo-1-((*S*)-2-oxopyrrolidin-3-yl)butan-2-yl)-4,4-dimethyl-2-((*S*)-3-(2-(trifluoromethoxy)phenyl)pentanamido)pentanamide;
(2*S*)-*N*-(4-(cyclopropylamino)-3,4-dioxo-1-((*S*)-2-oxopyrrolidin-3-yl)butan-2-yl)-2-((*R*)-3-(2-(difluoromethoxy)phenyl)pentanamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*S*)-3-(2-(difluoromethoxy)phenyl)pentanamido)-4,4-dimethylpentanamide;
(2*S*)-2-((*E*)-3-(4-chloro-2-cyanophenyl)acrylamido)-*N*-(4-(cyclopropylamino)-3,4-dioxo-1-((*S*)-2-oxopyrrolidin-3-yl)butan-2-yl)-4,4-dimethylpentanamide;
(2*S*)-2-((*E*)-3-(4-chloro-3-cyanophenyl)acrylamido)-*N*-(4-(cyclopropylamino)-3,4-dioxo-1-((*S*)-2-oxopyrrolidin-3-yl)butan-2-yl)-4,4-dimethylpentanamide;
(2*S*)-2-((*E*)-3-(2-chloro-4-cyanophenyl)acrylamido)-*N*-(4-(cyclopropylamino)-3,4-dioxo-1-((*S*)-2-oxopyrrolidin-3-yl)butan-2-yl)-4,4-dimethylpentanamide;
(2*S*)-*N*-(4-(cyclopropylamino)-3,4-dioxo-1-((*S*)-2-oxopyrrolidin-3-yl)butan-2-yl)-4,4-dimethyl-2-((*R*)-3-(3-(trifluoromethoxy)phenyl)butanamido)pentanamide;
(2*S*)-*N*-(4-(cyclopropylamino)-3,4-dioxo-1-((*S*)-2-oxopyrrolidin-3-yl)butan-2-yl)-4,4-dimethyl-2-((*S*)-3-(3-(trifluoromethoxy)phenyl)butanamido)pentanamide;
(2*S*)-*N*-(4-(cyclopropylamino)-3,4-dioxo-1-((*S*)-2-oxopyrrolidin-3-yl)butan-2-yl)-2-((*R*)-3-(3-(difluoromethoxy)phenyl)butanamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*S*)-3-(3-(difluoromethoxy)phenyl)butanamido)-4,4-dimethylpentanamide;
(2*S*)-2-((*R*)-3-(2-chloro-4-(methylsulfonyl)phenyl)pentanamido)-*N*-(4-(cyclopropylamino)-3,4-dioxo-1-((*S*)-2-oxopyrrolidin-3-yl)butan-2-yl)-4,4-dimethylpentanamide;
(2*S*)-2-((*S*)-3-(2-chloro-4-(methylsulfonyl)phenyl)pentanamido)-*N*-(4-(cyclopropylamino)-3,4-dioxo-1-((*S*)-2-oxopyrrolidin-3-yl)butan-2-yl)-4,4-dimethylpentanamide;
3-chloro-4-((3*R*)-1-(((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)amino)-1-oxopentan-3-yl)phenyl methanesulfonate;
3-chloro-4-((3*S*)-1-(((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)amino)-1-oxopentan-3-yl)phenyl methanesulfonate;
(2*S*)-2-((*R*)-3-(2-chloro-4-(dimethylphosphoryl)phenyl)pentanamido)-*N*-(4-(cyclopropylamino)-3,4-dioxo-1-((*S*)-2-oxopyrrolidin-3-yl)butan-2-yl)-4,4-dimethylpentanamide;
(2*S*)-2-((*S*)-3-(2-chloro-4-(dimethylphosphoryl)phenyl)pentanamido)-*N*-(4-(cyclopropylamino)-3,4-dioxo-1-((*S*)-2-oxopyrrolidin-3-yl)butan-2-yl)-4,4-dimethylpentanamide;
(2*S*)-*N*-(4-(cyclopropylamino)-3,4-dioxo-1-((*S*)-2-oxopyrrolidin-3-yl)butan-2-yl)-4,4-dimethyl-2-((*E*)-3-(4-(pentafluoro-I6-sulfaneyl)phenyl)acrylamido)pentanamide;
(2*S*)-*N*-(4-(cyclopropylamino)-3,4-dioxo-1-((*S*)-2-oxopyrrolidin-3-yl)butan-2-yl)-2-((*R*)-3-(4-methoxyphenyl)pentanamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*S*)-3-(4-methoxyphenyl)pentanamido)-4,4-dimethylpentanamide;
(2*S*)-2-(3-(4-chloro-2-(trifluoromethoxy)phenyl)propanamido)-*N*-(4-(cyclopropylamino)-3,4-dioxo-1-((*S*)-2-oxopyrrolidin-3-yl)butan-2-yl)-4,4-dimethylpentanamide;
(2*S*)-2-(3-(4-chloro-2-(difluoromethoxy)phenyl)propanamido)-*N*-(4-(cyclopropylamino)-3,4-dioxo-1-((*S*)-2-oxopyrrolidin-3-yl)butan-2-yl)-4,4-dimethylpentanamide;
(2*S*)-2-(3-(4-chloro-2-cyanophenyl)propanamido)-*N*-(4-(cyclopropylamino)-3,4-dioxo-1-((*S*)-2-oxopyrrolidin-3-yl)butan-2-yl)-4,4-dimethylpentanamide;
(2*S*)-2-(3-(2-chloro-4-cyanophenyl)propanamido)-*N*-(4-(cyclopropylamino)-3,4-dioxo-1-((*S*)-2-oxopyrrolidin-3-yl)butan-2-yl)-4,4-dimethylpentanamide;
(2*S*)-2-(3-(4-chloro-3-(trifluoromethoxy)phenyl)propanamido)-*N*-(4-(cyclopropylamino)-3,4-dioxo-1-((*S*)-2-oxopyrrolidin-3-yl)butan-2-yl)-4,4-dimethylpentanamide;
(2*S*)-*N*-(1-cyano-2-((*S*)-2-oxopyrrolidin-3-yl)ethyl)-2-(3-(2,4-dichlorophenyl)propanamido)-4,4-dimethylpentanamide;
2,4-dichlorobenzyl ((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)carbamate;
4-chloro-2-fluorobenzyl ((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)carbamate;
(*S*)-1-(4-chloro-2-fluorophenyl)ethyl ((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)carbamate;
(*R*)-1-(4-chloro-2-fluorophenyl)ethyl ((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)carbamate;
(2*S*)-*N*-(4-(cyclopropylamino)-3,4-dioxo-1-((*S*)-2-oxopyrrolidin-3-yl)butan-2-yl)-2-((*R*)-3-(2,4-difluorophenyl)-4,4,4-trifluorobutanamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*S*)-3-(2,4-difluorophenyl)-4,4,4-trifluorobutanamido)-4,4-dimethylpentanamide;
(2*S*)-2-((*R*)-2-benzyl-3,3,3-trifluoropropanamido)-*N*-(4-(cyclopropylamino)-3,4-dioxo-1-((*S*)-2-oxopyrrolidin-3-yl)butan-2-yl)-4,4-dimethylpentanamide;
(2*S*)-2-((*S*)-2-benzyl-3,3,3-trifluoropropanamido)-*N*-(4-(cyclopropylamino)-3,4-dioxo-1-((*S*)-2-oxopyrrolidin-3-yl)butan-2-yl)-4,4-dimethylpentanamide;
(1*S*,2*S*)-2-(4-chlorophenyl)-*N*-((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)cyclopropane-1-carboxamide;
(2*S*)-*N*-(4-(cyclopropylamino)-3,4-dioxo-1-((*S*)-2-oxopyrrolidin-3-yl)butan-2-yl)-2-(2-(2,4-dichlorophenoxy)acetamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*S*)-2-(2,4-dichlorophenoxy)propanamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*R*)-2-(2,4-dichlorophenoxy)propanamido)-4,4-dimethylpentanamide;
or a salt thereof.

The compounds of the invention may be presented in the form of a prodrug. By "prodrug" is meant for example any compound that is converted *in vivo* into a biologically active compound of the invention. For example, some prodrugs are esters or phosphate esters of the active compound (e.g., a physiologically acceptable metabolically labile ester). During metabolism, the ester group (-C(=O)OR) or phosphate ester group (P(=O)(OH)₂-OR) is cleaved to yield the active drug. Such esters may be formed by esterification, for example, of a hydroxyl group present in the parent compound with, where appropriate, prior protection of any other reactive groups present in the parent compound, followed by deprotection if required. Other functionality present in the active compound, for example an amide group or amino group, can be used to form a prodrug. Also, some prodrugs are activated enzymatically to yield the active compound, or a compound which, upon further chemical reaction, yields the active compound (for example, as in ADEPT, GDEPT, LIDEPT, etc.). For example, the prodrug may be a sugar derivative or other glycoside conjugate, or may be an amino acid ester derivative.

Accordingly, described herein is a prodrug of a compound as defined herein wherein the compound contains a functional group which is convertible under physiological conditions to form a hydroxyl group, amide group or amino group. Suitable prodrugs may for example include compounds where group Z is functionalised with a group which is cleaved *in vivo* to release the active compound.

For example, in prodrugs of the compounds, Z may be a group: 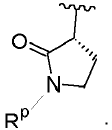 wherein R^{P} is any group which may be cleaved *in vivo* to afford a compound where Z is: 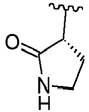 R^{P} can be selected from:

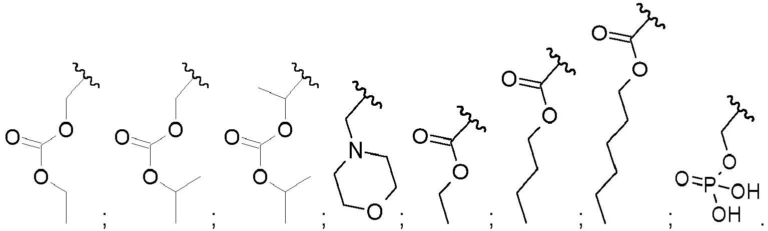

For example, in prodrugs of the compounds, Z may be selected from:

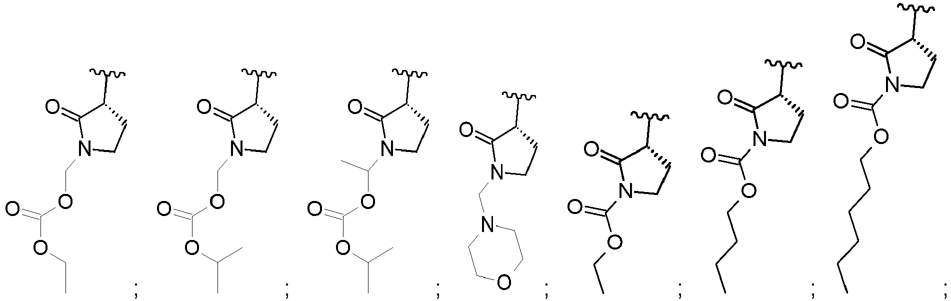

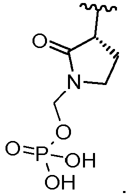

Further embodiments include the use of a compound of the invention or a salt thereof or a pharmaceutical composition comprising a compound of the invention as a SARS-CoV-2: Mpro inhibitor. Compounds of the present invention may be used as SARS-CoV-2: Mpro inhibitors. Compounds of the present invention may be used in the treatment of SARS-CoV-2 or a disease or disorder associated with SARS-CoV-2: Mpro. Compounds of the present invention may be useful in preventing death or complications arising due to chronic underlying conditions or comorbidities in patients infected with SARS-CoV-2. Such chronic underlying conditions or comorbidities may include for example hypertension, obesity, chronic lung conditions (TB, asthma and cystic fibrosis), diabetes and cardiovascular conditions (coronary heart disease, congenital heart disease and heart failure). Compounds of the present invention may be used in the manufacture of medicaments. The compounds or medicaments may be for use in treating, preventing, ameliorating, controlling or reducing the risk of SARS-CoV-2 and diseases or disorders in which SARS-CoV-2: Mpro is involved. The compounds or medicaments may be for use in treating, preventing, ameliorating, controlling or reducing the risk of chronic underlying conditions or comorbidities in patients infected with SARS-CoV-2.

Compounds of the present invention may be for use as a single agent or in combination with one or more additional pharmaceutical agents. Compounds of the present invention may be useful in the treatment of SARS-CoV-2 or conditions or symptoms related thereto.

As provided herein, compounds or salts thereof described herein and compositions described herein may be administered with an agent to treat any of the diseases and disorders disclosed herein.

### DEFINITIONS

In this application, the following definitions apply, unless indicated otherwise.

Any references to methods of treatment in the subsequent paragraphs of this description are to be interpreted as references to the compounds, pharmaceutical compositions and medicaments of the present invention for use in method of treatment of the human (or animal) body by therapy (or diagnosis).

The term "SARS-CoV-2: Mpro inhibitor" as used herein refers to any compound which binds to and modulates the function of SARS-CoV-2: Mpro.

The term "treatment", in relation to the uses of any of the compounds described herein, including those of Formula (1b) is used to describe any form of intervention where a compound is administered to a subject suffering from, or at risk of suffering from, or potentially at risk of suffering from the disease or disorder in question. Thus, the term "treatment" covers both preventative (prophylactic) treatment and treatment where measurable or detectable symptoms of the disease or disorder are being displayed.

The term "effective therapeutic amount" (for example in relation to methods of treatment of a disease or condition) refers to an amount of the compound which is effective to produce a desired therapeutic effect. For example, if the condition is pain, then the effective therapeutic amount is an amount sufficient to provide a desired level of pain relief. The desired level of pain relief may be, for example, complete removal of the pain or a reduction in the severity of the pain.

Terms such as "bicyclic", "hydrocarbon", "heterocyclic", "carbocyclic", "alkyl", "cycloalkyl" and "halo" are all used in their conventional sense (e.g. as defined in the IUPAC Gold Book), unless indicated otherwise. "optionally substituted" as applied to any group means that the said group may if desired be substituted with one or more substituents, which may be the same or different.

To the extent that any of the compounds described have chiral centres, the present invention extends to all optical isomers of such compounds, whether in the form of racemates or resolved enantiomers. The invention described herein relates to all crystal forms, solvates and hydrates of any of the disclosed compounds however so prepared. To the extent that any of the compounds disclosed herein have acid or basic centres such as carboxylates or amino groups, then all salt forms of said compounds are included herein. In the case of pharmaceutical uses, the salt should be seen as being a pharmaceutically acceptable salt.

Salts or pharmaceutically acceptable salts that may be mentioned include acid addition salts and base addition salts. Such salts may be formed by conventional means, for example by reaction of a free acid or a free base form of a compound with one or more equivalents of an appropriate acid or base, optionally in a solvent, or in a medium in which the salt is insoluble, followed by removal of said solvent, or said medium, using standard techniques (e.g. *in vacuo,* by freeze-drying or by filtration). Salts may also be prepared by exchanging a counter-ion of a compound in the form of a salt with another counter-ion, for example using a suitable ion exchange resin.

Examples of pharmaceutically acceptable salts include acid addition salts derived from mineral acids and organic acids, and salts derived from metals such as sodium, magnesium, potassium and calcium.

Examples of acid addition salts include acid addition salts formed with acetic, 2,2-dichloroacetic, adipic, alginic, aryl sulfonic acids (e.g. benzenesulfonic, naphthalene-2-sulfonic, naphthalene-1,5-disulfonic and *p*-toluenesulfonic), ascorbic (e.g. L-ascorbic), L-aspartic, benzoic, 4-acetamidobenzoic, butanoic, (+) camphoric, camphor-sulfonic, (+)-(1S)-camphor-10-sulfonic, capric, caproic, caprylic, cinnamic, citric, cyclamic, dodecylsulfuric, ethane-1,2-disulfonic, ethanesulfonic, 2-hydroxyethanesulfonic, formic, fumaric, galactaric, gentisic, glucoheptonic, gluconic (e.g. D-gluconic), glucuronic (e.g. D-glucuronic), glutamic (e.g. L-glutamic), α-oxoglutaric, glycolic, hippuric, hydrobromic, hydrochloric, hydriodic, isethionic, lactic (e.g. (+)-L-lactic and (±)-DL-lactic), lactobionic, maleic, malic (e.g. (-)-L-malic), malonic, (±)-DL-mandelic, metaphosphoric, methanesulfonic, 1-hydroxy-2-naphthoic, nicotinic, nitric, oleic, orotic, oxalic, palmitic, pamoic, phosphoric, propionic, L-pyroglutamic, salicylic, 4-amino-salicylic, sebacic, stearic, succinic, sulfuric, tannic, tartaric (e.g.(+)-L-tartaric), thiocyanic, undecylenic and valeric acids.

Also encompassed are any solvates of the compounds and their salts. Preferred solvates are solvates formed by the incorporation into the solid state structure (e.g. crystal structure) of the compounds of the invention of molecules of a non-toxic pharmaceutically acceptable solvent (referred to below as the solvating solvent). Examples of such solvents include water, alcohols (such as ethanol, isopropanol and butanol) and dimethylsulfoxide. Solvates can be prepared by recrystallising the compounds of the invention with a solvent or mixture of solvents containing the solvating solvent. Whether or not a solvate has been formed in any given instance can be determined by subjecting crystals of the compound to analysis using well known and standard techniques such as thermogravimetric analysis (TGA), differential scanning calorimetry (DSC) and X-ray crystallography.

The solvates can be stoichiometric or non-stoichiometric solvates. Particular solvates may be hydrates, and examples of hydrates include hemihydrates, monohydrates and dihydrates. For a more detailed discussion of solvates and the methods used to make and characterise them, see Bryn et al, Solid-State Chemistry of Drugs, Second Edition, published by SSCI, Inc of West Lafayette, IN, USA, 1999, ISBN 0-967-06710-3.

The term "pharmaceutical composition" in the context of this invention means a composition comprising an active agent and comprising additionally one or more pharmaceutically acceptable carriers. The composition may further contain ingredients selected from, for example, diluents, adjuvants, excipients, vehicles, preserving agents, fillers, disintegrating agents, wetting agents, emulsifying agents, suspending agents, sweetening agents, flavouring agents, perfuming agents, antibacterial agents, antifungal agents, lubricating agents and dispersing agents, depending on the nature of the mode of administration and dosage forms. The compositions may take the form, for example, of tablets, dragees, powders, elixirs, syrups, liquid preparations including suspensions, sprays, inhalants, tablets, lozenges, emulsions, solutions, cachets, granules, capsules and suppositories, as well as liquid preparations for injections, including liposome preparations.

The compounds of the invention may contain one or more isotopic substitutions, and a reference to a particular element includes within its scope all isotopes of the element. For example, a reference to hydrogen includes within its scope ¹H, ²H (D), and ³H (T). Similarly, references to carbon and oxygen include within their scope respectively ¹²C, ¹³C and ¹⁴C and ¹⁶O and ¹⁸O. In an analogous manner, a reference to a particular functional group also includes within its scope isotopic variations, unless the context indicates otherwise. For example, a reference to an alkyl group such as an ethyl group or an alkoxy group such as a methoxy group also covers variations in which one or more of the hydrogen atoms in the group is in the form of a deuterium or tritium isotope, e.g. as in an ethyl group in which all five hydrogen atoms are in the deuterium isotopic form (a perdeuteroethyl group) or a methoxy group in which all three hydrogen atoms are in the deuterium isotopic form (a trideuteromethoxy group). The isotopes may be radioactive or non-radioactive.

Therapeutic dosages may be varied depending upon the requirements of the patient, the severity of the condition being treated, and the compound being employed. Determination of the proper dosage for a particular situation is within the skill of the art. Generally, treatment is initiated with the smaller dosages which are less than the optimum dose of the compound. Thereafter the dosage is increased by small increments until the optimum effect under the circumstances is reached. For convenience, the total daily dosage may be divided and administered in portions during the day if desired.

The magnitude of an effective dose of a compound will, of course, vary with the nature of the severity of the condition to be treated and with the particular compound and its route of administration. The selection of appropriate dosages is within the ability of one of ordinary skill in this art, without undue burden. In general, the daily dose range may be from about 10 µg to about 30 mg per kg body weight of a human and non-human animal, preferably from about 50 µg to about 30 mg per kg of body weight of a human and non-human animal, for example from about 50 µg to about 10 mg per kg of body weight of a human and non-human animal, for example from about 100 µg to about 30 mg per kg of body weight of a human and non-human animal, for example from about 100 µg to about 10 mg per kg of body weight of a human and non-human animal and most preferably from about 100 µg to about 1 mg per kg of body weight of a human and non-human animal.

### PHARMACEUTICAL FORMULATIONS

While it is possible for the active compound to be administered alone, it is preferable to present it as a pharmaceutical composition (e.g. formulation).

Accordingly, in some embodiments of the invention, there is provided a pharmaceutical composition comprising at least one compound of the invention together with at least one pharmaceutically acceptable excipient.

The pharmaceutically acceptable excipient(s) can be selected from, for example, carriers (e.g. a solid, liquid or semi-solid carrier), adjuvants, diluents (e.g. solid diluents such as fillers or bulking agents; and liquid diluents such as solvents and co-solvents), granulating agents, binders, flow aids, coating agents, release-controlling agents (e.g. release retarding or delaying polymers or waxes), binding agents, disintegrants, buffering agents, lubricants, preservatives, anti-fungal and antibacterial agents, antioxidants, buffering agents, tonicity-adjusting agents, thickening agents, flavouring agents, sweeteners, pigments, plasticizers, taste masking agents, stabilisers or any other excipients conventionally used in pharmaceutical compositions.

The term "pharmaceutically acceptable" as used herein means compounds, materials, compositions, and/or dosage forms which are, within the scope of sound medical judgment, suitable for use in contact with the tissues of a subject (e.g. a human subject) without excessive toxicity, irritation, allergic response, or other problem or complication, commensurate with a reasonable benefit/risk ratio. Each excipient must also be "acceptable" in the sense of being compatible with the other ingredients of the formulation.

Pharmaceutical compositions containing compounds of the invention can be formulated in accordance with known techniques, see for example, Remington's Pharmaceutical Sciences, Mack Publishing Company, Easton, PA, USA. The pharmaceutical compositions can be in any form suitable for oral, parenteral, intravenous, intramuscular, intrathecal, subcutaneous, topical, intranasal, intrabronchial, sublingual, buccal, ophthalmic, otic, rectal, intra-vaginal, or transdermal administration.

Pharmaceutical dosage forms suitable for oral administration include tablets (coated or uncoated), capsules (hard or soft shell), caplets, pills, lozenges, syrups, solutions, powders, granules, elixirs and suspensions, sublingual tablets, wafers or patches such as buccal patches.

The composition may be a tablet composition or a capsule composition. Tablet compositions can contain a unit dosage of active compound together with an inert diluent or carrier such as a sugar or sugar alcohol, eg; lactose, sucrose, sorbitol or mannitol; and/or a non-sugar derived diluent such as sodium carbonate, calcium phosphate, calcium carbonate, or a cellulose or derivative thereof such as microcrystalline cellulose (MCC), methyl cellulose, ethyl cellulose, hydroxypropyl methyl cellulose, and starches such as corn starch. Tablets may also contain such standard ingredients as binding and granulating agents such as polyvinylpyrrolidone, disintegrants (e.g. swellable crosslinked polymers such as crosslinked carboxymethylcellulose), lubricating agents (e.g. stearates), preservatives (e.g. parabens), antioxidants (e.g. BHT), buffering agents (for example phosphate or citrate buffers), and effervescent agents such as citrate/bicarbonate mixtures. Such excipients are well known and do not need to be discussed in detail here.

Tablets may be designed to release the drug either upon contact with stomach fluids (immediate release tablets) or to release in a controlled manner (controlled release tablets) over a prolonged period of time or with a specific region of the GI tract.

The pharmaceutical compositions typically comprise from approximately 1% (w/w) to approximately 95%, preferably% (w/w) active ingredient and from 99% (w/w) to 5% (w/w) of a pharmaceutically acceptable excipient (for example as defined above) or combination of such excipients. Preferably, the compositions comprise from approximately 20% (w/w) to approximately 90% (w/w) active ingredient and from 80% (w/w) to 10% of a pharmaceutically excipient or combination of excipients. The pharmaceutical compositions comprise from approximately 1% to approximately 95%, preferably from approximately 20% to approximately 90%, active ingredient. Pharmaceutical compositions according to the invention may be, for example, in unit dose form, such as in the form of ampoules, vials, suppositories, pre-filled syringes, dragées, powders, tablets or capsules.

Tablets and capsules may contain, for example, 0-20% disintegrants, 0-5% lubricants, 0-5% flow aids and/or 0-99% (w/w) fillers/ or bulking agents (depending on drug dose). They may also contain 0-10% (w/w) polymer binders, 0-5% (w/w) antioxidants, 0-5% (w/w) pigments. Slow release tablets would in addition typically contain 0-99% (w/w) release-controlling (e.g. delaying) polymers (depending on dose). The film coats of the tablet or capsule typically contain 0-10% (w/w) polymers, 0-3% (w/w) pigments, and/or 0-2% (w/w) plasticizers.

The composition may be a parenteral composition. Parenteral formulations typically contain 0-20% (w/w) buffers, 0-50% (w/w) cosolvents, and/or 0-99% (w/w) Water for Injection (WFI) (depending on dose and if freeze dried). Formulations for intramuscular depots may also contain 0-99% (w/w) oils.

The pharmaceutical formulations may be presented to a patient in "patient packs" containing an entire course of treatment in a single package, usually a blister pack.

The compounds of the invention will generally be presented in unit dosage form and, as such, will typically contain sufficient compound to provide a desired level of biological activity. For example, a formulation may contain from 1 nanogram to 2 grams of active ingredient, e.g. from 1 nanogram to 2 milligrams of active ingredient. Within these ranges, particular sub-ranges of compound are 0.1 milligrams to 2 grams of active ingredient (more usually from 10 milligrams to 1 gram, e.g. 50 milligrams to 500 milligrams), or 1 microgram to 20 milligrams (for example 1 microgram to 10 milligrams, e.g. 0.1 milligrams to 2 milligrams of active ingredient).

For oral compositions, a unit dosage form may contain from 1 milligram to 2 grams, more typically 10 milligrams to 1 gram, for example 50 milligrams to 1 gram, e.g. 100 milligrams to 1 gram, of active compound.

The active compound will be administered to a patient in need thereof (for example a human or animal patient) in an amount sufficient to achieve the desired therapeutic effect (effective amount). The precise amounts of compound administered may be determined by a supervising physician in accordance with standard procedures.

The compounds may be administered alongside other agents, for example other agents used in treating subjects with SARS-CoV-2. The compounds may be co-administered with HIV drugs which are known to block cypP450 mediated metabolism, such as ritonavir or a combination of lopinavir/ritonavir.

### EXAMPLES

The invention will now be illustrated, but not limited, by reference to the following examples shown in Table 1.

**TABLE 1**

| | | |
|---|---|---|
| 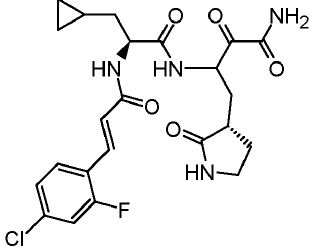 Example 1 | 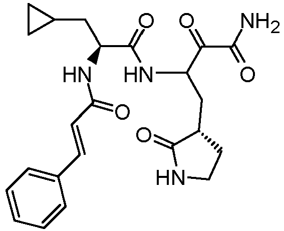 Example 2 | 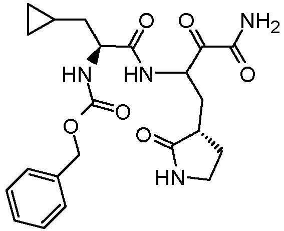 Example 3 |
| 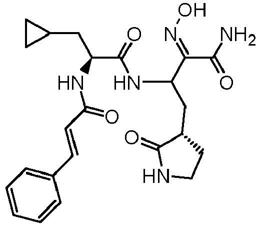 Example 4 | 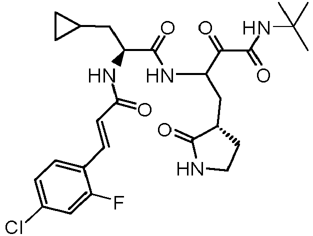 Example 5 | 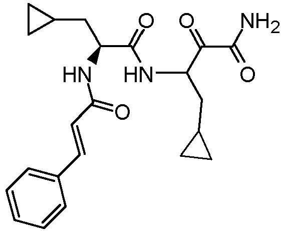 Example 6 |
| 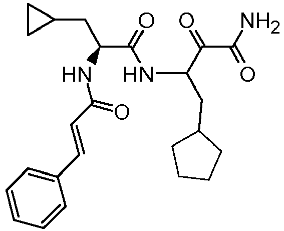 Example 7 | 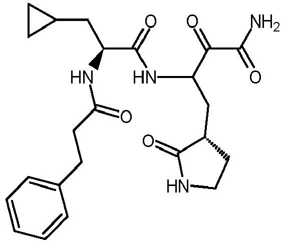 Example 8 | 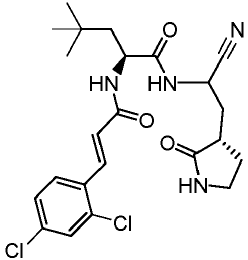 Example 9 |
| 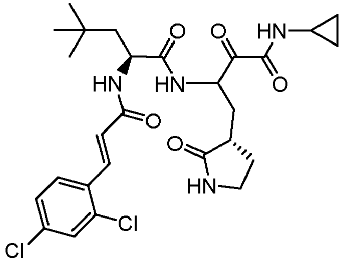 Example 10 | 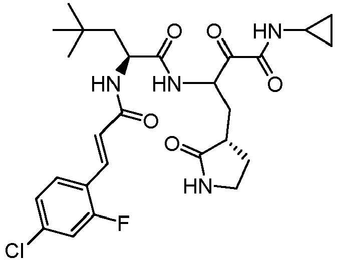 Example 11 | 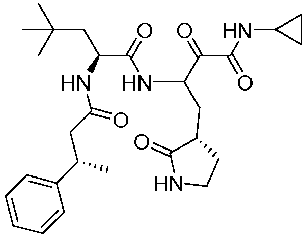 Example 12 |
| 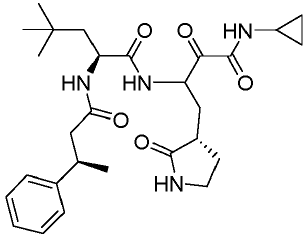 Example 13 | 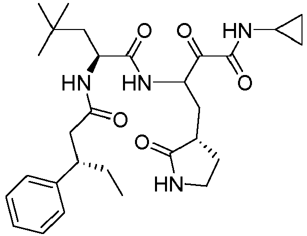 Example 14 | 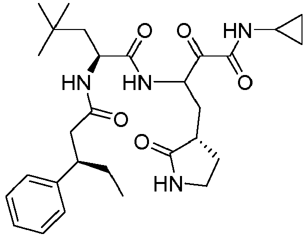 Example 15 |
| 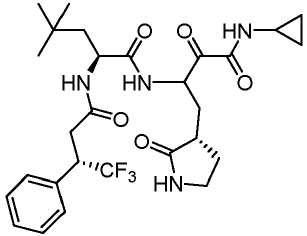 Example 16 | 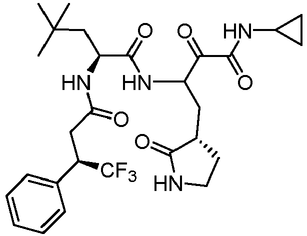 Example 17 | 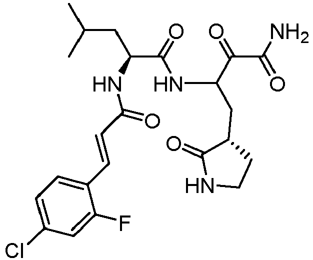 Example 18 |
| 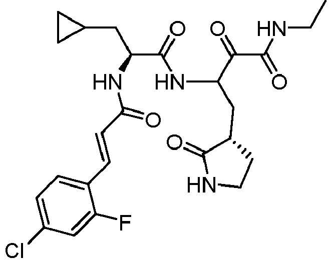 Example 19 | 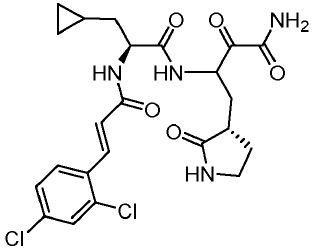 Example 20 | 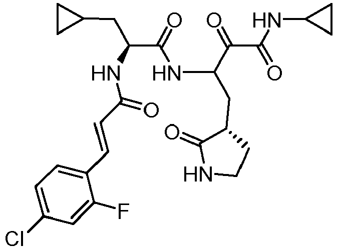 Example 21 |
| 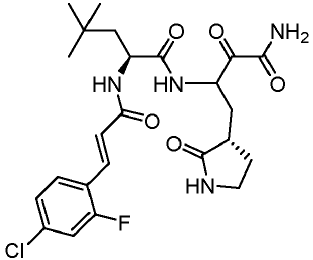 Example 22 | 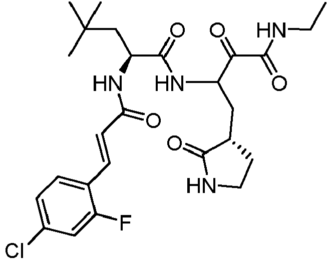 Example 23 | 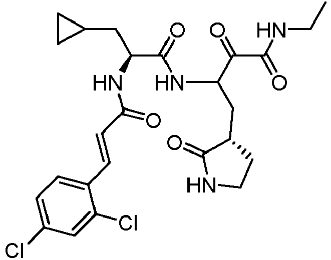 Example 24 |
| 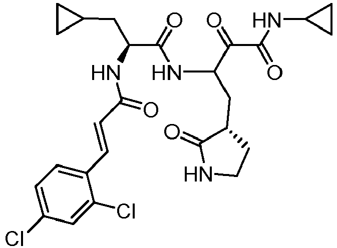 Example 25 | 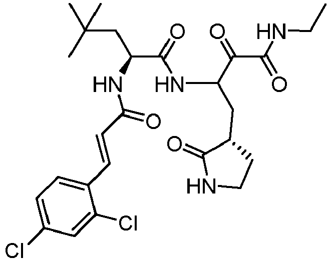 Example 26 | 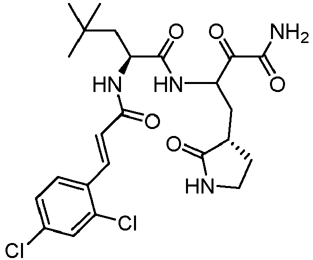 Example 27 |
| 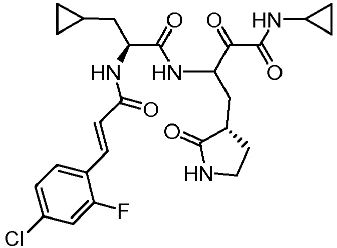 Example 28 | 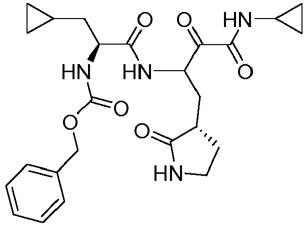 Example 29 | 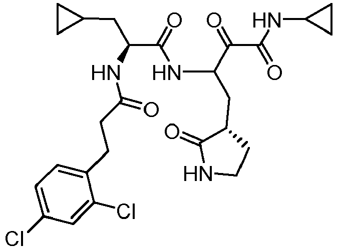 Example 30 |
| 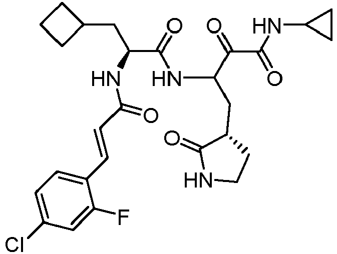 Example 31 | 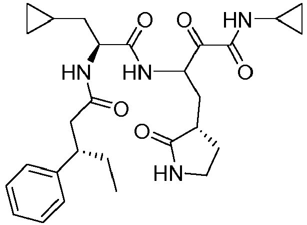 Example 32 | 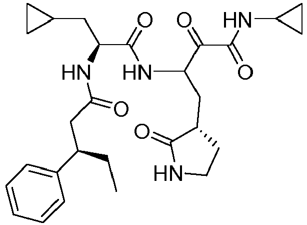 Example 33 |
| 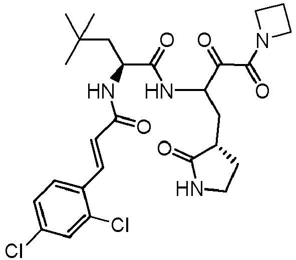 Example 34 | 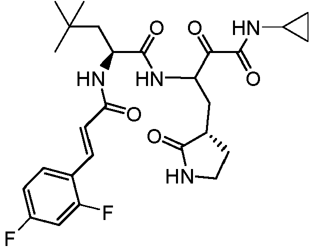 Example 35 | 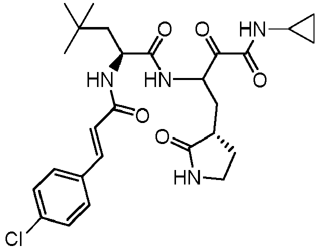 Example 36 |
| 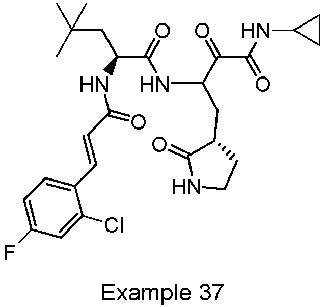 | 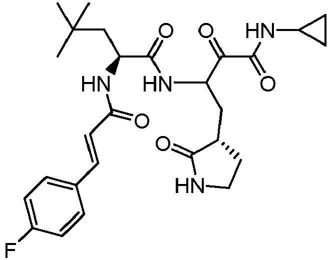 Example 38 | 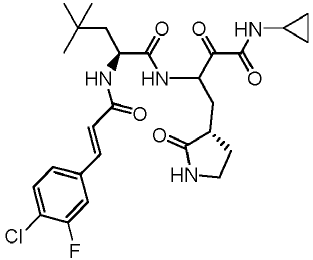 Example 39 |
| 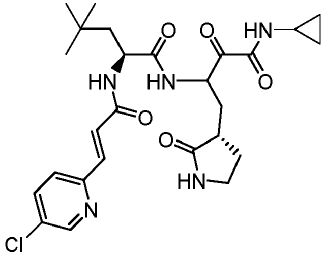 Example 40 | 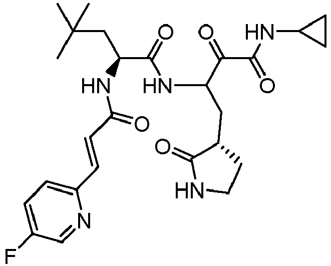 Example 41 | 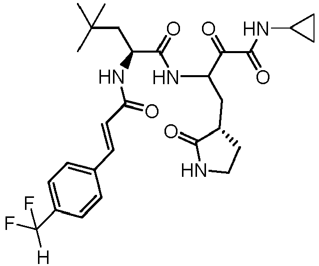 Example 42 |
| 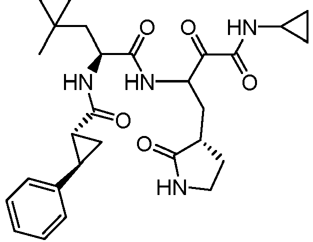 Example 43 | 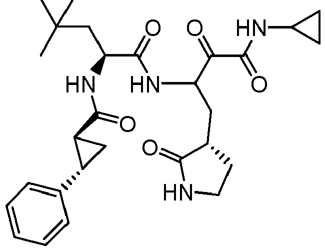 Example 44 | 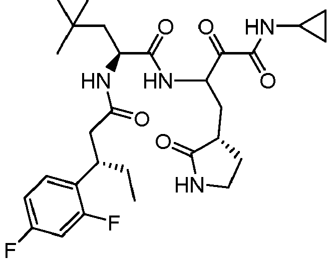 Example 45 |
| 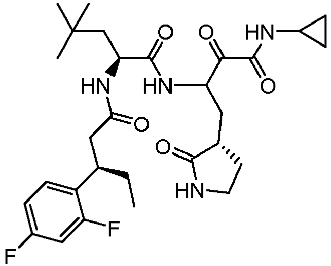 Example 46 | 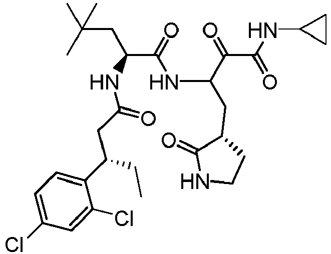 Example 47 | 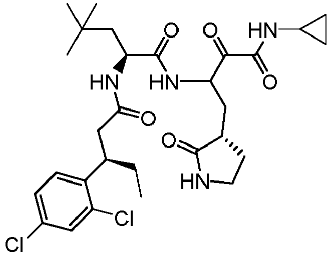 Example 48 |
| 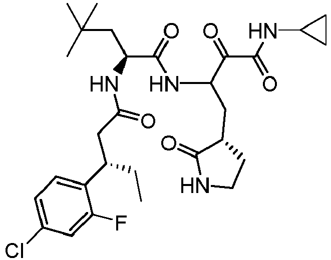 Example 49 | 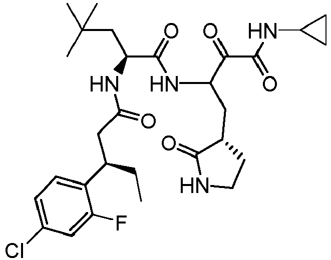 Example 50 | 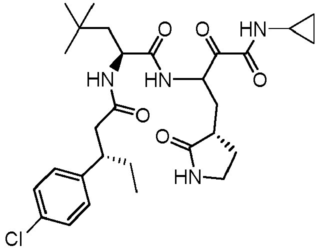 Example 51 |
| 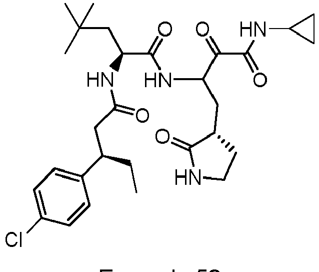 Example 52 | 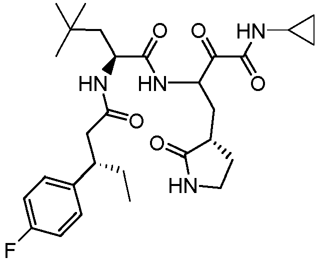 Example 53 | 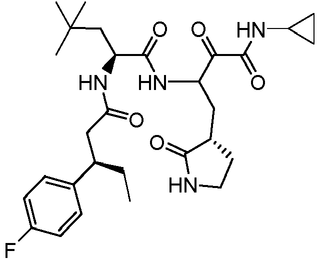 Example 54 |
| 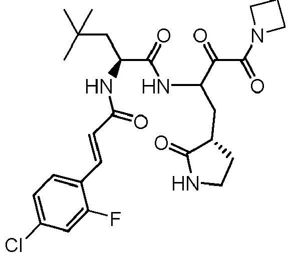 Example 55 | 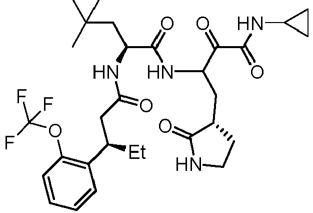 Example 56 | 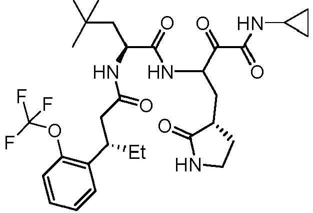 Example 57 |
| 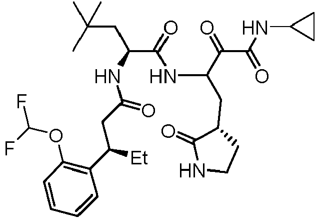 Example 58 | 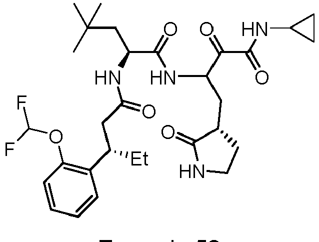 Example 59 | 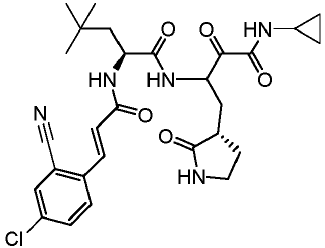 Example 60 |
| 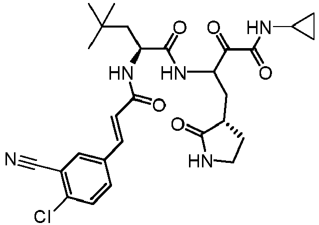 Example 61 | 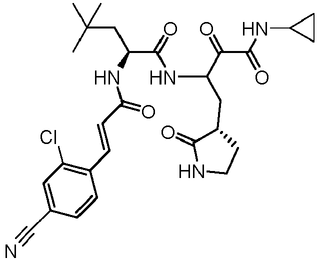 Example 62 | 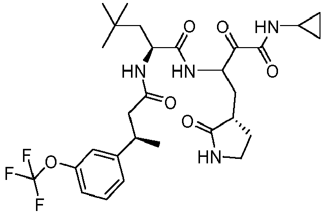 Example 63 |
| 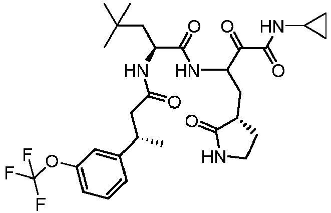 Example 64 | 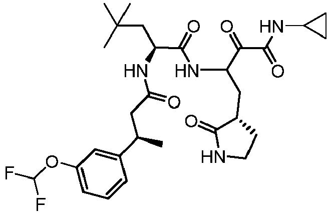 Example 65 | 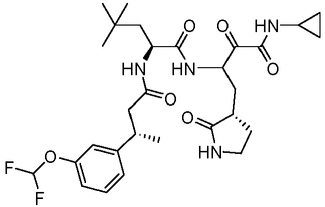 Example 66 |
| 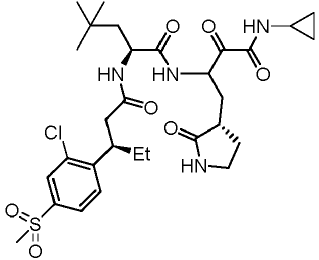 Example 67 | 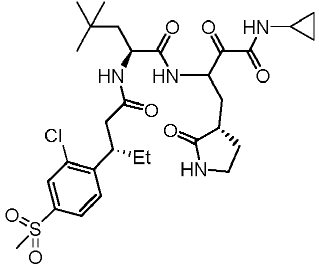 Example 68 | 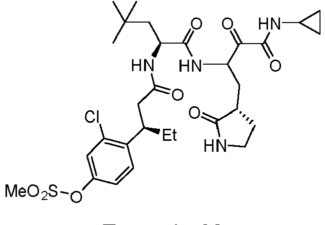 Example 69 |
| 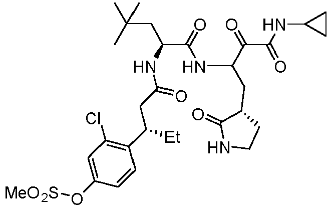 Example 70 | 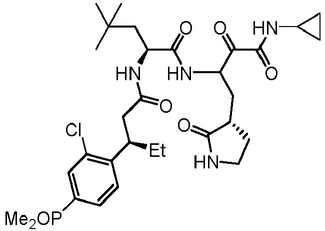 Example 71 | 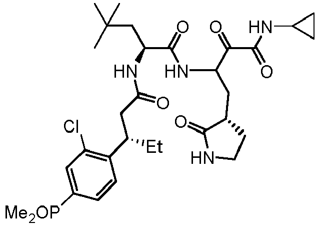 Example 72 |
| 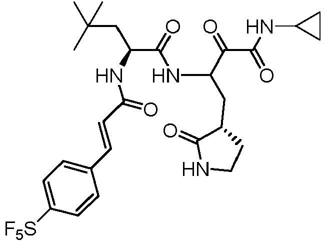 Example 73 | 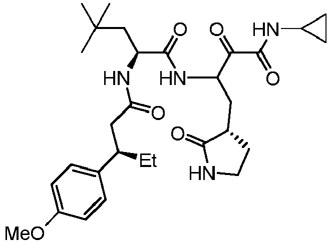 Example 74 | 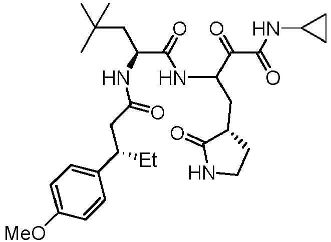 Example 75 |
| 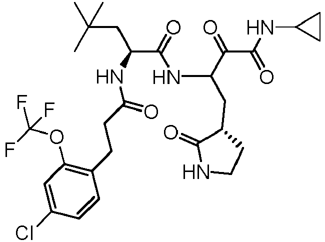 Example 76 | 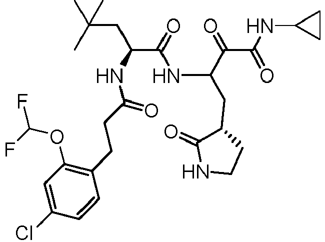 Example 77 | 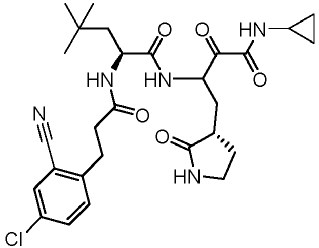 Example 78 |
| 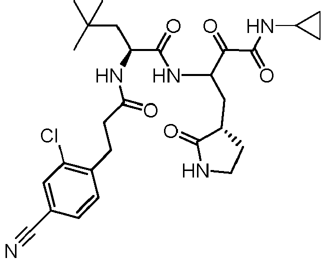 Example 79 | 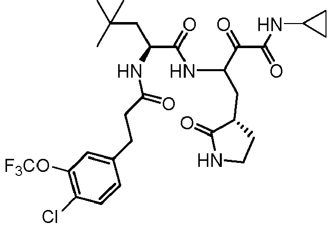 Example 80 | 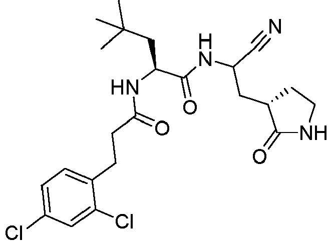 Example 81 |
| 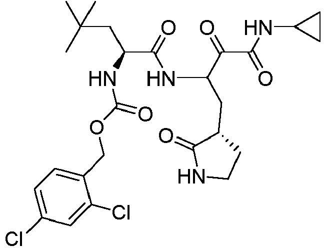 Example 82 | 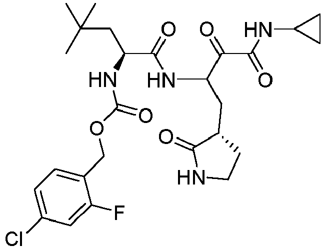 Example 83 | 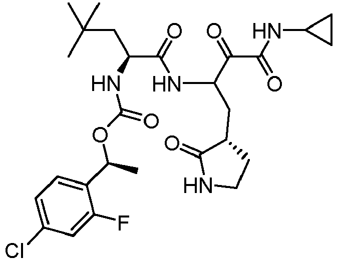 Example 84 |
| 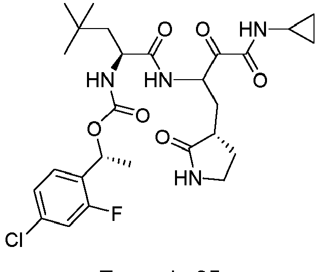 Example 85 | 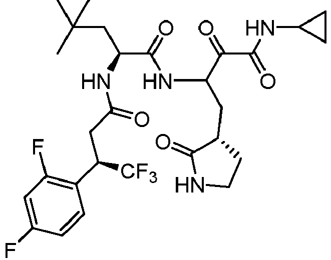 Example 86 | 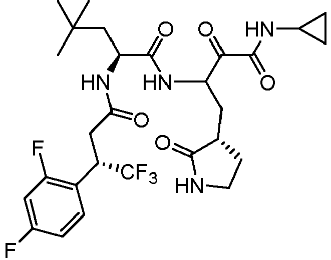 Example 87 |
| 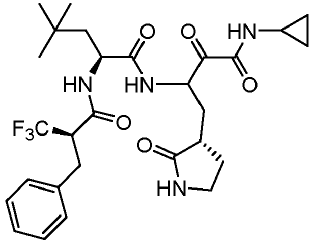 Example 88 | 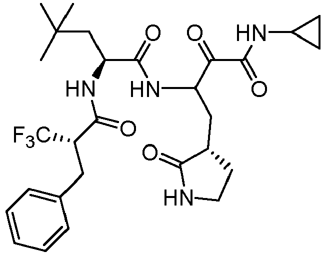 Example 89 | 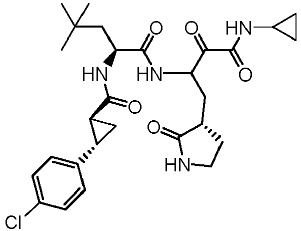 Example 90 |
| 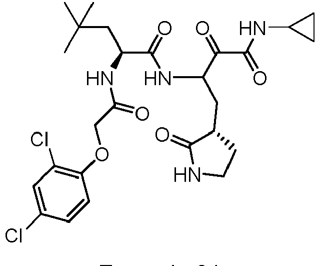 Example 91 | 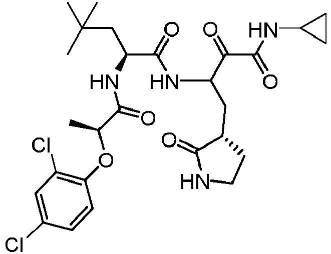 Example 92 | 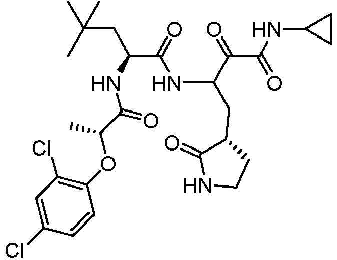 Example 93 |

### PREPARATION OF THE COMPOUNDS OF THE INVENTION

Some compounds of the invention and derivatives or synthetic intermediates thereof can be prepared in accordance with synthetic methods known to the skilled person. In some embodiments, the invention provides a process for the preparation of a compound of the invention. Certain compounds of the invention may be prepared according to the methods described below.

### PREPARATION OF THE COMPOUNDS OF THE INVENTION

Compounds of the invention may be prepared by routes including those in Figure 1. Details of many of the standard transformations such as those in the routes below and others which could be used to perform the same transformations can be found in standard reference textbooks such as "Organic Synthesis", M. B. Smith, McGraw-Hill (1994) or "Advanced Organic Chemistry", 4th edition, J. March, John Wiley & Sons (1992).

Ester derivatives of α-amino acids, for example methyl ester α-amino acid derivatives, are commercially available, or can be prepared by standard transformations which will be known to those skilled in the art, including transformations that are detailed in the following Synthesis of Intermediates and Synthesis of Examples sections. Ester derivatives of α-amino acids can be coupled with carboxylic acids to give the corresponding amide derivative (Route 1, step i). The amide coupling reaction conditions will typically use a coupling agent or agents, for example propylphosphonic anhydride (T3P) or HATU, with a suitable base such as DIPEA or Et₃N, in a solvent such as DCM or DMF, typically at room temperature. Alternatively, ester derivatives of α-amino acids can be coupled with carbamoyl chlorides, for example benzyl carbonochloridate, with a suitable base such as DIPEA, in a solvent such as DCM, typically at room temperature, to form a carbamate derivative. The ester functionality present in the product of the amide or carbamate formation can then be hydrolysed under acidic or basic conditions, for example using lithium hydroxide monohydrate in a solvent such as THF, MeOH, 1,4-dioxane or H₂O, or a mixture of these solvents, typically at room temperature (Route 1, step ii). The hydrolysis generates a carboxylic acid that can then be reacted with an ester derivative of an α-amino acid, under amide coupling conditions such as those above (Route 1, step iii). The ester functionality can be reduced to a primary alcohol using standard reduction conditions, for example the use of a reducing agent such as sodium borohydride, in a suitable solvent such as THF or MeOH and a combination of solvents, typically at room temperature (Route 1, step iv). Oxidation of the primary alcohol to an aldehyde can be performed using an oxidising agent such as Dess-Martin Periodinane (DMP) in a suitable solvent such as DCM, THF, or DMSO, or a mixture of these solvents, typically at room temperature (Route 1, step v). The aldehyde product can be reacted with acetone cyanohydrin, in the presence of a suitable base such as Et₃N, in a suitable solvent such as DCM, typically at room temperature to give a 1-cyano-1-hydroxy derivative (Route 1, step vi). The cyano group can be hydrolysed under standard conditions, such as using aqueous hydrogen peroxide in the presence of a base such as potassium carbonate, in a suitable solvent such as DMSO, typically at room temperature. A final oxidation step, using conditions such as those detailed above (Route 1, step v) can be used to synthesize compounds of the invention (Route 1, step viii).

Further compounds of the invention can be synthesised from other compounds of the invention. For example, the ketone group in the keto-amide functionality of compounds can be reacted with nucleophiles, for example hydroxylamine hydrochloride, in the presence of a suitable base such as potassium carbonate, in a suitable solvent such as ethanol, at an elevated temperature, for example 70 °C (Route 2).

In a further route, compounds of the invention may be prepared from aldehyde intermediates resulting from Route 1, Steps i) to v) inclusive. The aldehyde may be reacted with an isocyanate, for example an alkyl isocyanate, in the presence of acetic acid, in a suitable solvent such as DCM, typically at room temperature (Route 2, step i). The acetate group of the 1-acetoxy-1-alkylamide product of this step can be hydrolysed under conditions such as those described above (Route 1, step ii) to yield a 1-hydroxy-1-alkylamide product (Route 3, step ii). A final oxidation step, using conditions such as those detailed above (Route 1, step v) can be used to synthesize compounds of the invention (Route 1, step viii).

In a further route, compounds of the invention may be prepared from aldehyde intermediates resulting from Route 1, Steps i) to v) inclusive. The aldehyde may be reacted with hydroxylamine hydrochloride, in the presence of a base such as potassium carbonate, in a suitable solvent such as ethanol, at an elevated temperature, for example 70 °C (Route 4, step i). Dehydration of the resulting oxime, for example using a dehydrating agent such as methyl *N*-(triethylammoniumsulfonyl)carbamate (Burgess reagent) can be used to synthesize compounds of the invention (Route 4, step ii).

### General procedures

Where no preparative routes are included, the relevant intermediate is commercially available. Commercial reagents were utilized without further purification. Room temperature (rt) refers to approximately 20-27°C. ¹H NMR spectra were recorded at 300 or 400 MHz on Bruker instruments. Chemical shift values are expressed in parts per million (ppm), i.e. (δ)-values, relative to tetramethylsilane. The following abbreviations are used for the multiplicity of the NMR signals: s=singlet, br=broad, d=doublet, t=triplet, q=quartet, quin=quintet, h=heptet, dd=doublet of doublets, dt=double of triplets, m=multiplet. Coupling constants are listed as J values, measured in Hz. NMR and mass spectroscopy results were corrected to account for background peaks. TLC for monitoring reactions refers to TLC run using silica gel as a stationary phase.

LCMS experiments were carried out under the following conditions. Instruments: Agilent Technologies 1290 Infinity II Series LC / 6125 Quadrupole MSD SL (Methods A-E inclusive, H and K), ELSD detector (Polymer Laboratories PL-ELS 2100 ICE) used for UV inactive compounds in Method A; Columns: Waters XBridge C8 3.5µm, 4.6x50 mm (Method A), Atlantis dC18 5µm, 4.6x50mm (Method B), Zorbax XDB C18 5µm, 4.6x50mm (Method C), Zorbax extend C18 5µm, 4.6x50mm (Method D), XBridge C8 3.5µm, 4.6x50mm (Method E), Acquity BEH C18 1.7µm, 2.1x50mm (Method H), Xselect CSH C18 5µm, 4.6x50mm, CSH (Charged Surface Hybrid) (Method K); Gradient [time (min)/solvent B in A (%)]: 0.0/5, 2.5/95, 4.0/95, 4.5/5, 6.0/5 (Solvent A=0.1% TFA in H₂O:MeCN (95:5); Solvent B=0.1% TFA in MeCN (Method A)), 0.0/5, 2.5/95, 4.0/95, 4.5/5, 6.0/5 (Solvent A=0.1% HCO₂H in H₂O:MeCN (95:5); Solvent B=MeCN (Method B)), 0.0/5, 2.5/95, 4.0/95, 4.5/5, 6.0/5 (Solvent A=0.1% HCO₂H in H₂O:MeCN (95:5); Solvent B=MeCN (Method C)), 0.0/10, 4.0/95, 5.0/95, 5.0/10, 6.0/10 (Solvent A=770.1 mg of NH₄OAc in 1 L Milli-Q Water; Solvent B=MeCN (Method D)), 0.0/10, 4.0/95, 5.0/95, 5.5/10, 7.0/10 (Solvent A=790.1 mg of NH₄HCO₃ in 1 L Milli-Q Water; Solvent B=MeCN (Method E)), 0.0/5, 0.25/5, 2.5/100, 3.0/100, 3.1/5, 4.0/5 (Solvent A=770.1 mg of NH₄OAc in 1 L Milli-Q Water; Solvent B=MeCN (Method H)), 0.0/05, 2.5/95, 4.0/95, 4.5/05, 6.0/05 (Solvent A=0.1%v/v TFA in Milli-Q water; Solvent B=0.1% TFA in MeCN) (Method K)); Injection volume typically 1 µL; UV detection 210 to 400 nm (Methods A-E inclusive and H and K); column temperature 25°C; Flow rate 1.5 mL/min (Method A, B, C, K), 1.2 mL/min (Method D, E) or 0.8 mL/min (Method H). LCMS data in the experimental section are given in the format: Mass ion, retention time.

Analytical SFC experiments were carried out under the following conditions. Instrument: PIC-10 (Manufacturer PIC Solution Inc); Column: YMC Cellulose-SC 5µm, 4.5x250mm; Mobile phase: 60% CO₂/40% Co-solvent (0.5% Isopropyl Amine in Methanol); Injection Volume 15 µL; Flow Rate 4 mL/min; Column temperature 35°C.

Mass directed preparative HPLC was carried out under the following conditions. Instrument: Agilent Technologies 1260 Infinity II Series LC. Method B: Column: X Bridge C8 (19 mm X 150mm), 5µm; Gradient [time (min)/solvent B in A (%)]: 0.0/10, 15/95, 18/95, 19/10, 21/10 (Solvent A=0.1% HCO₂H in H₂O; Solvent B=MeCN). Method F:Column: Xselect CSH C18 (19 mm X 150 mm), 5µm; Gradient [time (min)/solvent B in A (%)]: 0.0/10, 10/50, 12/100, 16/100, 18/10, 20/10 (Solvent A=0.1% HCO₂H in H₂O; Solvent B=MeCN).

Preparative HPLC was carried out under the following conditions. Method B: Instrument: Agilent Technologies 1260 Infinity II Series LC; Column: YMC Exrs C18, 5µm, 30x150mm; Gradient [time (min)/solvent B in A (%)]: 0.0/10, 20/95, 23/95, 24/10, 26/10 (Solvent A 0.1% HCO₂H in H₂O, Solvent B=MeCN).

### Abbreviations

- DCM: = dichloromethane
- DMF: = *N*,*N*-dimethylformamide
- DMP: = Dess-Martin Periodinane
- DIPEA: = *N*,*N*-diisopropylethylamine
- DMSO: = dimethylsulfoxide
- EtOAc: = ethyl acetate
- h: = hour(s)
- HATU: = 1-[bis(dimethylamino)methylene]-1*H*-1,2,3-triazolo[4,5-*b*]pyridinium 3-oxide hexafluorophosphate
- HPLC: = high performance liquid chromatography
- L: = litre
- LC: = liquid chromatography
- LiHMDS: = lithium bis(trimethylsilyl)amide
- MeCN: = acetonitrile
- min: = minute(s)
- MS: = mass spectrometry
- NMR: = nuclear magnetic resonance
- Rt or RT: = room temperature
- SFC: = supercritical fluid chromatography
- T3P: = propylphosphonic anhydride
- THF: = tetrahydrofuran
- TLC: = thin layer chromatography
- UPLC: = ultra performance liquid chromatography

Prefixes *n-, s-, i-, t-* and *tert-* have their usual meanings: normal, secondary, iso, and tertiary.

### SYNTHESIS OF INTERMEDIATES

### Intermediate 1: methyl (S)-2-amino-3-((S)-2-oxopyrrolidin-3-yl)propanoate hydrochloride

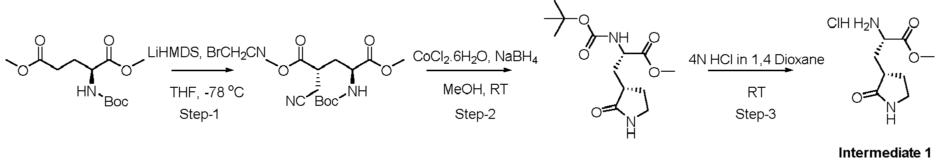

Step 1: To a suspension of dimethyl (*tert*-butoxycarbonyl)-L-glutamate (CAS No. 59279-60-6, 250 g, 0.908 mol) in THF (2500 mL) at-78 °C, LiHMDS (1M in THF, 1997 mL, 1.997 mol) was added dropwise and the resulting reaction mixture was stirred at -78 °C for 1 h followed by the addition of bromoacetonitrile (76.5 mL, 1.086 mol) at the same temperature. The resulting reaction mixture was stirred at -78 °C for 3 h; after the complete disappearance of the starting material as monitored by TLC, the reaction mixture was quenched by the addition of saturated aqueous ammonium chloride solution (2 L) at -78 °C and then warmed to rt. The resulting mixture was extracted with EtOAc (2 x 2000 mL) and the combined organic layers were dried over anhydrous Na₂SO₄ and concentrated *in vacuo.* Purification by gradient flash column chromatography using silica gel (100-200 mesh), eluting with 0-20% EtOAc in petroleum-ether, yielded (2*S*,4*R*)-2-((*tert*-butoxycarbonyl)amino)-4-(cyanomethyl)pentanedioate as a pale-yellow gum (207 g, 0.65 mol). The reaction was performed in 5 batches on 50 g scale.

**LCMS (Method C)**: m/z 337.1 (M+Na), at 1.93 min.

**¹H NMR**: (300 MHz, DMSO-*d*₆) δ 7.36 (d, J=8.1 Hz, 1H), 4.10-4.05 (m, 1H), 3.66 (s, 3H), 3.63 (s, 3H), 2.87-2.73 (m, 3H), 2.05-1.99 (m, 2 H), 1.39 (s, 9H).

Step 2: To a stirred suspension of (2S,4R)-2-((tert-butoxycarbonyl)amino)-4-(cyanomethyl)pentanedioate (207 g, 0.659 mol) in MeOH (2 L) at 0 °C was added CoCl₂.6H₂O (78.1 g, 0.329 mol) followed by the portion wise addition of NaBH₄ (149.6 g, 3.955 mol) during 30 min and the resulting reaction mixture was stirred at rt for 15 h. After completion of reaction as monitored by TLC, the reaction mixture was concentrated *in vacuo,* 20% MeOH in DCM (3 L) and H₂O (2 L) were added, and the mixture stirred for 10 min. The resulting suspension was filtered through celite and rinsed with 20% MeOH in DCM (2 L). The filtrate was transferred to a separating funnel, the organic layer was separated, washed with brine solution (2 L), dried over anhydrous Na₂SO₄ and concentrated *in vacuo.* Purification by gradient flash column chromatography using silica gel (100-200 mesh), eluting with 0-100% EtOAc in petroleum ether yielded methyl (S)-2-((tert-butoxycarbonyl)amino)-3-((*S*)-2-oxopyrrolidin-3-yl)propanoate as a pale yellow solid (92 g, 0.32 mol).

**LCMS (Method C):** m/z 187.2 (M+H-100), at 1.34 min.

**¹H NMR:** (300 MHz, DMSO-*d*₆) δ 7.64 (s, 1H), 7.42 (d, J=7.8 Hz, 1H), 4.06-4.02 (m, 1H), 3.69 (s, 3H), 3.16-3.08 (m, 2H), 2.32-2.21 (m, 1H), 2.16-2.11 (m, 1H), 2.03-1.93 (m, 1H), 1.69-1.66 (m, 2 H), 1.33 (s, 9H).

Step 3: To a stirred solution of methyl (S)-2-((tert-butoxycarbonyl)amino)-3-((S)-2-oxopyrrolidin-3-yl)propanoate (10 g, 0.034 mol) in 1,4-dioxane (50 mL) at 0 °C was added 4 N HCl in dioxane (100 mL) and the resulting reaction mixture was stirred at rt for 3h. After completion of reaction as monitored by TLC, the supernatant layer was decanted from the reaction mixture and the gummy solid material remaining was dried *in vacuo* to yield methyl (S)-2-amino-3-((S)-2-oxopyrrolidin-3-yl)propanoate hydrochloride (Intermediate 1, 8.5 g crude, 0.038 mol) as a yellow gum which was used without further purification.

**LCMS (Method E):** m/z 187.3 (M+H), at 0.82-1.19 min.

**¹H NMR:** (400 MHz, DMSO-*d*₆) δ 8.67 (s, 3H), 7.98 (s, 1H), 4.19 (t, J=5.2 Hz, 1H), 3.76 (s, 3H), 3.21-3.18 (m, 2H), 2.53-2.51 (m, 1 H), 2.28-2.27 (m, 1H), 2.05-2.03 (m, 1H), 1.90-1.89 (m, 1 H), 1.70-1.65 (m, 1H).

### SYNTHESIS OF EXAMPLES

### Example 1: 3-((S)-2-((E)-3-(4-chloro-2-fluorophenyl)acrylamido)-3-cyclopropylpropanamido)-2-oxo-4-((S)-2-oxopyrrolidin-3-yl)butanamide

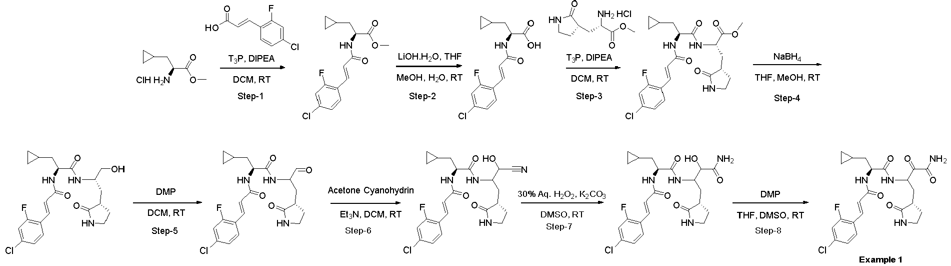

Step 1: To a stirred solution of methyl (*S*)-2-amino-3-cyclopropylpropanoate hydrochloride (CAS No. 206438-31-5, 17 g, 0.096 mol) and (*E*)-3-(4-chloro-2-fluorophenyl)acrylic acid (CAS No. 312693-55-3, 16 g, 0.08 mol) in DCM (10 mL), was added DIPEA (59 mL, 0.32 mol) followed by the addition of T3P in 50% EtOAc (101.7 mL, 0.16 mol). The resulting reaction mixture was stirred at rt for 16 h. After completion of the reaction as monitored by TLC, the reaction mass was partitioned between DCM (1 L) and 10% aqueous NaHCOs solution (1 L). The organic layer was separated, dried over anhydrous Na₂SO₄, and concentrated *in vacuo.* Purification by gradient flash column chromatography (Biotage-Isolera) using a 100 g silica SNAP cartridge (230-400 mesh), eluting with 0-40% EtOAc in petroleum-ether yielded methyl (*S*,*E*)-2-(3-(4-chloro-2-fluorophenyl)acrylamido)-3-cyclopropylpropanoate as a white solid (18 g, 0.055 mol).

**LCMS (Method D):** m/z 326.1 (M+H), at 3.07 min.

**¹H NMR:** (400 MHz, DMSO-*d*₆) δ 8.67 (d, J=7.2 Hz, 1H), 7.70 (t, J=8.4 Hz, 1H), 7.56-7.53 (m, 1H), 7.47 (d, J=16.0 Hz, 1H), 7.39-7.36 (m, 1H), 6.86 (d, J=15.6 Hz, 1H), 4.45-4.44 (m, 1H), 3.65 (s, 3H), 1.69-1.67 (m, 1H), 1.59-1.57 (m, 1H), 0.82-0.78 (m, 1H), 0.44-0.40 (m, 2H), 0.15-0.04 (m, 2H).

Step 2: To a stirred solution of methyl (*S*,*E*)-2-(3-(4-chloro-2-fluorophenyl)acrylamido)-3-cyclopropylpropanoate (18 g, 0.055 mol) in THF (200 mL), MeOH (20 mL) and H₂O (50 mL) at rt was added LiOH.H₂O (6.92 g, 0.165 mol). The resulting reaction mixture was stirred at rt for 2 h. After completion of the reaction as monitored by TLC, the reaction mixture was concentrated *in vacuo* and the residue obtained was dissolved in H₂O (300 mL), acidified to approximately pH 5 using 1.5 N HCl and then extracted with 10% MeOH in DCM (2 x 500 mL). The organic layers were separated, combined and dried over anhydrous Na₂SO₄ and concentrated *in vacuo* to yield (S,E)-2-(3-(4-chloro-2-fluorophenyl)acrylamido)-3-cyclopropylpropanoic acid as an off-white solid (13 g, 0.044 mol).

**LCMS (Method C):** m/z 312.0 (M+H), at 2.06 min.

**¹H NMR:** (400 MHz, DMSO-*d*₆) δ 7.83 (d, J=6.0 Hz, 1H), 7.77-7.73 (m, 1H), 7.51 (d, J=10.8 Hz, 1H), 7.41-7.34 (m, 2H), 7.03 (d, J=15.6 Hz, 1H), 4.04-4.00 (m, 1H), 1.65-1.52 (m, 2H), 0.82-0.78 (m, 1H), 0.30-0.29 (m, 2H), 0.04-0.01 (m, 2H).

Step 3: To a stirred solution of (*S*,*E*)-2-(3-(4-chloro-2-fluorophenyl)acrylamido)-3-cyclopropylpropanoic acid (10 g, 0.0365 mol) and methyl (S)-2-amino-3-((S)-2-oxopyrrolidin-3-yl)propanoate hydrochloride (Intermediate 1, 8.5 g, 0.0365 mol) in DCM (150 mL), was added DIPEA (31.7 mL, 0.182 mol) followed by the addition of T3P (50% solution in EtOAc, 34.8 mL, 0.054 mol). The resulting reaction mixture was stirred at rt for 16 h. After completion of the reaction as monitored by TLC, the reaction mixture was partitioned between DCM (500 mL) and 10% aqueous NaHCOs solution (500 mL). The organic layer was separated, washed with 10% aqueous NaHCO₃ solution (500 mL), dried over anhydrous Na₂SO₄ and concentrated *in vacuo.* Purification by gradient flash column chromatography (Biotage-Isolera) using a 100 g silica SNAP cartridge (230-400 mesh), eluting with 0-90% EtOAc in petroleum-ether yielded methyl (S)-2-((S)-2-((E)-3-(4-chloro-2-fluorophenyl)acrylamido)-3-cyclopropylpropanamido)-3-((S)-2-oxopyrrolidin-3-yl)propanoate as an off-white solid (9.2 g, 0.019 mmol).

**LCMS (Method C):** m/z 480.2 (M+H), at 1.81 min.

**¹H NMR:** (300 MHz, DMSO-*d*₆) δ 8.58 (d, J=7.8 Hz, 1H), 8.42 (d, J=7.8 Hz, 1H), 7.71-7.65 (m, 2H), 7.56-7.52 (m, 1H), 7.46-7.41 (m, 1H), 7.39-7.36 (m, 1H), 6.89 (d, J=15.9 Hz, 1H), 4.50-4.35 (m, 2H), 3.62 (s, 3H), 3.15-3.09 (m, 2H), 2.28-2.28 (m, 1H), 2.09-2.03 (m, 2H), 1.65-1.49 (m, 4H), 0.80-0.79 (m, 1H), 0.39-0.38 (m, 2H), 0.10-0.09 (m, 2H).

Step 4: To a stirred solution of methyl (*S*)-2-((*S*)-2-((*E*)-3-(4-chloro-2-fluorophenyl)acrylamido)-3-cyclopropylpropanamido)-3-((*S*)-2-oxopyrrolidin-3-yl)propanoate (9.2 g, 0.019 mol) in THF (100 mL), was added MeOH (30 mL) followed by the addition of sodium borohydride (1.45 g, 0.038 mol) portion wise at 0 °C. The resulting reaction mixture was stirred at rt for 16 h. After the completion of reaction as monitored by TLC, the reaction mixture was partitioned between 20% MeOH in DCM (500 mL) and H₂O (500 mL). The organic layer was separated, dried over anhydrous Na₂SO₄ and concentrated *in vacuo.* Purification by gradient flash column chromatography (Biotage-Isolera) using a 100 g silica SNAP cartridge (230-400 mesh), eluting with 0-6% MeOH in DCM yielded (*E*)-3-(4-chloro-2-fluorophenyl)-*N-*((*S*)-3-cyclopropyl-1-(((*S*)-1-hydroxy-3-((*S*)-2-oxopyrrolidin-3-yl)propan-2-yl)amino)-1-oxopropan-2-yl)acrylamide as an off-white solid (6.4 g, 0.014 mol).

**LCMS (Method C):** m/z 452.2 (M+H), at 1.58 min.

**¹H NMR:** (300 MHz, DMSO-*d*₆) δ 8.39 (d, J=8.1 Hz, 1H), 7.83 (d, J=8.7 Hz, 1H), 7.68-7.65 (m, 1H), 7.55-7.46 (m, 2H), 7.41-7.36 (m, 2H), 6.90 (d, J=15.9 Hz, 1H), 4.65 (t, J=5.7 Hz, 1H), 4.44-4.42 (m, 1H), 3.82-3.72 (m, 1H), 3.26-3.06 (m, 4H), 2.27-2.18 (m, 2H), 1.78 (t, J=11.7 Hz, 1H), 1.60-1.57 (m, 2H), 1.47-1.39 (m, 2H), 0.80-0.79 (m, 1H), 0.38-0.37 (m, 2H), 0.11-0.08 (m, 2H).

Step 5: To a stirred solution of (*E*)-3-(4-chloro-2-fluorophenyl)-*N*-((*S*)-3-cyclopropyl-1-(((*S*)-1-hydroxy-3-((*S*)-2-oxopyrrolidin-3-yl)propan-2-yl)amino)-1-oxopropan-2-yl)acrylamide (4.5 g, 9.95 mmol) in DCM (100 mL), was added Dess-Martin periodinane (6.34 g, 14.9 mmol) portion wise at rt. The resulting reaction mixture was stirred at rt for 1 h. After completion of the reaction as monitored by TLC, the reaction mixture was quenched with 10% aqueous NaHCOs solution (200 mL) and extracted with DCM (2 x 200 mL). The combined organic layers were dried over anhydrous Na₂SO₄ and concentrated *in vacuo.* Purification by gradient flash column chromatography (Biotage-Isolera) using a 100 g silica SNAP cartridge (230-400 mesh), eluting with 0-6% MeOH in DCM yielded (*E*)-3-(4-chloro-2-fluorophenyl)-*N*-((2*S*)-3-cyclopropyl-1-oxo-1-((1-oxo-3-((S)-2-oxopyrrolidin-3-yl)propan-2-yl)amino)propan-2-yl)acrylamide as an off-white solid (4.4 g, 9.77 mmol) as a mixture of two diastereomers.
**LCMS (Method E):** m/z 450.0 (M+H), at 1.78-1.96 min.
**Chiral SFC analysis (Method 2):** 1.73 & 2.01 min (48.8% & 45.0%)
**¹H NMR:** (300 MHz, DMSO-*d*₆) δ 9.43 (s, 0.5H), 8.63 (d, J=7.6 Hz, 0.5H), 8.50 (d, J=7.6 Hz, 0.5H), 8.38 (d, J=7.6 Hz, 0.5H), 7.69-7.65 (m, 2H), 7.56-7.53 (m, 2H), 7.47-7.37 (m, 2H), 6.93-6.88 (m, 1H), 4.51-4.41 (m, 1H), 4.38-4.31 (m, 0.5H), 4.20-4.17 (m, 0.5H), 3.21-3.12 (m, 3H), 2.25-2.15 (m, 1.5H), 1.91-1.85 (m, 1.5H), 1.69-1.51 (m, 3H), 0.81-0.70 (m, 1H), 0.42-0.39 (m, 2H), 0.10-0.09 (m, 2H).

Step 6: To a stirred solution of (*E*)-3-(4-chloro-2-fluorophenyl)-*N*-((2*S*)-3-cyclopropyl-1-oxo-1-((1-oxo-3-((*S*)-2-oxopyrrolidin-3-yl)propan-2-yl)amino)propan-2-yl)acrylamide (4.4 g, 9.7 mmol) in DCM (40 mL) at rt was added triethylamine (2.75 mL, 19.5 mmol) followed by acetone cyanohydrin (1.66 mL, 19.5 mmol). The resulting reaction mixture was stirred at rt for 16 h. After completion of the reaction as monitored by TLC, the reaction mixture was concentrated *in vacuo.* Purification by gradient flash column chromatography (Biotage-Isolera) using a 50 g silica SNAP cartridge (230-400 mesh), eluting with 0-5% MeOH in DCM yielded (*E*)-3-(4-chloro-2-fluorophenyl)-*N*-((2*S*)-1-((1-cyano-1-hydroxy-3-((*S*)-2-oxopyrrolidin-3-yl)propan-2-yl)amino)-3-cyclopropyl-1-oxopropan-2-yl)acrylamide as an off-white solid (2.7 g, 5.66 mmol). **LCMS (Method E):** m/z 450.1 (M-CN), at 1.79-1.96 min.

**¹H NMR:** (300 MHz, DMSO-*d*₆) δ 8.37-8.07 (m, 2H), 7.60-7.24 (m, 5H), 6.82-6.74 (m, 1H), 6.64-6.55 (m, 1H), 4.46-4.22 (m, 2H), 3.91-3.85 (m, 1H), 3.15-2.93 (m, 2H), 2.33-2.23 (m, 1H), 2.02-1.71 (m, 2H), 1.52-1.31 (m, 4H), 0.71-0.54 (m, 1H), 0.29-0.27 (m, 2H), 0.15-0.09 (m, 2H).

Step 7: To a stirred solution of (*E*)-3-(4-chloro-2-fluorophenyl)-*N*-((2S)-1-((1-cyano-1-hydroxy-3-((S)-2-oxopyrrolidin-3-yl)propan-2-yl)amino)-3-cyclopropyl-1-oxopropan-2-yl)acrylamide (2.7 g, 5.6 mmol) in DMSO (30 mL) was added K₂CO₃ (2.34 g, 16.9 mmol) followed by the addition of 30% aqueous H₂O₂ solution (6.4 mL, 56.6 mmol). The resulting reaction mixture was stirred at rt for 16 h. After completion of the reaction as monitored by TLC, the reaction mixture was partitioned between 10% MeOH in DCM (100 mL) and brine (100 mL). The aqueous layer was further extracted with 10% MeOH in DCM (3 x 200 mL), and the combined organic layers were dried over anhydrous Na₂SO₄ and concentrated *in vacuo.* Purification by gradient flash column chromatography (Biotage-Isolera) using a 100 g silica SNAP cartridge (230-400 mesh), eluting with 0-10% MeOH in DCM yielded 3-((*S*)-2-((*E*)-3-(4-chloro-2-fluorophenyl)acrylamido)-3-cyclopropylpropanamido)-2-hydroxy-4-((*S*)-2-oxopyrrolidin-3-yl)butanamide as an off-white solid (960 mg, 1.93 mmol).

**LCMS (Method E):** m/z 495.0 (M+H), at 1.78 min.

**¹H NMR:** (300 MHz, DMSO-*d*₆) δ 8.43 (d, J=7.6 Hz, 1H), 7.71-7.23 (m, 8H), 6.90 (d, J=21.2 Hz, 1H), 5.66 (d, J=7.6 Hz, 1H), 4.49-4.41 (m, 1H), 4.11-4.09 (m, 1H), 3.85-3.83 (m, 1H), 3.12-3.04 (m, 2H), 2.27-2.12 (m, 4H), 1.60-1.37 (m, 3H), 0.80-0.79 (m, 1H), 0.38-0.37 (m, 2H), 0.11-0.08 (m, 2H).

Step 8: To a stirred solution of 3-((*S*)-2-((*E*)-3-(4-chloro-2-fluorophenyl)acrylamido)-3-cyclopropylpropanamido)-2-hydroxy-4-((*S*)-2-oxopyrrolidin-3-yl)butanamide (960 mg, 1.93 mmol) in DCM (40 mL) and DMSO (10 mL) at rt was added Dess-Martin periodinane (1.22 g, 2.895 mmol). The resulting reaction mixture was stirred at rt for 1 h. After completion of the reaction as monitored by TLC, the reaction mixture was quenched with 10% aqueous NaHCOs solution (200 mL) and extracted with EtOAc (200 mL). The organic layer was separated, washed with 10% aqueous NaHCOs solution (150 mL), dried over anhydrous Na₂SO₄ and concentrated *in vacuo.* Purification by gradient flash column chromatography (Biotage-Isolera) using a 50 g silica SNAP cartridge (230-400 mesh), eluting with 0-10% MeOH in DCM yielded 3-((S)-2-((E)-3-(4-chloro-2-fluorophenyl)acrylamido)-3-cyclopropylpropanamido)-2-oxo-4-((S)-2-oxopyrrolidin-3-yl)butanamide as an off-white solid (Example 1, 400 mg, 0.811 mmol).

**LCMS (Method C):** m/z 493.1 (M+H), at 1.21-1.44 min.

**¹H NMR:** (400 MHz, DMSO-*d*₆) δ 8.61 (d, J=7.6 Hz, 0.5H), 8.43-8.41 (m, 1H), 8.04-8.09 (m, 0.6H), 7.79-7.71 (m, 0.6H), 7.69-7.66 (m, 1.7H), 7.56-7.42 (m, 3.9H), 7.39-7.36 (m, 0.9H), 6.89 (d, J=16.0 Hz, 1H), 6.12 (d, J=40.8 Hz, 0.7H), 5.15-5.05 (m, 0.5H), 4.61-4.45 (m, 1H), 4.05-4.01 (m, 0.5H), 3.15-3.12 (m, 2H), 2.26-2.16 (m, 2H), 1.82-1.53 (m, 5H), 0.79-0.73 (m, 1H), 0.40-0.36 (m, 2H), 0.13-0.09 (m, 2H).

### Example 2: 3-((S)-2-cinnamamido-3-cyclopropylpropanamido)-2-oxo-4-((S)-2-oxopyrrolidin-3-yl)butanamide

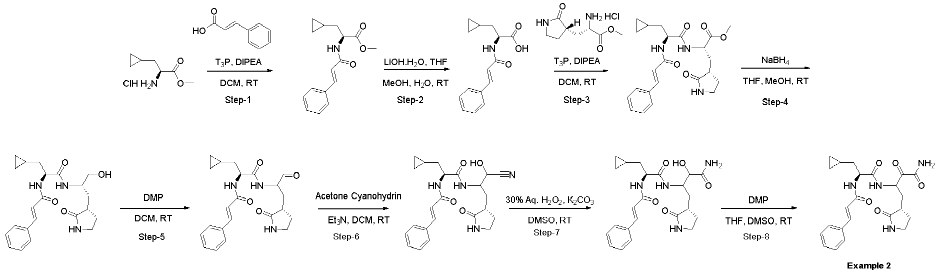

Step 1: To a stirred solution of methyl (*S*)-2-amino-3-cyclopropylpropanoate hydrochloride (CAS No. 206438-31-5, 3.34 g, 18.57 mmol) and cinnamic acid (CAS No. 140-10-3, 2.75 g, 18.57 mmol) in DCM (40 mL), was added DIPEA (14 mL, 77.35 mmol) followed by the addition of T3P in 50% EtOAc (14.7 mL, 23.20 mmol). The resulting reaction mixture was stirred at rt for 16 h. After completion of the reaction as monitored by TLC, the reaction mixture was partitioned between DCM (300 mL) and 10% aqueous NaHCOs solution (300 mL). The organic layer was separated, dried over anhydrous Na₂SO₄ and concentrated *in vacuo.* Purification by gradient flash column chromatography (Biotage-Isolera) using a 100 g silica SNAP cartridge (230-400 mesh), eluting with 0-40% EtOAc in petroleum-ether yielded methyl (*S*)-2-cinnamamido-3-cyclopropylpropanoate as an off-white solid (4.40 g, 16.1 mmol).

**LCMS (Method C):** m/z 274.1 (M+H), at 2.10 min.

**¹H NMR:** (300 MHz, DMSO-*d*₆) δ 8.52 (d, J=7.2 Hz, 1H), 7.58 (d, J=6.3 Hz, 2H), 7.48-7.39 (m, 4H), 6.75 (d, J=15.9 Hz, 1H), 4.42 (q, J=5.7 Hz, 1H), 3.64 (s, 3H), 1.71-1.54 (m, 2H), 0.82-0.77 (m, 1H), 0.43-0.41 (m, 2H), 0.16-0.03 (m, 2H).

Step 2: To a stirred solution of methyl (*S*)-2-cinnamamido-3-cyclopropylpropanoate (4.0 g, 14.63 mmol) in 1,4-Dioxane (50 mL), MeOH (10 mL) and H₂O (10 mL) at rt was added LiOH.H₂O (1.23 g, 29.26 mmol). The resulting reaction mixture was stirred at rt for 2 h. After completion of the reaction as monitored by TLC, the reaction mixture was concentrated *in vacuo.* The residue obtained was dissolved in H₂O (100 mL), acidified to approximately pH 5 using 1.5 N HCl and then extracted with 20% MeOH in DCM (150 mL). The organic layer was separated, dried over anhydrous Na₂SO₄ and concentrated *in vacuo* to yield (S)-2-cinnamamido-3-cyclopropylpropanoic acid as an off-white solid (3.30 g, 12.7 mmol).

**LCMS (Method B):** m/z 260.0 (M+H), at 2.10 min.

**¹H NMR:** (400 MHz, DMSO-*d*₆) δ 12.70 (s, 1H), 8.39 (d, J=8.0 Hz, 1H), 7.59-7.57 (m, 2H), 7.47-7.39 (m, 4H), 6.78 (d, J=15.6 Hz, 1H), 4.40 (t, J=2.8 Hz, 1H), 1.66-1.59 (m, 2H), 0.81-0.81 (m, 1H), 0.44-0.41 (m, 2H), 0.17-0.08 (m, 2H).

Steps 3-8: The title compound, Example 2, 3-((*S*)-2-cinnamamido-3-cyclopropylpropanamido)-2-oxo-4-((*S*)-2-oxopyrrolidin-3-yl)butanamide (25 mg, 0.079 mmol) was prepared from Step 2 product (3.30 g, 12.7 mmol) and Intermediate 1 (2.01 g, 10.7 mmol) over steps 3-8 using the procedures detailed for Example 1.

**LCMS (Method K):** m/z 441.2 (M+H), at 1.60-1.71 min.

**¹H NMR:** (400 MHz, DMSO-*d*₆) δ 8.60 (d, J=7.2 Hz, 0.5H), 8.26-8.24 (m, 1H), 8.03 (s, 0.5H), 7.78 (s, 0.5H), 7.68-7.51 (m, 3H), 7.45-7.38 (m, 4H), 7.30-7.21 (m, 1H), 6.82-6.77 (m, 1H), 6.16-6.07 (m, 0.5H), 5.15-5.05 (m, 0.5H), 4.61-4.45 (m, 1H), 4.05-4.01 (m, 0.5H), 3.15-3.12 (m, 2H), 2.19-2.12 (m, 2H), 1.95-1.53 (m, 5H), 0.81-0.70 (m, 1H), 0.42-0.37 (m, 2H), 0.14-0.09 (m, 2H).

### Example 3: benzyl ((2S)-1-((4-amino-3,4-dioxo-1-((S)-2-oxopyrrolidin-3-yl)butan-2-yl)amino)-3-cyclopropyl-1-oxopropan-2-yl)carbamate

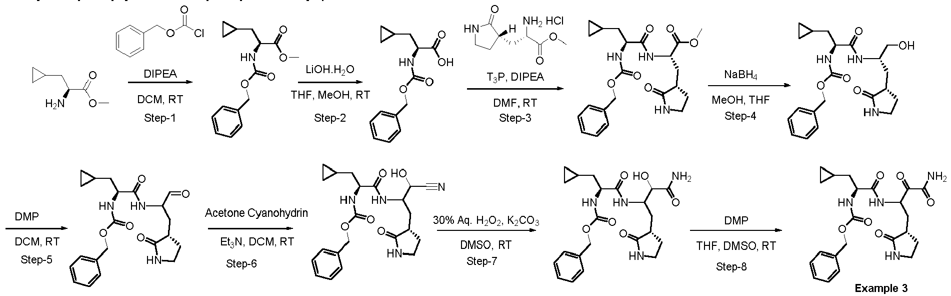

Step 1: To a stirred solution of methyl (*S*)-2-amino-3-cyclopropylpropanoate (CAS No. 732231-41-3, 1 g, 6.98 mmol) in DCM (15 mL), was added DIPEA (3.6 mL, 20.95 mmol) followed by the addition of benzyl carbonochloridate (1.42 g, 8.38 mmol) at 0 °C. The resulting reaction mixture was stirred at rt for 16 h. After completion of the reaction, as monitored by TLC, the reaction mass was partitioned between DCM (20 mL) and H₂O (50 mL). The organic layer was separated, washed with 10% aqueous NaHCOs solution (30 mL) and brine (30 mL), dried over anhydrous Na₂SO₄ and concentrated *in vacuo.* Purification by gradient flash column chromatography (Biotage-Isolera) using a 25 g silica SNAP cartridge (230-400 mesh), eluting with 0-20% EtOAc in petroleum-ether yielded methyl (*S*)-2-(((benzyloxy)carbonyl)amino)-3-cyclopropylpropanoate as an off-white solid (1.1 g, 3.96 mmol).

**LCMS (Method A):** m/z 278.2 (M+H), at 2.39 min.

**¹H NMR:** (400 MHz, DMSO-*d*₆) δ 7.40-7.30 (m, 6H), 5.17 (s, 2H), 4.50-4.49 (m, 1H), 3.63 (s, 3H), 1.65-1.61 (m, 1H), 1.48-1.46 (m, 1H), 0.78 (s, 1H), 0.42-0.37 (m, 2H), 0.13-0.10 (m, 1H), 0.02-0.01 (m, 1H)

Step 2: To a stirred solution of methyl (*S*)-2-(((benzyloxy)carbonyl)amino)-3-cyclopropylpropanoate (1.1 g, 3.96 mmol) in THF (10 mL) was added MeOH (2 mL) and H₂O (5 mL) followed by the addition of LiOH.H₂O (0.142 g, 5.94 mmol). The resulting reaction mixture was stirred at rt for 30 min. After completion of the reaction, as monitored by TLC, the reaction mixture was acidified to approximately pH 5 with 1.5 N HCl (5 mL) and partitioned between EtOAc (50 mL) and H₂O (50 mL). The organic layer was separated, dried over anhydrous Na₂SO₄ and concentrated *in vacuo.* Purification by gradient flash column chromatography (Biotage-Isolera) using a 25 g silica SNAP cartridge (230-400 mesh), eluting with 0-5% MeOH in DCM yielded methyl (*S*)-2-(((benzyloxy)carbonyl)amino)-3-cyclopropylpropanoate as an off-white solid (1.0 g, 3.79 mmol).

**LCMS (Method C):** m/z 264.1 (M+H), at 1.77 min.

**¹H NMR:** (400 MHz, DMSO-*d*₆) δ 7.58 (d, J=8.0 Hz, 1H), 7.40-7.22 (m, 6H), 5.13 (s, 2H), 4.03-3.97 (m, 1H), 1.63-1.59 (m, 1H), 1.50-1.47 (m, 1H), 0.79 (t, J=7.2 Hz, 1H), 0.42-0.36 (m, 2H), 0.16-0.01 (m, 2H)

Steps 3-8: The title compound, Example 3, benzyl ((2S)-1-((4-amino-3,4-dioxo-1-((S)-2-oxopyrrolidin-3-yl)butan-2-yl)amino)-3-cyclopropyl-1-oxopropan-2-yl)carbamate (5 mg, 0.015 mmol) was prepared from Step 2 product (1.0 g, 3.79 mmol) and Intermediate 1 (0.84 g, 4.55 mmol) over steps 3-8 using the procedures detailed for Example 1.

**LCMS (Method A):** m/z 445.1 (M+H), at 1.95-2.09 min.

¹H NMR: (400 MHz, DMSO-*d*₆) δ 8.43 (d, J=7.2 Hz, 0.5H), 8.01-7.96 (m, 1H), 7.77 (s, 0.5H), 7.65 (s, 0.5H), 7.59 (s, 0.5H), 7.49-7.47 (m, 1H), 7.37-7.31 (m, 5.5H), 7.04 (s, 0.5H), 5.08-5.03 (m, 2.5H), 4.31-4.28 (m, 0.5H), 4.15-4.05 (m, 1H), 3.15-3.05 (m, 2 H), 2.29-1.79 (m, 3H), 1.64-1.40 (m, 4H), 0.75-0.71 (m, 1H), 0.40-0.34 (m, 2H), 0.13-0.07 (m, 2H).

### Example 4: 3-((S)-2-((E)-3-(4-chloro-2-fluorophenyl)acrylamido)-3-cyclopropylpropanamido)-2-(hydroxyimino)-4-((S)-2-oxopyrrolidin-3-yl)butanamide

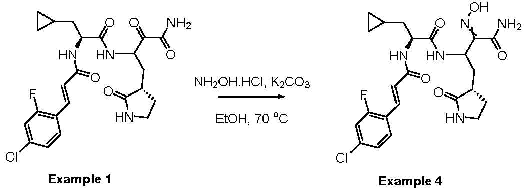

To a stirred solution of 3-((*S*)-2-((*E*)-3-(4-chloro-2-fluorophenyl)acrylamido)-3-cyclopropylpropanamido)-2-oxo-4-((*S*)-2-oxopyrrolidin-3-yl)butanamide (Example 1, 0.1 g, 0.20 mmol) in EtOH (10 mL), was added K₂CO₃ (55 mg, 0.4 mmol) and hydroxylamine hydrochloride (28 mg, 0.4 mmol) at rt. The resulting reaction mixture was stirred at 70 °C for 3 h. After completion of the reaction, as monitored by UPLC-MS, the reaction mixture was filtered and rinsed with EtOH (5 mL). The filtrate was concentrated *in vacuo* and purified by mass directed preparative HPLC (Method F) to yield the title compound, Example 4, 3-((*S*)-2-((*E*)-3-(4-chloro-2-fluorophenyl)acrylamido)-3-cyclopropylpropanamido)-2-(hydroxyimino)-4-((*S*)-2-oxopyrrolidin-3-yl)butanamide as a mixture of isomers (off-white solid, 10 mg, 0.019 mmol).

**LCMS (Method C):** m/z 508.0 (M+H), at 2.04 min.

**¹H NMR:** (400 MHz, DMSO-*d*₆) δ 11.98 (s, 1H), 8.51-8.50 (m, 1H), 8.02-8.00 (m, 1H), 7.70-7.68 (m, 1H), 7.59-7.56 (m, 2H), 7.54-7.31 (m, 4H), 6.88 (d, J=16.0 Hz, 1H), 5.47-5.45 (m, 1H), 4.42-4.39 (m, 1H), 3.15-3.08 (m, 2H), 2.26-2.23 (m, 3H), 1.55-1.51 (m, 4H), 0.79-0.74 (m, 1H), 0.39-0.35 (m, 2H), 0.12-0.10 (m, 2H).

### Example 5: N-(tert-butyl)-3-((S)-2-((E)-3-(4-chloro-2-fluorophenyl)acrylamido)-3-cyclopropylpropanamido-2-oxo-4-((S)-2-oxopyrrolidin-3-yl)butanamide

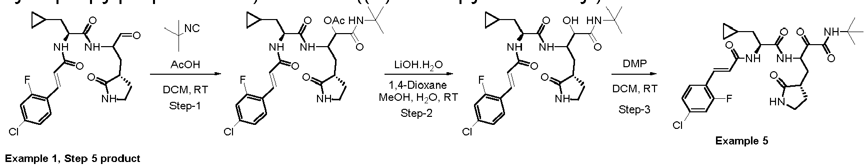

Step 1: To a stirred solution of (*E*)-3-(4-chloro-2-fluorophenyl)-*N*-((2*S*)-3-cyclopropyl-1-oxo-1-((1-oxo-3-((*S*)-2-oxopyrrolidin-3-yl)propan-2-yl)amino)propan-2-yl)acrylamide (Example 1, step 5 product, 430 mg, 0.955 mmol) in DCM (5 mL) at 0 °C was added AcOH (0.060 mL, 1.051 mmol) followed by *tert*-butylisocyanide (0.12 mL, 1.051 mmol). The resulting reaction mixture was stirred at rt for 16 h. After completion of the reaction, as monitored by TLC, the reaction mass was concentrated *in vacuo.* Purification by gradient flash column chromatography (Biotage-Isolera) using a 25 g silica SNAP cartridge (230-400 mesh), eluting with 0-5% MeOH in DCM yielded 1-(*tert*-butylamino)-3-((*S*)-2-((*E*)-3-(4-chloro-2-fluorophenyl)acrylamido)-3-cyclopropylpropanamido)-1-oxo-4-((S)-2-oxopyrrolidin-3-yl)butan-2-yl acetate as an off-white solid (300 mg, 0.50 mmol).

**LCMS (Method C):** m/z 593.2 (M+H), at 2.34 min.

**¹H NMR:** (400 MHz, DMSO-*d*₆) δ 8.43-8.37 (m, 1H), 8.01-7.84 (m, 1H), 7.69-7.66 (m, 1H), 7.57-7.46 (m, 3H), 7.42-7.36 (m, 2H), 6.91-6.87 (m, 1H), 4.92-4.76 (m, 1H), 4.48-4.47 (m, 1H), 4.35-4.21 (m, 1H), 3.12-3.05 (m, 2H), 2.21-2.19 (m, 2H), 2.18-2.11 (m, 1H), 2.10-2.06 (m, 3H), 2.00-1.93 (m, 1H), 1.56-1.45 (m, 3H), 1.25-1.18 (m, 9H), 0.85-0.65 (m, 1H), 0.41-0.37 (m, 2H), 0.12-0.08 (m, 2H).

Step 2: To a stirred solution of 1-(*tert*-butylamino)-3-((*S*)-2-((*E*)-3-(4-chloro-2-fluorophenyl)acrylamido)-3-cyclopropylpropanamido)-1-oxo-4-((*S*)-2-oxopyrrolidin-3-yl)butan-2-yl acetate (300 mg, 0.629 mmol) in 1,4-Dioxane (3 mL), MeOH (1 mL) and H₂O (2 mL) was added LiOH.H₂O (52.2 mg, 1.25 mmol). The resulting reaction mixture was stirred at rt for 16 h. After completion of the reaction, as monitored by TLC, the reaction mass was partitioned between EtOAc (25 mL) and H₂O (25 mL). The organic layer was separated, dried over anhydrous Na₂SO₄ and concentrated *in vacuo.* Purification by gradient flash column chromatography (Biotage-Isolera) using a 25 g silica SNAP cartridge (230-400 mesh), eluting with 0-5% MeOH in DCM yielded *N*-(*tert*-butyl)-3-((*S*)-2-((*E*)-3-(4-chloro-2-fluorophenyl)acrylamido)-3-cyclopropylpropanamido)-2-hydroxy-4-((S)-2-oxopyrrolidin-3-yl)butanamide as an off-white solid (200 mg, 0.36 mmol).

**LCMS (Method A):** m/z 551.2 (M+H), at 2.07 min.

**¹H NMR:** (400 MHz, DMSO-*d*₆) δ 8.45-8.43 (m, 1H), 7.73-7.67 (m, 1H), 7.56-7.37 (m, 5H), 7.02 (s, 1H), 6.98-6.86 (m, 1H), 5.77-5.74 (m, 1H), 4.46-4.45 (m, 1H), 4.19-4.01 (m, 1H), 3.77-3.75 (m, 1H), 3.29-3.05 (m, 2H), 2.34-2.13 (m, 2H), 2.05-1.95 (m, 1H), 1.63-1.53 (m, 2H), 1.49-1.39 (m, 1H), 1.48-1.45 (m, 10H), 0.79-0.69 (m, 1H), 0.46-0.29 (m, 2H), 0.15 - 0.10 (m, 2H).

Step 3: To a stirred solution of *N*-(*tert*-butyl)-3-((*S*)-2-((*E*)-3-(4-chloro-2-fluorophenyl)acrylamido)-3-cyclopropylpropanamido)-2-hydroxy-4-((*S*)-2-oxopyrrolidin-3-yl)butanamide (200 mg, 0.36 mmol) in DCM (10 mL) and DMSO (3 mL) was added Dess-Martin periodinane (230 mg, 0.54 mmol) portion wise at rt. The resulting reaction mixture was stirred at rt for 1 h. After the completion of the reaction, as monitored by TLC, 10% aqueous NaHCO₃ solution (50 mL) was added and the mixture extracted with DCM (2 x 50 mL). The combined organic layers were dried over anhydrous Na₂SO₄ and concentrated *in vacuo.* Purification by gradient flash column chromatography (Biotage-Isolera) using a 25 g silica SNAP cartridge (230-400 mesh), eluting with 0-6% MeOH in DCM yielded *N*-(*tert*-butyl)-3-((*S*)-2-((*E*)-3-(4-chloro-2-fluorophenyl)acrylamido)-3-cyclopropylpropanamido)-2-oxo-4-((S)-2-oxopyrrolidin-3-yl)butanamide as an off-white solid (Example 5, 80 mg, 0.15 mmol). **LCMS (Method C):** m/z 547.1 (M-H), at 2.19-2.62 min.

**¹H NMR:** (400 MHz, DMSO-*d*₆) δ 8.58-8.56 (m, 1H), 8.42-8.40 (m, 1H), 7.92 (s, 1H), 7.70-7.66 (m, 2H), 7.56-7.53 (m, 1H), 7.46-7.42 (m, 1H), 7.39-7.36 (m, 1H), 6.91-6.87 (m, 1H), 5.01-5.10 (m, 1H), 4.54-4.52 (m, 1H), 3.18-3.12 (m, 2H), 2.35-2.49 (m, 1H), 2.37-2.34 (m, 1H), 2.08-1.92 (m, 1H), 1.72-1.61 (m, 1H), 1.61-1.50 (m, 3H), 1.39-1.20 (m, 9H), 0.85-0.70 (m, 1H), 0.42-0.37 (m, 2H), 0.14-0.09 (m, 2H).

### Example 6: 3-((S)-2-((E)-3-(4-chloro-2-fluorophenyl)acrylamido)-3-cyclopropylpropanamido)-4-cyclopropyl-2-oxobutanamide

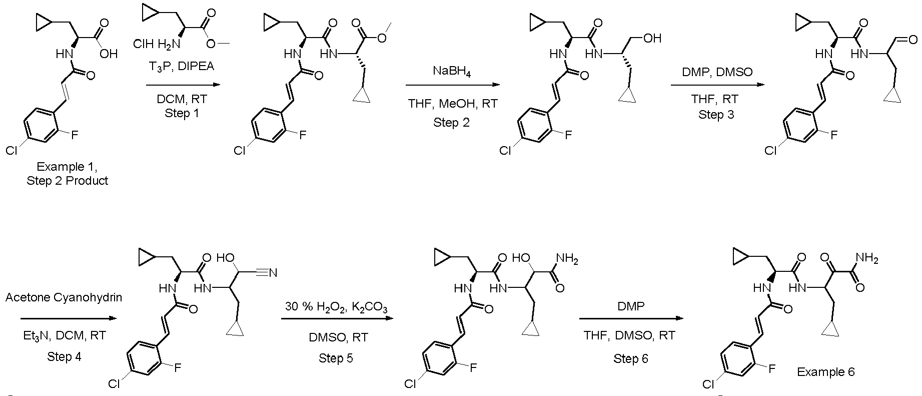

Steps 1-6: The title compound, Example 6, 3-((*S*)-2-((*E*)-3-(4-chloro-2-fluorophenyl)acrylamido)-3-cyclopropylpropanamido)-4-cyclopropyl-2-oxobutanamide (25 mg, 0.05 mmol, white solid) was prepared from (*S*,*E*)-2-(3-(4-chloro-2-fluorophenyl)acrylamido)-3-cyclopropylpropanoic acid (Example 1, Step 2 product, 2.0 g, 6.41 mmol) and methyl (S)-2-amino-3-cyclopropylpropanoate hydrochloride (CAS No. 206438-31-5, 1.38 g, 7.69 mmol) over steps 1-6 using the procedures detailed for Example 1. Step 3 used THF/DMSO (2:1, 15 mL) as solvent.

**LCMS (Method C):** m/z 450.1 (M+H), at 1.79-2.06 min.

**¹H NMR:** (400 MHz, DMSO-*d*₆) δ 8.50-8.39 (m, 2H), 8.01 (d, J=4.8 Hz, 1H), 7.74-7.66 (m, 2H), 7.56-7.53 (m, 1H), 7.48-7.43 (m, 1H), 7.39-7.36 (m, 1H), 6.94-6.88 (m, 1H), 5.15-5.10 (m, 1H), 4.66-4.58 (m, 1H), 1.67-1.47 (m, 4H), 0.84-0.74 (m, 2H), 0.42-0.35 (m, 4H), 0.13-0.02 (m, 4H).

### Example 7: 3-((S)-2-((E)-3-(4-chloro-2-fluorophenyl)acrylamido)-3-cyclopropylpropanamido)-4-cyclopentyl-2-oxobutanamide

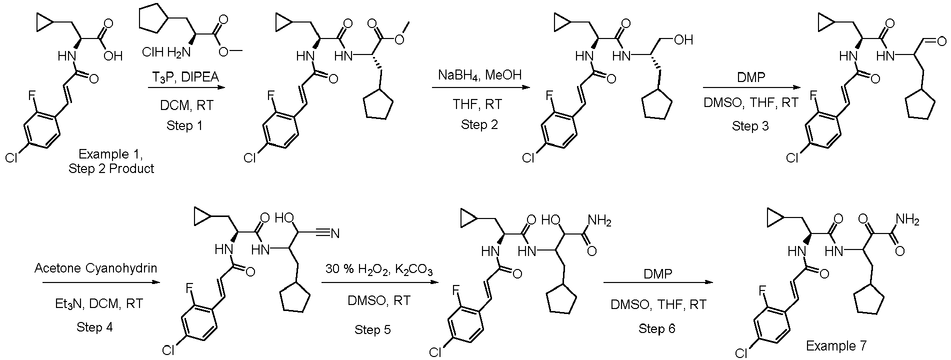

Steps 1-6: The title compound, Example 7, 3-((*S*)-2-((*E*)-3-(4-chloro-2-fluorophenyl)acrylamido)-3-cyclopropylpropanamido)-4-cyclopentyl-2-oxobutanamide (10 mg, 0.02 mmol, off-white solid) was prepared from (*S*,*E*)-2-(3-(4-chloro-2-fluorophenyl)acrylamido)-3-cyclopropylpropanoic acid (Example 1, Step 2 product, 1.0 g, 3.21 mmol) and methyl (*S*)-2-amino-3-cyclopentylpropanoate hydrochloride (CAS No. 1191996-99-2, 0.79 g, 3.85 mmol) over steps 1-6 using the procedures detailed for Example 1. Step 3 used THF/DMSO (10:1, 22 mL) as solvent.

**LCMS (Method C):** m/z 478.1 (M+H), at 2.00-2.28 min.

**¹H NMR:** (400 MHz, DMSO-*d*₆) δ 8.48-8.34 (m, 2H), 8.02 (s, 1H), 7.76 (s, 1H), 7.70-7.65 (m, 1H), 7.56-7.53 (m, 1H), 7.45 (d, J=16.0 Hz, 1H), 7.39-7.36 (m, 1H), 6.93-6.88 (m, 1H), 5.05-5.05 (m, 1H), 4.60-4.51 (m, 1H), 1.90-1.44 (m, 11H), 1.15-1.09 (m, 2H), 0.87-0.76 (m, 1H), 0.42-0.36 (m, 2H), 0.12-0.07 (m, 2H).

### Example 8: 3-((S)-3-cyclopropyl-2-(3-phenylpropanamido)propanamido)-2-oxo-4-((S)-2-oxopyrrolidin-3-yl)butanamide

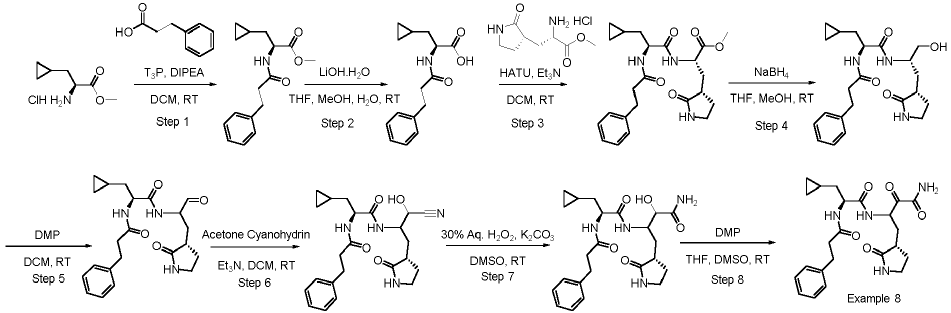

Step 1: To a stirred solution of methyl (*S*)-2-amino-3-cyclopropylpropanoate hydrochloride (CAS No. 206438-31-5, 2.38 g, 13.20 mmol) and 3-phenylpropanoic acid (2.01 g, 13.20 mmol) in DCM (30 mL) was added DIPEA (11.57 mL, 66.00 mmol) followed by T3P (50% in EtOAc, 12.68 mL, 19.90 mmol). The resulting reaction mixture was stirred at rt for 16 h. After completion of the reaction, as monitored by TLC, the reaction mixture was partitioned between DCM (100 mL) and 10% aqueous NaHCOs solution (100 mL). The organic layer was separated, dried over anhydrous Na₂SO₄ and concentrated *in vacuo.* Purification by gradient flash column chromatography (Biotage-Isolera) using a 100 g silica SNAP cartridge (230-400 mesh), eluting with 0-10% EtOAc in petroleum-ether yielded methyl (S)-3-cyclopropyl-2-(3-phenylpropanamido)propanoate as an off-white solid (2.12 g, 7.69 mmol). **LCMS (Method C):** m/z 276.1 (M+H), at 2.04 min.

**¹H NMR:** (400 MHz, DMSO-*d*₆) δ 8.27 (s, 1H), 7.26-7.21 (m, 5H), 4.30-4.28 (m, 1H), 3.61 (s, 3H), 2.81-2.80 (m, 2H), 2.43-2.25 (m, 2H), 1.59-1.44 (m, 2H), 0.78-0.56 (m, 1H), 0.37-0.35 (m, 2H), 0.07-0.01 (m, 2H).

Step 2: To a stirred solution of methyl (S)-3-cyclopropyl-2-(3-phenylpropanamido)propanoate (2.12 g, 7.69 mmol) in 1,4-Dioxane (10 mL), MeOH (5 mL) and H₂O (5 mL) at rtwas added LiOH.H₂O (0.368 g, 15.3 mmol). The resulting reaction mixture was stirred at rt for 2 h. After completion of the reaction, as monitored by TLC, the reaction mixture was concentrated *in vacuo.* The residue obtained was dissolved in H₂O (100 mL), acidified with 1.5 N HCl to approximately pH 5 and extracted with 20% MeOH in DCM (150 mL). The organic layer was separated, dried over anhydrous Na₂SO₄ and concentrated *in vacuo* to yield (*S*)-3-cyclopropyl-2-(3-phenylpropanamido)propanoic acid as an off-white solid (1.90 g, 7.23 mmol) that was used without further purification.

**LCMS (Method H):** m/z 262.3 (M+H), at 1.50 min.

**¹H NMR:** (400 MHz, DMSO-*d*₆) δ 12.50 (s, 1H), 8.13 (d, J=8.0 Hz, 1H), 7.28-7.17 (m, 5H), 4.25-4.24 (m, 1H), 2.84-2.70 (m, 2H), 2.56-2.31 (m, 2H), 1.56-1.46 (m, 2H), 0.81-0.65 (m, 1H), 0.52-0.35 (m, 2H), 0.15-0.09 (m, 2H).

Step 3: To a stirred solution of (*S*)-3-cyclopropyl-2-(3-phenylpropanamido)propanoic acid (1.90 g, 7.20 mmol) and methyl (*S*)-2-amino-3-((*S*)-2-oxopyrrolidin-3-yl)propanoate hydrochloride (Intermediate 1, 1.35 g, 7.20 mmol) in DCM (10 mL) was added Et₃N (4.05 mL, 29.1 mmol) followed by HATU (4.14 g, 10.9 mmol). The resulting reaction mixture was stirred at rt for 16 h. After the completion of the reaction as monitored by TLC, the reaction mixture was partitioned between DCM (100 mL) and 10% aqueous NaHCO₃ solution (100 mL). The organic layer was separated, washed with saturated brine (100 mL), dried over anhydrous Na₂SO₄ and concentrated *in vacuo.* Purification by gradient flash column chromatography (Biotage-Isolera) using a 25 g silica SNAP cartridge (230-400 mesh), eluting with 0-2% MeOH in DCM yielded methyl (*S*)-2-((*S*)-3-cyclopropyl-2-(3-phenylpropanamido)propanamido)-3-((S)-2-oxopyrrolidin-3-yl)propanoate as an off-white solid (1.24 g, 2.88 mmol).

**LCMS (Method A):** m/z 430.3 (M+H), at 1.83 min.

**¹H NMR:** (400 MHz, DMSO-*d*₆) δ 8.47-8.45 (m, 1H), 8.01-7.98 (m, 1H), 7.65 (s, 1H), 7.25-7.21 (m, 5H), 4.50-4.23 (m, 2H), 4.12-3.95 (m, 1H), 3.45-3.75 (m, 3H), 3.25-3.01 (m, 2H), 2.80-2.78 (m, 2H), 2.09-1.91 (m, 2H), 1.60-1.41 (m, 4H), 1.18 (t, J=9.2 Hz, 2H), 0.75-0.58 (m, 1H), 0.42-0.15 (m, 2H), 0.13-0.09 (m, 2H).

Steps 4-8: The title compound, Example 8, 3-((*S*)-3-cyclopropyl-2-(3-phenylpropanamido)propanamido)-2-oxo-4-((*S*)-2-oxopyrrolidin-3-yl)butanamide (off-white solid, 20 mg, 0.045 mmol) was prepared from Step 3 product (1.24 g, 2.80 mmol) over steps 4-8 using the procedures detailed for Example 1. Example 8 was isolated after purification by preparative HPLC using Method B.

**LCMS (Method C):** m/z 443.1 (M+H), at 1.69-1.84 min.

**¹H NMR:** (400 MHz, DMSO-*d*₆) δ 8.58-8.46 (m, 1H), 8.00-7.97 (m, 2H), 7.76 (s, 1H), 7.68 (s, 1H), 7.27-7.14 (m, 5H), 5.11-5.01 (m, 1H), 4.44-4.25 (m, 1H), 3.18-3.11 (m, 2H), 2.81 (t, J=8.0 Hz, 2H), 2.47-2.46 (m, 2H), 2.18-2.17 (m, 2H), 1.91-1.85 (m, 1H), 1.65-1.41 (m, 4H), 0.65-0.58 (m, 1H), 0.37-0.32 (m, 2H), 0.10-0.02 (m, 2H).

### Example 9: (2S)-N-(1-Cyano-2-((S)-2-oxopyrrolidin-3-yl)ethyl)-2-((E)-3-(2,4-dichlorophenyl)acrylamido)-4,4-dimethylpentanamide

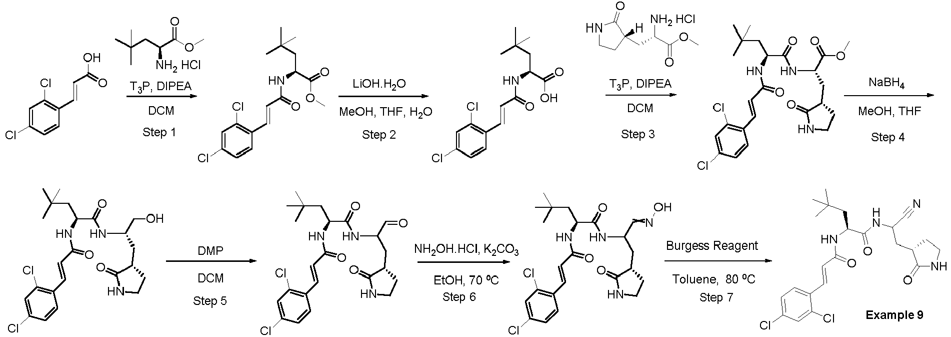

Step 1: To a stirred solution of (*E*)-3-(2,4-dichlorophenyl)acrylic acid (2.50 g, 11.5 mmol) and methyl (*S*)-2-amino-4,4-dimethylpentanoate hydrochloride (2.70 g, 13.8 mmol) in DCM (40 mL), was added DIPEA (6.00 mL, 34.6 mmol) followed by the addition of T3P (50% solution in EtOAc, 10.0 mL, 17.3 mmol). The reaction mixture was stirred at rt for 16 h, then partitioned between EtOAc (300 mL) and H₂O (200 mL). The phases were separated, the organic layer was washed with 10% aqueous NaHCOs solution (200 mL) and brine (200 mL), dried over anhydrous Na₂SO₄ and concentrated *in vacuo.* Purification by gradient flash column chromatography (Biotage-Isolera) using a 50 g silica SNAP cartridge (230-400 mesh), eluting with 0-8% EtOAc in Pet-ether gradient to afford methyl (*S*,*E*)-2-(3-(2,4-dichlorophenyl)acrylamido)-4,4-dimethylpentanoate as a white solid (3.37 g, 9.40 mmol).

**LCMS (Method C):** m/z 357.8 (M+H), at 2.69 min.

¹H **NMR:** (300 MHz, DMSO-*d*₆) δ 8.66 (d, J=8.0 Hz, 1H), 7.75-7.66 (m, 3H), 7.53-7.50 (m, 1H), 6.76 (d, J=16.0 Hz, 1H), 4.50-4.45 (m, 1H), 3.65 (s, 3H), 1.76-1.57 (m, 2H), 0.92-0.90 (m, 9H).

Step 2: LiOH.H₂O (0.59 g, 14.1 mmol) was added to a solution of methyl (*S*,*E*)-2-(3-(2,4-dichlorophenyl)acrylamido)-4,4-dimethylpentanoate (3.37 g, 9.40 mmol) in THF (40 mL), MeOH (5 mL) and H₂O (10 mL) and the mixture stirred at rt for 1 h. After concentration *in vacuo* the residue obtained was dissolved in H₂O (100 mL), acidified to approximately pH 5 using 1.5 N HCl and extracted with 20% MeOH in DCM (150 mL). The organic phase was separated, dried over anhydrous Na₂SO₄ and concentrated *in vacuo* to yield (*S*,*E*)-2-(3-(2,4-dichlorophenyl)acrylamido)-4,4-dimethylpentanoic acid as a white solid (3.10 g, 9.00 mmol).

**LCMS (Method C):** m/z 344.0 (M+H), at 2.33 min.

**¹H NMR:** (400 MHz, DMSO-*d*₆) δ 12.90 (s, 1H), 8.48 (d, J=8.4 Hz, 1H), 7.74-7.72 (m, 2H), 7.66 (d, J=15.6 Hz, 1H), 7.52-7.50 (m, 1H), 6.79 (d, J=15.6 Hz, 1H), 4.39-4.35 (m, 1H), 1.74-1.70 (m, 1H), 1.59-1.53 (m, 1H), 0.91 (s, 9H).

Step 3: To a stirred solution of (*S*,*E*)-2-(3-(2,4-dichlorophenyl)acrylamido)-4,4-dimethylpentanoic acid (3.10 g, 9.00 mmol) and methyl (*S*)-2-amino-3-((*S*)-2-oxopyrrolidin-3-yl)propanoate hydrochloride (Intermediate 1, 3.00 g, 13.5 mmol) in DCM (30 mL) was added DIPEA (4.70 mL, 27.2 mmol) followed by T3P (50% solution in EtOAc, 8.00 mL, 13.5 mmol) at 0°C. After stirring at rt for 16 h the reaction mixture was partitioned between EtOAc (150 mL) and H₂O (150 mL). and the organic layer was washed with 10% aqueous NaHCOs (100 mL) and saturated brine solution (50 mL), dried over anhydrous Na₂SO₄ and concentrated *in vacuo.* Purification by gradient flash column chromatography (Biotage-Isolera) using a 25 g silica SNAP cartridge (230-400 mesh), eluting with 0-5% MeOH in DCM yielded methyl (S)-2-((S)-2-((E)-3-(2,4-dichlorophenyl)acrylamido)-4,4-dimethylpentanamido)-3-((S)-2-oxopyrrolidin-3-yl)propanoate as a pale yellow solid (1.50 g, 2.92mmol).

**LCMS (Method C):** m/z 511.7 (M+H), at 2.15 min.

Step 4: To a stirred solution of methyl (*S*)-2-((*S*)-2-((*E*)-3-(2,4-dichlorophenyl)acrylamido)-4,4-dimethylpentanamido)-3-((S)-2-oxopyrrolidin-3-yl)propanoate (1.50 g, 2.92 mmol) in THF (20 mL) was added MeOH (2 mL) followed by NaBH₄ (166 mg, 4.39 mmol) portion wise at 0°C. After stirring at rt for 2 h the reaction mixture was quenched with brine solution (50 mL) and extracted with EtOAc (100 mL). The organic phase was dried over anhydrous Na₂SO₄ and concentrated under reduced pressure to obtain (*S*)-2-((*E*)-3-(2,4-dichlorophenyl)acrylamido)-*N*-((*S*)-1-hydroxy-3-((*S*)-2-oxopyrrolidin-3-yl)propan-2-yl)-4,4-dimethylpentanamide as an off-white solid (1.2 g, 2.47 mmol).

**LCMS (Method C):** m/z 484.1 (M+H), at 1.68 min.

Step 5: Dess-Martin periodinane (1.57 g, 3.71 mmol) was added to a solution of (*S*)-2-((*E*)-3-(2,4-dichlorophenyl)acrylamido)-*N*-((*S*)-1-hydroxy-3-((*S*)-2-oxopyrrolidin-3-yl)propan-2-yl)-4,4-dimethylpentanamide (1.20 g, 2.47 mmol) in DCM (15 mL) added at 0°C. After stirring at rt for 1 h 10% aqueous NaHCOs solution (50 mL) and EtOAc (200 mL) were added and the phases were separated. The organic phase was dried over anhydrous Na₂SO₄ and concentrated *in vacuo.* Purification by gradient flash column chromatography (Biotage-Isolera) using a 25 g silica SNAP cartridge (230-400 mesh), eluting with 0-3% MeOH in DCM yielded (2*S*)-2-((*E*)-3-(2,4-dichlorophenyl)acrylamido)-4,4-dimethyl-*N*-(1-oxo-3-((*S*)-2-oxopyrrolidin-3-yl)propan-2-yl)pentanamide as an off-white solid (0.75 g, 1.55 mmol).

**LCMS (Method E):** m/z 482.2 (M+H), at 1.90-2.10 min.

**¹H NMR:** (400 MHz, DMSO-*d*₆) δ 9.41-9.38 (m, 1H), 8.65 (d, J=7.2 Hz, 1H), 8.51 (d, J=8.4 Hz, 1H), 7.79-7.63 (m, 3H), 7.53-7.50 (m, 2H), 6.83-6.78 (m, 1H), 4.51-4.50 (m, 1H), 4.48-4.30 (m, 1H), 3.21-3.09 (m, 2H), 2.50-2.41 (m, 1H), 2.34-2.26 (m, 1H), 2.13-2.12 (m, 3H), 1.72-1.49 (m, 2H), 0.93-0.91 (m, 9H).

Step 6: NH₂OH.HCl (57 mg, 0.82 mmol) and K₂CO₃ (171 mg, 1.24 mmol) were added to a solution of (2*S*)-2-((*E*)-3-(2,4-dichlorophenyl)acrylamido)-4,4-dimethyl-*N*-(1-oxo-3-((*S*)-2-oxopyrrolidin-3-yl)propan-2-yl)pentanamide (0.20 g, 0.41 mmol) in EtOH (10 mL). After stirring at 70°C for 16 h then reaction mixture was cooled to rt and partitioned between EtOAc (10 mL) and H₂O (10 mL). The organic phase was dried over anhydrous Na₂SO₄, concentrated *in vacuo,* and purified by gradient flash column chromatography (Biotage-Isolera) using a 25 g silica SNAP cartridge (230-400 mesh), eluting with 0-5% MeOH in DCM to yield (2*S*)-2-((*E*)-3-(2,4-dichlorophenyl)acrylamido)-*N*-(1-(hydroxyimino)-3-((S)-2-oxopyrrolidin-3-yl)propan-2-yl)-4,4-dimethylpentanamide as an off-white solid 150 mg, 0.30 mmol).

**LCMS (Method E):** m/z 497.1 (M+H), at 2.09 min.

Step 7: Methyl *N*-(triethylammoniumsulfonyl)carbamate (0.071 g, 0.30 mmol) was added to a stirred solution of (2*S*)-2-((*E*)-3-(2,4-dichlorophenyl)acrylamido)-*N*-(1-(hydroxyimino)-3-((*S*)-2-oxopyrrolidin-3-yl)propan-2-yl)-4,4-dimethylpentanamide (0.15 g, 0.30 mmol) in PhMe (50 mL) and the resulting reaction mixture was stirred at 80°C for 16 h. After partitioning between EtOAc (50 mL) and H₂O (50 mL) the organic phase was dried over anhydrous Na₂SO₄, and concentrated *in vacuo.* The resulting crude material was purified by gradient flash column chromatography (Biotage-Isolera) using a 10 g silica SNAP cartridge (230-400 mesh), eluting with 0-5% MeOH in DCM, followed by mass directed preparative HPLC (Method B). Pure fractions were concentrated *in vacuo,* then partitioned between EtOAc (10 mL) and H₂O (10 mL). The organic phase was dried over anhydrous Na₂SO₄, concentrated *in vacuo* and lyophilised to yield the title compound, Example 9, (2*S*)-*N*-(1-cyano-2-((*S*)-2-oxopyrrolidin-3-yl)ethyl)-2-((*E*)-3-(2,4-dichlorophenyl)acrylamido)-4,4-dimethylpentanamide as an off-white solid (10 mg, 0.02 mmol).

**LCMS (Method C):** m/z 479.1 (M+H), at 2.59 min.

**Chiral SFC analysis (Method 2):** 1.82 & 3.69 min (47.6% & 49.1%).

**¹H NMR:** (400 MHz, DMSO-*d*₆) δ 8.99 (d, J=8.0 Hz, 1H), 8.55 (d, J=8.4 Hz, 1H), 7.73-7.65 (m, 4H), 7.54-7.51 (m, 1H), 6.78 (d, J=15.6 Hz, 1H), 4.96-4.94 (m, 1H), 4.46-4.45 (m, 1H), 3.14-3.10 (m, 2H), 2.15-2.09 (m, 2H), 1.73-1.50 (m, 5H), 0.93 (s, 9H).

### Example 10: (2S)-N-(4-(Cyclopropylamino)-3,4-dioxo-1-((S)-2-oxopyrrolidin-3-yl)butan-2-yl)-2-((E)-3-(2,4-dichlorophenyl)acrylamido)-4,4-dimethylpentanamide

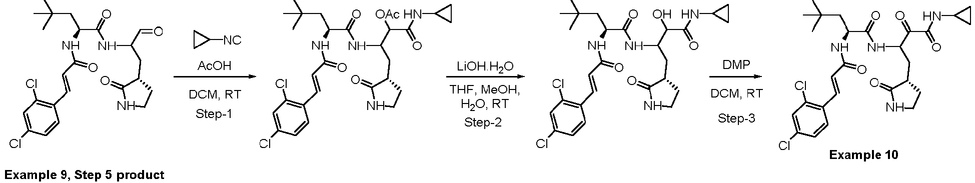

Step 1: AcOH (0.1 g, 1.65 mmol) followed by isocyanocyclopropane (55 mg, 0.82 mmol) were added to a stirred solution of (2*S*)-2-((*E*)-3-(2,4-dichlorophenyl)acrylamido)-4,4-dimethyl-*N*-(1-oxo-3-((*S*)-2-oxopyrrolidin-3-yl)propan-2-yl)pentanamide (Example 9, Step 5 product, 0.40 g, 0.82 mmol) in DCM (15 mL). After stirring for 16 h at rt the reaction mixture was concentrated *in vacuo.* Purification by gradient flash column chromatography (Biotage-Isolera) using a 25 g silica SNAP cartridge (230-400 mesh), eluting with 0-5% MeOH in DCM yielded 1-(cyclopropylamino)-3-((*S*)-2-((*E*)-3-(2,4-dichlorophenyl)acrylamido)-4,4-dimethylpentanamido)-1-oxo-4-((*S*)-2-oxopyrrolidin-3-yl)butan-2-yl acetate as an off-white solid (380 mg, 0.62 mmol).

**LCMS (Method C):** m/z 609.1 (M+H), at 1.91 min.

**¹H NMR:** (400 MHz, DMSO-*d*₆) δ 8.40 (d, J=8.4 Hz, 1H), 8.08-8.03 (m, 2H), 7.89 (d, J=9.2 Hz, 1H), 7.72-7.63 (m, 3H), 7.57-7.50 (m, 1H), 6.81 (d, J=16.0 Hz, 1H), 5.76-5.66 (m, 1H), 4.76-4.74 (m, 1H), 4.74-4.68 (m, 1H), 3.27-3.12 (m, 2H), 2.12-2.05 (m, 4H), 2.00-1.92 (m, 3H), 1.54-1.49 (m, 4H), 0.92-0.89 (m, 9H), 0.63-0.57 (m, 2H), 0.44-0.42 (m, 2H).

Step 2: MeOH (3 mL) and H₂O (5 mL), followed by LiOH.H₂O (392 mg, 0.93 mmol) were added to a stirred solution of 1-(cyclopropylamino)-3-((*S*)-2-((*E*)-3-(2,4-dichlorophenyl)acrylamido)-4,4-dimethylpentanamido)-1-oxo-4-((*S*)-2-oxopyrrolidin-3-yl)butan-2-yl acetate (380 mg, 0.62 mmol) in THF (5 mL) and the reaction mixture was stirred at rt for 1 h. EtOAc (50 mL) and H₂O (50 mL) were added, the phases were separated, dried over anhydrous Na₂SO₄ and concentrated *in vacuo* to yield (2*S*)-*N*-(4-(cyclopropylamino)-3-hydroxy-4-oxo-1-((*S*)-2-oxopyrrolidin-3-yl)butan-2-yl)-2-((*E*)-3-(2,4-dichlorophenyl)acrylamido)-4,4-dimethylpentanamide as an off-white solid (350 mg, 0.61 mmol).

**LCMS (Method C):** m/z 567.1 (M+H), at 1.75 min.

**¹H NMR:** (400 MHz, DMSO-*d*₆) δ 8.47-8.44 (m, 1H), 7.78-7.64 (m, 3H), 7.58-7.50 (m, 2H), 7.46-7.43 (m, 1H), 7.34-7.33 (m, 1H), 6.88-6.77 (m, 1H), 5.82-5.63 (m, 2H), 4.06-4.03 (m, 1H), 3.83-3.81 (m, 1H), 3.13-3.04 (m, 2H), 2.68-2.67 (m, 1H), 2.12-2.05 (m, 2H), 2.00-1.90 (m, 1H), 1.54-1.50 (m, 4H), 0.92-0.83 (m, 9H), 0.59-0.55 (m, 2H), 0.48-0.46 (m, 2H).

Step 3: Dess-Martin Periodinane (392 mg, 0.92 mmol) was added to a stirred solution of (2S)-*N*-(4-(cyclopropylamino)-3-hydroxy-4-oxo-1-((*S*)-2-oxopyrrolidin-3-yl)butan-2-yl)-2-((*E*)-3-(2,4-dichlorophenyl)acrylamido)-4,4-dimethylpentanamide (350 mg, 0.61 mmol) in DCM (10 mL) at 0°C. After stirring at rt for 1 h, 10% aqueous NaHCOs solution (20 mL) was added and the reaction mixture extracted with EtOAc (2 X 50 mL). The combined organic layers were dried over anhydrous Na₂SO₄ and concentrated *in vacuo.* After purification by mass directed preparative HPLC (Method B), pure fractions were concentrated *in vacuo,* and partitioned between 10% aqueous NaHCOs solution (25 mL) and EtOAc (50 mL). The organic phase was dried over anhydrous Na₂SO₄ and concentrated *in vacuo* to yield the title compound, Example 10, (2*S*)*-N*-(4-(cyclopropylamino)-3,4-dioxo-1-((*S*)-2-oxopyrrolidin-3-yl)butan-2-yl)-2-((*E*)-3-(2,4-dichlorophenyl)acrylamido)-4,4-dimethylpentanamide as a white solid (145 mg, 0.25 mmol).

**LCMS (Method E):** m/z 565.2 (M+H), at 2.05-2.23 min.

**¹H NMR:** (400 MHz, DMSO-*d*₆) δ 8.73-8.66 (m, 2H), 8.41 (d, J=8.4 Hz, 1H), 7.72-7.64 (m, 3H), 7.56-7.50 (m, 1H), 7.34-7.33 (m, 1H), 6.81 (d, J=16.0 Hz, 1H), 4.98-4.90 (m, 1H), 4.56-4.55 (m, 1H), 3.18-3.12 (m, 2H), 2.91-2.86 (m, 1H), 2.74-2.73 (m, 2H), 2.53-2.50 (m, 2H), 2.22-2.17 (m, 1H), 1.70-1.63 (m, 2H), 0.92-0.83 (m, 9H), 0.67-0.63 (m, 2H), 0.59-0.57 (m, 2H).

### Example 11: (2S)-2-((E)-3-(4-chloro-2-fluorophenyl)acrylamido)-N-(4-(cyclopropylamino)-3,4-dioxo-1-((S)-2-oxopyrrolidin-3-yl)butan-2-yl)-4,4-dimethylpentanamide

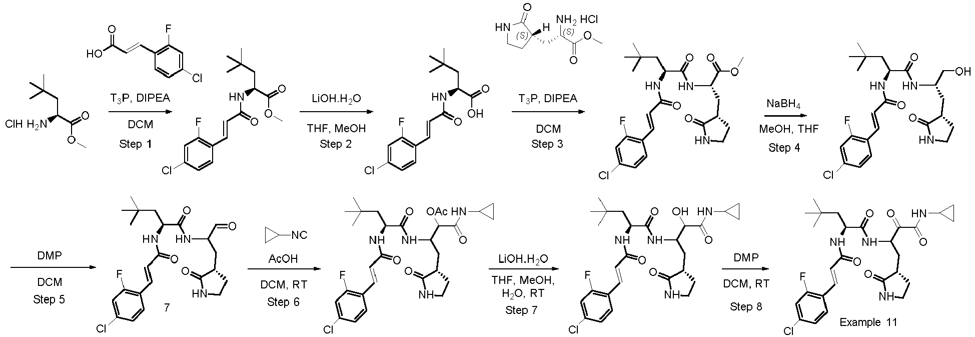

Steps 1-8: The title compound, Example 11, (2*S*)-2-((*E*)-3-(4-chloro-2-fluorophenyl)acrylamido)-*N*-(4-(cyclopropylamino)-3,4-dioxo-1-((*S*)-2-oxopyrrolidin-3-yl)butan-2-yl)-4,4-dimethylpentanamide was prepared as a white solid (17 mg, 0.03 mmol) using procedures similar to those detailed above.

**LCMS (Method A):** m/z 549.0 (M+H), at 1.87-2.04 min.

**¹H NMR:** (400 MHz, DMSO-*d*₆) δ 8.73-8.62 (m, 1H), 8.46-8.40 (m, 1H), 7.75-7.65 (m, 2H), 7.55-7.52 (m, 1H), 7.49-7.42 (m, 1H), 7.38-7.33 (m, 1H), 6.86-6.80 (m, 1H), 6.25-6.14 (m, 1H), 5.10-5.00 (m, 1H), 4.61-4.59 (m, 1H), 3.18-3.11 (m, 2H), 2.74-2.67 (m, 1H), 2.18-2.15 (m, 2H), 1.67-1.50 (m, 3H), 0.97-0.91 (m, 2H), 0.86-0.82 (m, 9H), 0.66-0.51 (m, 4H).

### Example 12: (2S)-N-(4-(cyclopropylamino)-3,4-dioxo-1-((S)-2-oxopyrrolidin-3-yl)butan-2-yl)-4,4-dimethyl-2-((S)-3-phenylbutanamido)pentanamide

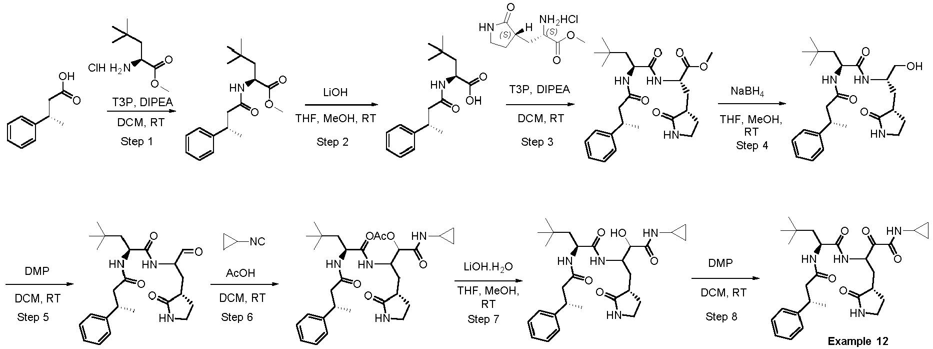

Steps 1-8: The title compound, Example 12, (2*S*)-*N*-(4-(cyclopropylamino)-3,4-dioxo-1-((*S*)-2-oxopyrrolidin-3-yl)butan-2-yl)-4,4-dimethyl-2-((*S*)-3-phenylbutanamido)pentanamide was prepared as an off-white solid (9.9 mg, 0.02 mmol) using procedures similar to those detailed above.

**LCMS (Method A):** m/z 513.3 (M+H), at 1.74-1.92 min.

**¹H NMR:** (400 MHz, DMSO-*d*₆) δ 8.73-8.71 (m, 1H), 8.48-8.46 (m, 1H), 8.03-8.01 (m, 1H), 7.72-7.69 (m, 1H), 7.29-7.15 (m, 5H), 4.95-4.90 (m, 1H), 4.37-4.36 (m, 1H), 3.18-3.15 (m, 4H), 2.68-2.67 (m, 1H), 2.40-2.36 (m, 2H), 2.30-2.10 (m, 1H), 1.90-1.88 (m, 1H), 1.68-1.56 (m, 3H), 1.46-1.40 (m, 1H), 1.17-1.14 (m, 3H), 0.90-0.85 (m, 9H), 0.66-0.57 (m, 4H).

### Example 13: (2S)-N-(4-(cyclopropylamino)-3,4-dioxo-1-((S)-2-oxopyrrolidin-3-yl)butan-2-yl)-4,4-dimethyl-2-((S)-3-phenylbutanamido)pentanamide

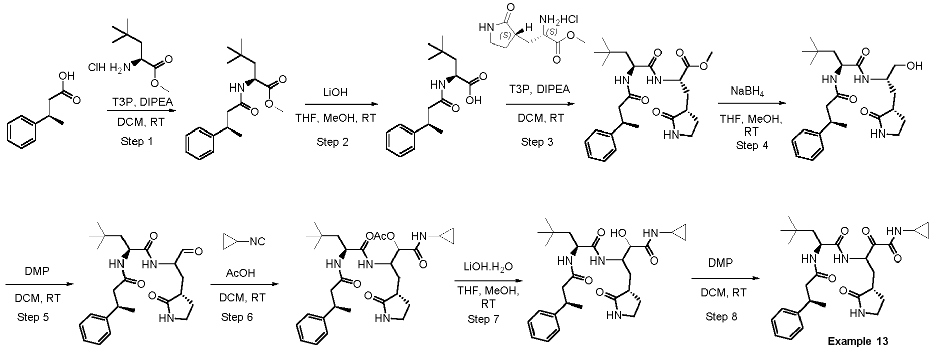

Steps 1-8: The title compound, Example 13, (2*S*)-*N*-(4-(cyclopropylamino)-3,4-dioxo-1-((*S*)-2-oxopyrrolidin-3-yl)butan-2-yl)-4,4-dimethyl-2-((*R*)-3-phenylbutanamido)pentanamide was prepared as an off-white solid (10 mg, 0.02 mmol) using procedures similar to those detailed above.

**LCMS (Method A):** m/z 513.3 (M+H), at 1.78-1.96 min.

**¹H NMR:** (400 MHz, DMSO-*d*₆) δ 8.74-8.71 (m, 1H), 8.58-8.48 (m, 1H), 8.06-7.97 (m, 1H), 7.72-7.70 (m, 1H), 7.30-7.15 (m, 5H), 5.05-5.01 (m, 1H), 4.31-4.29 (m, 1H), 3.20-3.20 (m, 3H), 2.74-2.73 (m, 1H), 2.39-2.34 (m, 2H), 2.19-2.14 (m, 1H), 1.91-1.86 (m, 1H), 1.76-1.50 (m, 3H), 1.36-1.32 (m, 2H), 1.28-1.25 (m, 3H), 0.89-0.80 (m, 9H), 0.67-0.64 (m, 2H), 0.59-0.58 (m, 2H).

### Examples 14 and 15: (2S)-N-(4-(cyclopropylamino)-3,4-dioxo-1-((S)-2-oxopyrrolidin-3-yl)butan-2-yl)-4,4-dimethyl-2-((S)-3-phenylpentanamido)pentanamide and (2S)-N-(4-(cyclopropylamino)-3,4-dioxo-1-((S)-2-oxopyrrolidin-3-yl)butan-2-yl)-4,4-dimethyl-2-((R)-3-phenylpentanamido)pentanamide

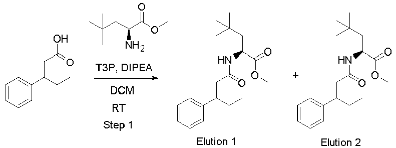

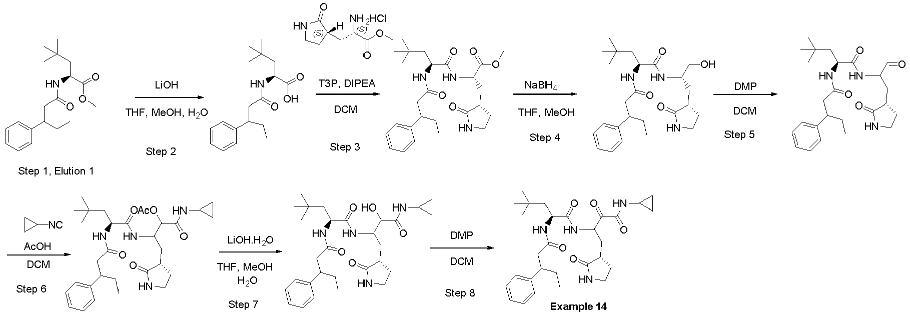

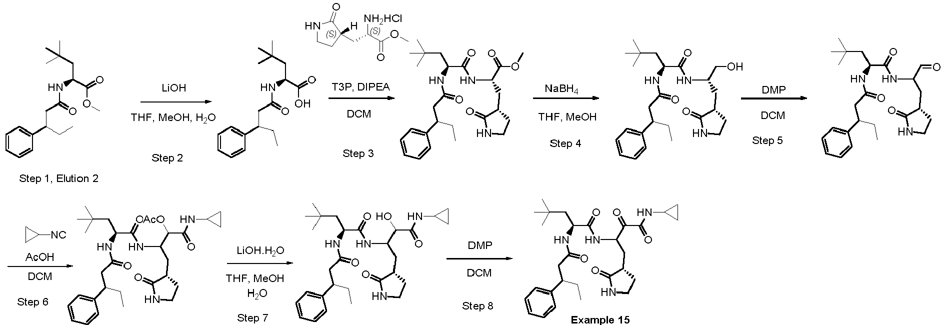

Step 1: To a stirred solution of 3-phenylpentanoic acid (2.00 g, 11.2 mmol) and methyl (S)-2-amino-4,4-dimethylpentanoate hydrochloride (3.29 g, 16.8 mmol) in DCM (30 mL) was added DIPEA (5.80 mL, 33.7 mmol) followed by the dropwise addition of T3P (50% solution in EtOAc, 10.6 mL, 16.8 mmol) at 0 °C. After stirring at rt for 16 h the reaction mixture was partitioned between EtOAc (200 mL) and H₂O (200 mL) and the phases were separated. The organic phase was washed with 10% aqueous NaHCOs solution (200 mL) and Brine solution (100 mL). The organic phase was dried over anhydrous Na₂SO₄ and concentrated *in vacuo.* Purification by gradient flash column chromatography (Biotage-Isolera) using a 25 g silica SNAP cartridge (230-400 mesh), eluting with 0-14% EtOAc in petroleum ether, yielded methyl (2S)-4,4-dimethyl-2-(3-phenylpentanamido)pentanoate as an off-white solid, as a mixture of diastereomers.

The diastereomers were separated by preparative chiral SFC purification, using the below method. Instrument: PIC 100 (PIC Solution, Inc.); Column: Chiralcel OX-H (250*30) mm, 5µm; Mobile phase: CO₂:0.5% Isopropyl amine in MeOH (80:20); Flow rate: 70 g/min; Back pressure: 100 bar; Wavelength: 210 nm; Cycle time: 4.5 min.

The peak eluting first in the purification was named as Elution 1 and the second eluting peak was named as Elution 2. After separation the elutions were concentrated *in vacuo* to yield Step 1, Elution 1 product (1.0 g) and Step 1, Elution 2 product (0.8 g), which were used in the subsequent steps without determination of absolute stereochemistry.

### Step 1, Elution 1:

**LCMS (Method A):** m/z 320.4 (M+H), at 2.41 min.

**¹H NMR:** (400 MHz, DMSO-*d*₆) δ 8.19 (d, J=7.6 Hz, 1H), 7.30-7.26 (m, 2H), 7.19-7.16 (m, 3H), 4.28-4.23 (m, 1H), 3.53 (s, 3H), 2.93 (d, J=4.8 Hz, 1H), 2.47-2.41 (m, 1H), 2.36-2.31 (m, 1H), 1.64-1.48 (m, 4H), 0.87-0.84 (m, 9H), 0.75-0.66 (m, 3H).

### Step 1, Elution 2:

**LCMS (Method A):** m/z 320.3 (M+H), at 2.36 min.

**¹H NMR:** (400 MHz, DMSO-*d*₆) δ 8.16 (d, J=7.6 Hz, 1H), 7.28-7.24 (m, 2H), 7.18-7.14 (m, 3H), 4.23-4.18 (m, 1H), 3.60 (s, 3H), 2.93-2.90 (m, 1H), 2.42-2.34 (m, 2H), 1.68-1.62 (m, 1H), 1.55-1.49 (m, 2H), 1.45-1.39 (m, 1H), 0.75-0.73 (m, 9H), 0.71-0.67 (m, 3H).

Steps 2 to 8, Example 14: The title compound, (2*S*)-*N*-(4-(cyclopropylamino)-3,4-dioxo-1-((*S*)-2-oxopyrrolidin-3-yl)butan-2-yl)-4,4-dimethyl-2-((*S*)-3-phenylpentanamido)pentanamide or (2*S*)-*N*-(4-(cyclopropylamino)-3,4-dioxo-1-((*S*)-2-oxopyrrolidin-3-yl)butan-2-yl)-4,4-dimethyl-2-((*R*)-3-phenylpentanamido)pentanamide was prepared as an off-white solid (35 mg, 0.07 mmol) using procedures similar to those detailed above, from Step 1, Elution 1 product (1.4 g).

**LCMS (Method A):** m/z 527.4 (M+H), at 1.83-2.00 min.

**¹H NMR:** (400 MHz, DMSO-*d*₆) δ 8.71 (d, J=4.8 Hz, 1H), 8.42 (d, J=6.8 Hz, 1H), 7.98 (d, J=8.4 Hz, 1H), 7.71-7.69 (m, 1H), 7.28-7.24 (m, 2H), 7.17-7.14 (m, 3H), 4.94-4.89 (m, 1H), 4.34-4.30 (m, 1H), 3.25-3.09 (m, 2H), 2.97-2.90 (m, 1H), 2.67-2.67 (m, 1H), 1.90-1.80 (m, 1H), 1.66-1.53 (m, 5H), 1.49-1.37 (m, 2H), 0.87 (s, 9H), 0.71-0.60 (m, 7H), 0.59-0.56 (m, 3H).

Steps 2 to 8, Example 15: The title compound, (2*S*)-*N*-(4-(cyclopropylamino)-3,4-dioxo-1-((*S*)-2-oxopyrrolidin-3-yl)butan-2-yl)-4,4-dimethyl-2-((*S*)-3-phenylpentanamido)pentanamide or (2*S*)-*N*-(4-(cyclopropylamino)-3,4-dioxo-1-((*S*)-2-oxopyrrolidin-3-yl)butan-2-yl)-4,4-dimethyl-2-((*R*)-3-phenylpentanamido)pentanamide was prepared as an off-white solid (30 mg, 0.06 mmol) using procedures similar to those detailed above, from Step 1, Elution 2 product (1.0 g).

**LCMS (Method A):** m/z 527.4 (M+H), at 1.83-2.00 min.

**¹H NMR:** (400 MHz, DMSO-*d*₆) δ 8.73-8.70 (m, 1H), 8.50-8.45 (m, 1H), 8.00-7.90 (m, 1H), 7.70 (d, J=5.6 Hz, 1H), 7.29-7.23 (m, 2H), 7.17-7.13 (m, 3H), 4.95-4.82 (m, 1H), 4.25-4.13 (m, 1H), 4.08-3.94 (m, 1H), 3.22-3.04 (m, 2H), 2.89-2.87 (m, 1H), 2.45-2.29 (m, 2H), 2.18-2.05 (m, 2H), 1.89-1.77 (m, 1H), 1.68-1.53 (m, 3H), 1.50-1.39 (m, 2H), 1.33-1.20 (m, 1H), 0.89-0.84 (m, 3H), 0.70-0.66 (m, 11H), 0.53-0.53 (m, 2H).

### Examples 16 and 17: (2S)-N-(4-(cyclopropylamino)-3,4-dioxo-1-((S)-2-oxopyrrolidin-3-yl)butan-2-yl)-4,4-dimethyl-2-((S)-4,4,4-trifluoro-3-phenylbutanamido)pentanamide and (2S)-N-(4-(cyclopropylamino)-3,4-dioxo-1-((S)-2-oxopyrrolidin-3-yl)butan-2-yl)-4,4-dimethyl-2-((R)-4,4,4-trifluoro-3-phenylbutanamido)pentanamide

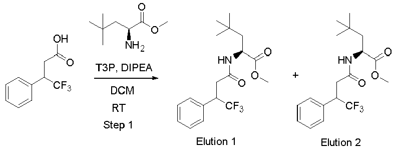

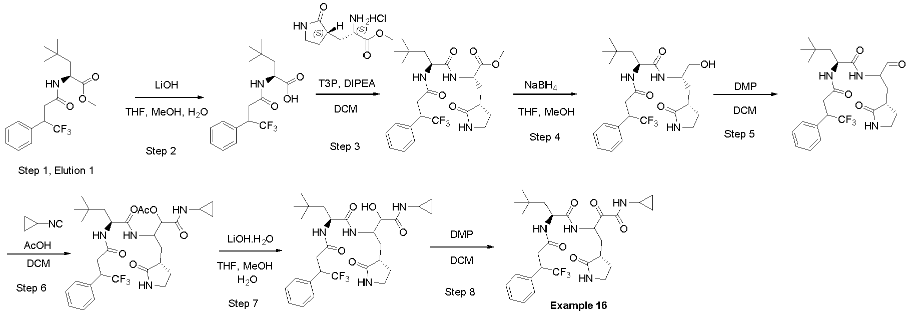

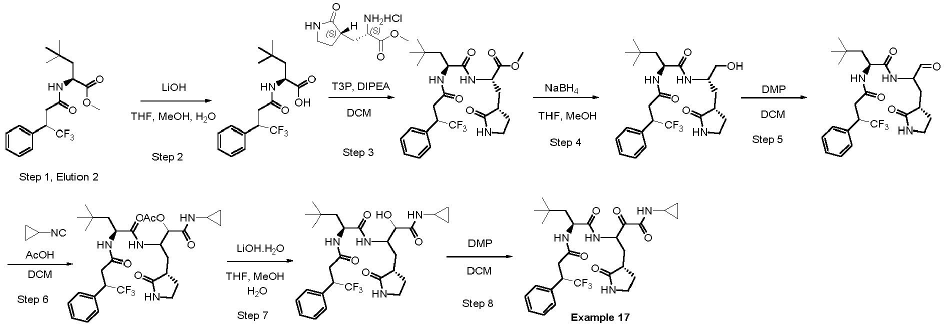

Step 1: To a stirred solution of 4,4,4-trifluoro-3-phenylbutanoic acid (2.0 g, 9.16 mmol) and methyl (*S*)-2-amino-4,4-dimethylpentanoate hydrochloride (2.18 g, 11.0 mmol) in DCM (30 mL) was added DIPEA (6.30 mL, 33.7 mmol) followed by the addition of T3P (50% solution in EtOAc, 8.60 mL, 13.8 mmol) at 0 °C. After stirring at rt for 16 h the reaction mixture was partitioned between EtOAc (200 mL) and H₂O (200 mL) and the phases were separated. The organic phase was washed with 10% aqueous NaHCO₃ solution (200 mL) and Brine solution (100 mL). The organic phase was dried over anhydrous Na₂SO₄ and concentrated *in vacuo.* Purification by gradient flash column chromatography (Biotage-Isolera) using a 25 g silica SNAP cartridge (230-400 mesh), eluting with 0-14% EtOAc in petroleum ether, yielded methyl (*S*)-4,4-dimethyl-2-(4,4,4-trifluoro-3-phenylbutanamido)pentanoate as an off-white solid, as a mixture of diastereomers.

The diastereomers were separated by preparative chiral SFC purification, using the below method. Instrument: PIC 100 (PIC Solution, Inc.); Column: (*R*,*R*) Whelk 250*30mm, 5µm; Mobile phase: CO₂:MeOH (90:10); Flow rate: 70 g/min; Back pressure: 100 bar; Wavelength: 210 nm; Cycle time: 5.0 min.

The peak eluting first in the purification was named as Elution 1 and the second eluting peak was named as Elution 2. After separation the elutions were concentrated *in vacuo* to yield Step 1, Elution 1 product (1.0 g) and Step 1, Elution 2 product (0.8 g), which were used in the subsequent steps without determination of absolute stereochemistry.

Steps 2 to 8, Example 16: The title compound, (2*S*)-*N*-(4-(cyclopropylamino)-3,4-dioxo-1-((*S*)-2-oxopyrrolidin-3-yl)butan-2-yl)-4,4-dimethyl-2-((*S*)-4,4,4-trifluoro-3-phenylbutanamido)pentanamide or (2*S*)-*N*-(4-(cyclopropylamino)-3,4-dioxo-1-((*S*)-2-oxopyrrolidin-3-yl)butan-2-yl)-4,4-dimethyl-2-((*R*)-4,4,4-trifluoro-3-phenylbutanamido)pentanamide was prepared as an off-white solid (5 mg, 0.01 mmol) using procedures similar to those detailed above, from Step 1, Elution 1 product (1.0 g).

**LCMS (Method A):** m/z 567.3 (M+H), at 1.88-2.07 min.

**¹H NMR:** (400 MHz, DMSO-*d*₆) δ 8.71-8.70 (m, 1H), 8.55 (d, J=6.8 Hz, 1H), 8.17 (d, J=8.4 Hz, 1H), 7.72 (d, J=5.6 Hz, 1H), 7.34-7.31 (m, 5H), 4.98-4.97 (m, 1H), 4.18-4.16 (m, 1H), 4.02-4.01 (m, 2H), 3.19-3.12 (m, 3H), 2.95-2.89 (m, 2H), 2.78-2.74 (m, 2H), 2.18-2.17 (m, 2H), 1.89-1.87 (m, 1H), 1.63-1.57 (m, 3H), 1.48-1.44 (m, 1H), 1.19-1.17 (m, 1H), 0.63-0.59 (m, 9H).

Steps 2 to 8, Example 17: The title compound, (2*S*)-*N*-(4-(cyclopropylamino)-3,4-dioxo-1-((*S*)-2-oxopyrrolidin-3-yl)butan-2-yl)-4,4-dimethyl-2-((*S*)-4,4,4-trifluoro-3-phenylbutanamido)pentanamide or (2*S*)-*N*-(4-(cyclopropylamino)-3,4-dioxo-1-((*S*)-2-oxopyrrolidin-3-yl)butan-2-yl)-4,4-dimethyl-2-((*R*)-4,4,4-trifluoro-3-phenylbutanamido)pentanamide was prepared as an off-white solid (40 mg, 0.07 mmol) using procedures similar to those detailed above, from Step 1, Elution 2 product (0.8 g).

**LCMS (Method A):** m/z 567.3 (M+H), at 1.86-2.05 min.

**¹H NMR:** (400 MHz, DMSO-*d*₆) δ 8.71 (d, J=4.4 Hz, 1H), 8.47 (d, J=7.2 Hz, 1H), 8.24-8.18 (m, 1H), 7.74-7.69 (m, 1H), 7.34-7.31 (m, 5H), 4.89-4.85 (m, 1H), 4.32-4.31 (m, 1H), 4.04-4.02 (m, 1H), 3.16-3.11 (m, 2H), 2.85-2.67 (m, 3H), 2.17-2.16 (m, 1H), 2.05-2.02 (m, 1H), 1.84-1.81 (m, 1H), 1.65-1.54 (m, 2H), 1.40-1.24 (m, 1H), 1.22-1.19 (m, 1H), 0.71 (s, 7H), 0.59-0.56 (m, 6H).

Examples 18 to 33 were synthesized using procedures similar to those detailed above.

### BIOLOGICAL ACTIVITY

### Construct design of SARS-CoV-2 Mpro

The SARS CoV-2-Mpro (Main Protease/3C-like protease, UniProt ID: P0DTD1) protein sequence, up to and including its autocleavage boundaries, as well as the preceding N-terminal 5 amino acid residues, including the P1 glutamine residue, were codon optimised for *E. coli* expression and cloned into pET26b (Merck, #US169862-3) or pGEX6P1 (Fisher Scientific, #10350355) vectors using *Bam*HI and *Xhol* sites. The expression constructs thus featured a native viral N-terminal sequence, as well as a C-terminal modified 3C-protease cleavage site (LEVLFQGK), with an alternative lysine residue at the P2' position, followed by a polyhistidine (His-8) tag.

### Protein expression and protein purification

Chemically competent BL21(DE3)-RIL *E. coli* (Agilent, #230240) cells were transformed with the relevant coronavirus Mpro construct and grown overnight at 37 °C on LB agar plates supplemented with the appropriate antibiotics. All culture steps were performed at 37 °C unless otherwise stated. A scraping of colonies was grown in 15 mL of antibiotic supplemented LB media, for a period of approximately 2 hours, taking care not to exceed an optical density (OD) density of 2.0 as measured in a spectrophotometer at 600 nm. This preculture was used to inoculate a 500 mL expression culture: either LB media for IPTG induced expression or autoinduction superbroth media (Formedium, #AIMSB0210). In LB media, expression was induced at an OD of 0.7-1.0 by the addition of IPTG to a final concentration of 0.5 mM. The culture was then grown at 18 °C overnight. In autoinduction expression, the temperature was dropped to 18 °C once an OD of 0.7-1.0 was observed then grown overnight. The cells were harvested by centrifugation and frozen until use.

Thawed cells were resuspended into resuspension buffer: 20 mM Tris-HCl pH 8.0, 150 mM NaCl, and DNase I (Merck #4716728001) and lysed by sonication. The lysate was clarified by centrifugation at 23,000 rcf for 15 mins at 4 °C. The supernatant was loaded onto 5 mL of NiNTA resin (Cytiva, #17-5248-02) at a flow-rate of 0.5 mL/min. The resin was washed with the same buffer as above containing 20 mM imidazole. Mpro protein was eluted using the same buffer containing 250 mM imidazole. The target protein was further purified using a Superdex S75 16/60 pg (GE, #GE28-9893-33) column in resuspension buffer. Protein purity was assessed by SDS-PAGE and identity confirmed by mass spectrometry. Purified protein was concentrated and frozen until later use.

### SARS-CoV-2 Mpro enzyme assay

The activity of SARS-Cov-2 Mpro was determined in a Fluorescence Resonance Energy Transfer (FRET)-based enzymatic assay using FRET Substrate Dabcyl-KTSAVLQSGFRKM-E(Edans)-Amide. In brief, 5 µL of test compounds (concentrations ranging from 100 µM to 0.0017 µM) was preincubated with 5 µL of 20 nM (final concentration) Mpro enzyme for 30 min at 30 °C in an assay buffer containing 20 mM HEPES, 120 mM NaCl, 0.4 mM EDTA, and 4 mM DTT and 20% glycerol. Reaction was initiated by addition of 10 µL of 20 µM (final concentration) of FRET substrate (Dabcyl-KTSAVLQSGFRKM-E (Edans)-Amide). The reaction was incubated for 1 h and the resulting fluorescent intensity was measured at Ex=360 nm/Em=490 nm at 30 °C using a SPARK 20M plate reader (Tecan). Boceprevir was used a reference standard compound. plC₅₀ and pKi were determined using 4PL GraphPad Prism and data were represented as a mean n=2± SD.

**Table 2**

| **Example No.** | **SARS-CoV-2 Mpro enzyme assay pKi** |
|---|---|
| 1 | 7.9 |
| 2 | 7.7 |
| 3 | 7.6 |
| 4 | 6.0 |
| 5 | 6.3 |
| 6 | 5.3 |
| 7 | 5.1 |
| 8 | 7.3 |
| 9 | 6.4 |
| 10 | 8.2 |
| 11 | 1 |
| 12 | 7.8 |
| 13 | 7.8 |
| 14 | 8.1 |
| 15 | 8.0 |
| 16 | 7.7 |
| 17 | 7.9 |
| 18 | 7.9 |
| 19 | 7.9 |
| 20 | 7.0 |
| 21 | 8.1 |
| 22 | 8.2 |
| 23 | 7.9 |
| 24 | 7.9 |
| 25 | 8.0 |
| 26 | 8.0 |
| 27 | 8.1 |
| 28 | 7.8 |
| 29 | 7.8 |
| 30 | 7.7 |
| 31 | 7.6 |
| 32 | 7.9 |
| 33 | 7.6 |

## Claims

1. A compound of Formula (1'): 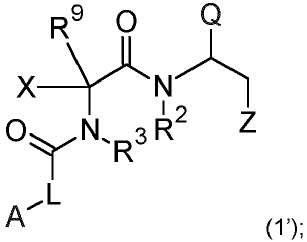 or a salt thereof, wherein;
A is selected from: 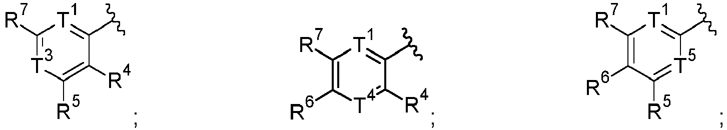 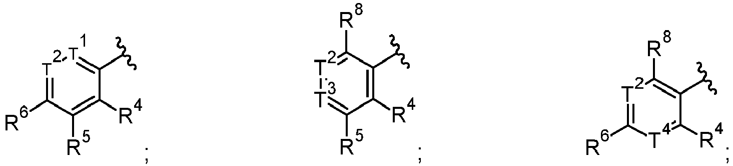
Q is CN or a group of formula: 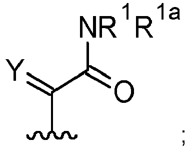
X is a C₁₋₆ saturated or unsaturated hydrocarbon group optionally substituted with 1 to 6 fluorine atoms or X is joined with R⁹ to form a C₃₋₆ cycloalkyl ring which is optionally substituted with 1 to 3 fluorine atoms or 1 to 3 C₁₋₃ alkyl groups;
Y is O or NOR¹⁶;
T¹ is CR⁸ or N;
T² is CR⁷ or N;
T³ is CR⁶ or N;
T⁴ is CR⁵ or N;
T⁵ is CR⁴ or N;
Z is a 5- or 6-membered heterocyclic ring optionally substituted with oxo or 1 to 6 fluorine atoms, or Z is C₃₋₆ cycloalkyl optionally substituted with 1 to 6 fluorine atoms, or Z is -(CH₂)ₚCONHR¹³, or Z is -(CH₂)ₚCO₂R¹³;
L is -CR¹¹=CR¹²-, -CHR¹¹-CHR¹²- or -O-CHR¹¹-;
R¹ and R^{1a} are independently H or a C₁₋₆ saturated hydrocarbon group optionally substituted with 1 to 6 fluorine atoms, or R¹ and R^{1a} are linked together to form a 3 to 6-membered saturated ring optionally containing an additional heteroatom;
R² and R³ are independently H or C₁₋₃ alkyl optionally substituted with 1 to 6 fluorine atoms;
R⁴, R⁵, R⁶, R⁷ and R⁸ are independently H, halo, CN, CO₂R¹⁴, OR¹⁴, SO₂R¹⁴, SONHR¹⁴, OSO₂R¹⁴, PO(R¹⁴)₂, SF₅ or C₁₋₃ alkyl optionally substituted with 1 to 6 fluorine atoms;
R⁹ is H or is joined with X to form a C₃₋₆ cycloalkyl ring which is optionally substituted with 1 to 3 fluorine atoms or 1 to 3 C₁₋₃ alkyl groups;
R¹¹ and R¹² are independently H, -(CH₂)ₘCO₂R¹⁵ or C₁₋₃ alkyl optionally substituted with 1 to 6 fluorine atoms; or R¹¹ and R¹² are joined to form a cyclopropyl ring;
R¹³, R¹⁴ and R¹⁵ are independently H or C₁₋₃ alkyl optionally substituted with 1 to 6 fluorine atoms;
R¹⁶ is H or C₁₋₃ alkyl optionally substituted with 1 to 6 fluorine atoms;
p and m are independently 0-3;
wherein when R¹ and R^{1a} are not both H, L is:
-CHR¹¹-CHR¹²-;
-CR¹¹=CR¹²- where R³, R⁴, R⁵, R⁶, R⁷ and R⁸ are not all H;
or -O-CHR¹¹- where R³ is not H, or where two or more of R⁴, R⁵, R⁶, R⁷ and R⁸ are not H.

2. The compound according to claim 1, which is a compound of Formula (1c): 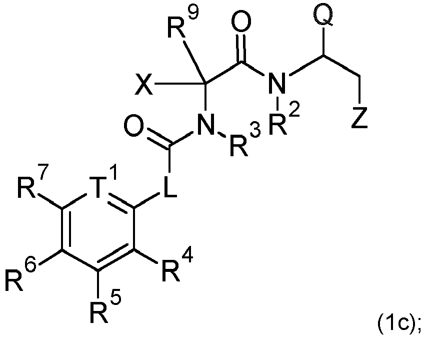 or a salt thereof.

3. The compound according to claim 1, which is a compound of Formula (1b): 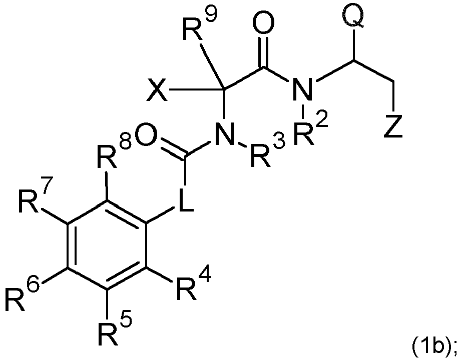 or a salt thereof, wherein;
Q is CN or a group of formula: 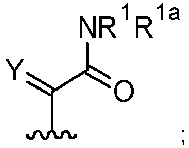
X is a C₁₋₆ saturated or unsaturated hydrocarbon group optionally substituted with 1 to 6 fluorine atoms or X is joined with R⁹ to form a C₃₋₆ cycloalkyl ring which is optionally substituted with 1 to 3 fluorine atoms or 1 to 3 C₁₋₃ alkyl groups;
Y is O or NOR¹⁶;
Z is a 5- or 6-membered heterocyclic ring optionally substituted with oxo or 1 to 6 fluorine atoms, or Z is C₃₋₆ cycloalkyl optionally substituted with 1 to 6 fluorine atoms, or Z is -(CH₂)ₚCONHR¹³, or Z is -(CH₂)ₚCO₂R¹³;
L is -CR¹¹=CR¹²-, -CHR¹¹-CHR¹²- or -O-CHR¹¹-;
R¹ and R^{1a} are independently H or a C₁₋₆ saturated hydrocarbon group optionally substituted with 1 to 6 fluorine atoms, or R¹ and R^{1a} are linked together to form a 3 to 6-membered saturated ring optionally containing an additional heteroatom;
R² and R³ are independently H or C₁₋₃ alkyl optionally substituted with 1 to 6 fluorine atoms;
R⁴, R⁵, R⁶, R⁷ and R⁸ are independently H, halo, CO₂R¹⁴ or C₁₋₃ alkyl optionally substituted with 1 to 6 fluorine atoms;
R⁹ is H or is joined with X to form a C₃₋₆ cycloalkyl ring which is optionally substituted with 1 to 3 fluorine atoms or 1 to 3 C₁₋₃ alkyl groups;
R¹¹ and R¹² are independently H, -(CH₂)ₘCO₂R¹⁵ or C₁₋₃ alkyl optionally substituted with 1 to 6 fluorine atoms;
R¹³, R¹⁴ and R¹⁵ are independently H or C₁₋₃ alkyl optionally substituted with 1 to 6 fluorine atoms;
R¹⁶ is H or C₁₋₃ alkyl optionally substituted with 1 to 6 fluorine atoms;
p and m are independently 0-3;
wherein when R¹ and R^{1a} are not both H, L is:
-CHR¹¹-CHR¹²-;
-CR¹¹=CR¹²- where R³, R⁴, R⁵, R⁶, R⁷ and R⁸ are not all H;
or -O-CHR¹¹- where R³ is not H, or where two or more of R⁴, R⁵, R⁶, R⁷ and R⁸ are not H;
preferably wherein
(a) Q is 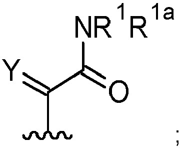 and
(b) R^{1a} is H..

4. The compound according to claim 1, which is a compound of Formula (1ai) or Formula (1aii): 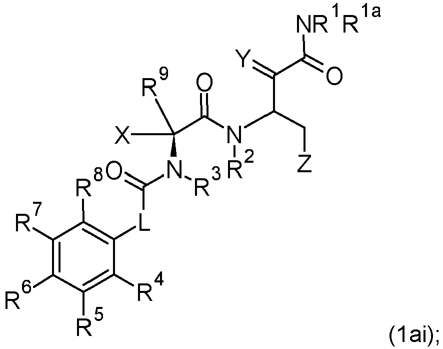 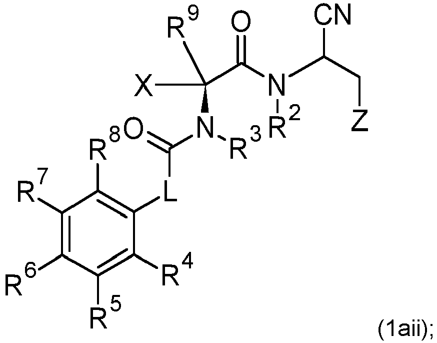 or a salt thereof.

5. The compound according to any one of claims 1 to 4, wherein Y is O.

6. The compound according to any one of claims 1 to 3, wherein R¹ and R^{1a} are independently H or selected from the group consisting of: 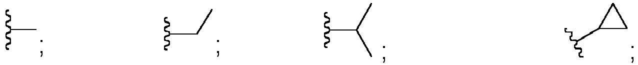 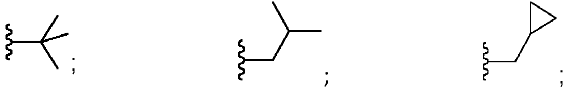 or wherein R¹ and R^{1a} are joined to form a ring such that the group NR¹ R^{1a} is:

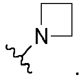

7. The compound according to any one of claims 1 to 6, wherein R² is H.

8. The compound according to any one of claims 1 to 7, wherein X is selected from the group consisting of:

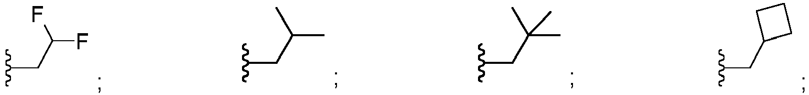

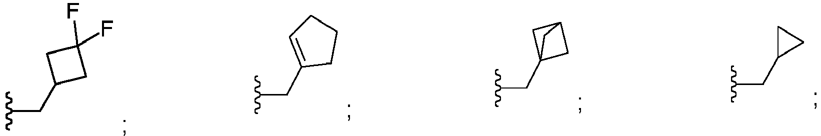

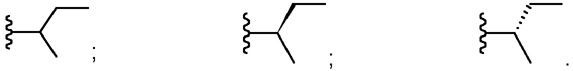

9. The compound according to claim 1, which is a compound of Formula (3bi), (3bii), (3ci) or (3cii): 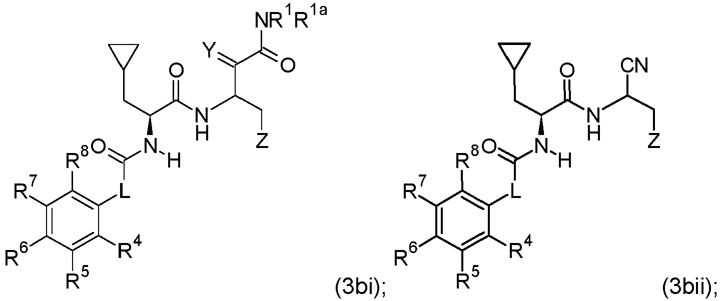 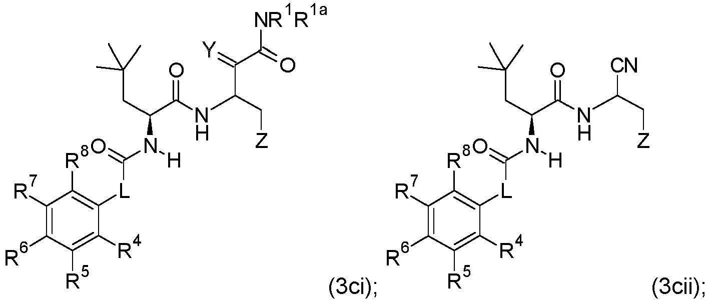 and salts thereof, wherein
Y is O or NOH;
Z is a 5- or 6-membered heterocyclic ring optionally substituted with oxo or 1 to 6 fluorine atoms, or Z is C₃₋₆ cycloalkyl optionally substituted with 1 to 6 fluorine atoms; L is -CH=CH-, -CH₂-CH₂- or -O-CH₂-;
R¹ and R^{1a} are independently H or a C₁₋₄ saturated hydrocarbon group optionally substituted with 1 to 6 fluorine atoms, or R¹ and R^{1a} are linked together to form a 3 to 6-membered saturated ring optionally containing an additional heteroatom;
R⁴, R⁵, R⁶, R⁷ and R⁸ are independently H or halo.

10. The compound according to any one of claims 1 to 9, wherein Z is selected from the group consisting of:

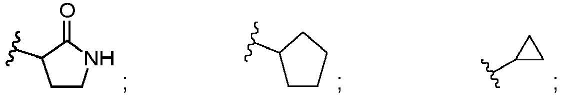

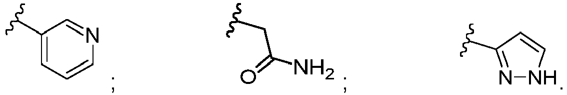

11. The compound according to claim 1, which is a compound of Formula (4ai) or (4aii): 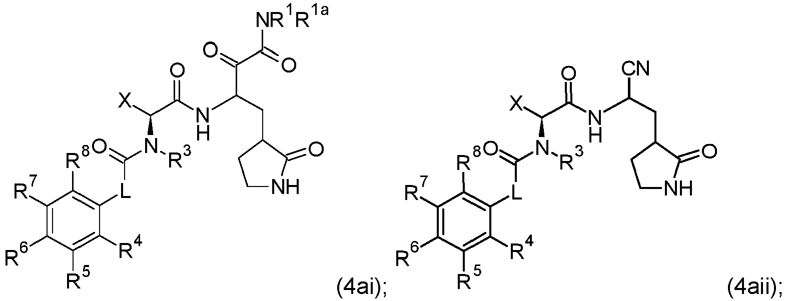 or a salt thereof.

12. The compound according to claim 1, wherein
R¹ and R^{1a} are both H and L is -CR¹¹=CR¹²- or -O-CHR¹¹-; or
R¹ and R^{1a} are not both H and
(i) L is -CR¹¹=CR¹²- where R³, R⁴, R⁵, R⁶, R⁷ and R⁸ are not all H or
(ii) L is -O-CHR¹¹- where R³ is not H, or where two or more of R⁴, R⁵, R⁶, R⁷ and R⁸ are not H.

13. The compound according to claim 1, wherein L is -CH=CH-, -CH₂CH₂-, -CH₂-CH(CH₂CH₃)-, -CH₂-CH(CH₂CO₂H)-, -OCH₂-, -CH₂-CH(CH₃)-, -CH₂-CH(CF₃)-, - OCH(CH₃)-, or

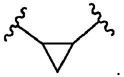

14. The compound according to any one of claims 1 to 13, wherein R³ is H, methyl or CH₂CF₃.

15. The compound according to claim 1, which is a compound of Formula (9b): 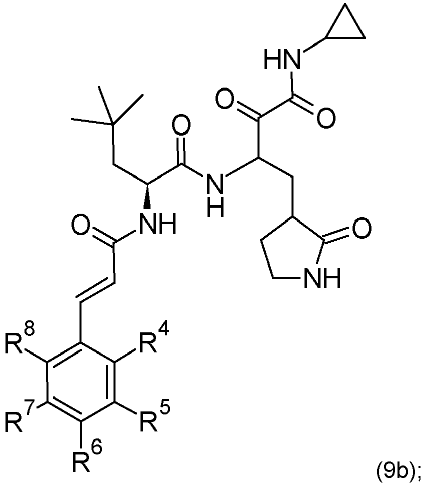 or a salt thereof.

16. The compound according to any one of claims 1 to 15, wherein R⁴, R⁵, R⁶, R⁷, and R⁸ are independently selected from H, CN, Cl, F, CF₂H, OCH₃, OCF₃, OCF₂H, SO₂CH₃, OSO₂CH₃, PO(CH₃)₂, SF₅ and CO₂H; preferably wherein R⁴ is F, R⁵ is H, R⁶ is Cl and R⁷ and R⁸ are H.

17. The compound according to claim 1 which is selected from the group consisting of:
3-((*S*)-2-((*E*)-3-(4-chloro-2-fluorophenyl)acrylamido)-3-cyclopropylpropanamido)-2-oxo-4-((*S*)-2-oxopyrrolidin-3-yl)butanamide;
3-((*S*)-2-cinnamamido-3-cyclopropylpropanamido)-2-oxo-4-((S)-2-oxopyrrolidin-3-yl)butanamide;
benzyl ((2*S*)-1-((4-amino-3,4-dioxo-1-((*S*)-2-oxopyrrolidin-3-yl)butan-2-yl)amino)-3-cyclopropyl-1-oxopropan-2-yl)carbamate;
3-((*S*)-2-((*E*)-3-(4-chloro-2-fluorophenyl)acrylamido)-3-cyclopropylpropanamido)-2-(hydroxyimino)-4-((*S*)-2-oxopyrrolidin-3-yl)butanamide;
*N*-(*tert*-butyl)-3-((*S*)-2-((*E*)-3-(4-chloro-2-fluorophenyl)acrylamido)-3-cyclopropylpropanamido)-2-oxo-4-((S)-2-oxopyrrolidin-3-yl)butanamide;
3-((*S*)-2-((*E*)-3-(4-chloro-2-fluorophenyl)acrylamido)-3-cyclopropylpropanamido)-4-cyclopropyl-2-oxobutanamide;
3-((*S*)-2-((*E*)-3-(4-chloro-2-fluorophenyl)acrylamido)-3-cyclopropylpropanamido)-4-cyclopentyl-2-oxobutanamide;
3-((*S*)-3-cyclopropyl-2-(3-phenylpropanamido)propanamido)-2-oxo-4-((*S*)-2-oxopyrrolidin-3-yl)butanamide;
(2*S*)-*N*-(1-cyano-2-((*S*)-2-oxopyrrolidin-3-yl)ethyl)-2-((*E*)-3-(2,4-dichlorophenyl)acrylamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*E*)-3-(2,4-dichlorophenyl)acrylamido)-4,4-dimethylpentanamide;
(2*S*)-2-((*E*)-3-(4-chloro-2-fluorophenyl)acrylamido)-*N*-(4-(cyclopropylamino)-3,4-dioxo-1-((*S*)-2-oxopyrrolidin-3-yl)butan-2-yl)-4,4-dimethylpentanamide
(2S)-*N-*(4-(cyclopropylamino)-3,4-dioxo-1-((*S*)-2-oxopyrrolidin-3-yl)butan-2-yl)-4,4-dimethyl-2-((*S*)-3-phenylbutanamido)pentanamide;
(2S)-*N*-(4-(cyclopropylamino)-3,4-dioxo-1-((*S*)-2-oxopyrrolidin-3-yl)butan-2-yl)-4,4-dimethyl-2-((*R*)-3-phenylbutanamido)pentanamide;
(2S)-*N*-(4-(cyclopropylamino)-3,4-dioxo-1-((*S*)-2-oxopyrrolidin-3-yl)butan-2-yl)-4,4-dimethyl-2-((*S*)-3-phenylpentanamido)pentanamide;
(2S)-*N*-(4-(cyclopropylamino)-3,4-dioxo-1-((*S*)-2-oxopyrrolidin-3-yl)butan-2-yl)-4,4-dimethyl-2-((*R*)-3-phenylpentanamido)pentanamide;
(2S)-*N*-(4-(cyclopropylamino)-3,4-dioxo-1-((*S*)-2-oxopyrrolidin-3-yl)butan-2-yl)-4,4-dimethyl-2-((*S*)-4,4,4-trifluoro-3-phenylbutanamido)pentanamide;
(2S)-*N*-(4-(cyclopropylamino)-3,4-dioxo-1-((*S*)-2-oxopyrrolidin-3-yl)butan-2-yl)-4,4-dimethyl-2-((*R*)-4,4,4-trifluoro-3-phenylbutanamido)pentanamide;
(2S)-*N*-(4-amino-3,4-dioxo-1-((*S*)-2-oxopyrrolidin-3-yl)butan-2-yl)-2-((*E*)-3-(4-chloro-2-fluorophenyl)acrylamido)-4-methylpentanamide;
3-((*S*)-2-((*E*)-3-(4-chloro-2-fluorophenyl)acrylamido)-3-cyclopropylpropanamido)-*N-*ethyl-2-oxo-4-((*S*)-2-oxopyrrolidin-3-yl)butanamide;
3-((*S*)-3-cyclopropyl-2-((*E*)-3-(2,4-dichlorophenyl)acrylamido)propanamido)-2-oxo-4-((*S*)-2-oxopyrrolidin-3-yl)butanamide;
3-((*S*)-2-((*E*)-3-(4-chloro-2-fluorophenyl)acrylamido)-3-cyclopropylpropanamido)-*N-*cyclopropyl-2-oxo-4-((S)-2-oxopyrrolidin-3-yl)butanamide;
(2*S*)-*N*-(4-amino-3,4-dioxo-1-((*S*)-2-oxopyrrolidin-3-yl)butan-2-yl)-2-((*E*)-3-(4-chloro-2-fluorophenyl)acrylamido)-4,4-dimethylpentanamide;
(2*S*)-2-((*E*)-3-(4-chloro-2-fluorophenyl)acrylamido)-*N*-(4-(ethylamino)-3,4-dioxo-1-((*S*)-2-oxopyrrolidin-3-yl)butan-2-yl)-4,4-dimethylpentanamide;
3-((*S*)-3-cyclopropyl-2-((*E*)-3-(2,4-dichlorophenyl)acrylamido)propanamido)-*N*-ethyl-2-oxo-4-((*S*)-2-oxopyrrolidin-3-yl)butanamide;
*N*-cyclopropyl-3-((*S*)-3-cyclopropyl-2-((*E*)-3-(2,4-dichlorophenyl)acrylamido)propanamido)-2-oxo-4-((*S*)-2-oxopyrrolidin-3-yl)butanamide;
(2*S*)-2-((*E*)-3-(2,4-dichlorophenyl)acrylamido)-*N*-(4-(ethylamino)-3,4-dioxo-1-((*S*)-2-oxopyrrolidin-3-yl)butan-2-yl)-4,4-dimethylpentanamide;
(2*S*)-*N*-(4-amino-3,4-dioxo-1-((*S*)-2-oxopyrrolidin-3-yl)butan-2-yl)-2-((E)-3-(2,4-dichlorophenyl)acrylamido)-4,4-dimethylpentanamide;
3-((*S*)-2-((*E*)-3-(4-chloro-2-fluorophenyl)acrylamido)-3-cyclopropylpropanamido)-N-cyclopropyl-2-oxo-4-((*S*)-2-oxopyrrolidin-3-yl)butanamide;
benzyl ((2*S*)-3-cyclopropyl-1-((4-(cyclopropylamino)-3,4-dioxo-1-((*S*)-2-oxopyrrolidin-3-yl)butan-2-yl)amino)-1-oxopropan-2-yl)carbamate;
*N*-cyclopropyl-3-((*S*)-3-cyclopropyl-2-(3-(2,4-dichlorophenyl)propanamido)propanamido)-2-oxo-4-((*S*)-2-oxopyrrolidin-3-yl)butanamide;
3-((*S*)-2-((*E*)-3-(4-chloro-2-fluorophenyl)acrylamido)-3-cyclobutylpropanamido)-N-cyclopropyl-2-oxo-4-((*S*)-2-oxopyrrolidin-3-yl)butanamide;
(3*S*)-*N*-((2*S*)-3-cyclopropyl-1-((4-(cyclopropylamino)-3,4-dioxo-1-((*S*)-2-oxopyrrolidin-3-yl)butan-2-yl)amino)-1-oxopropan-2-yl)-3-phenylpentanamide;
(3*R*)-*N*-((2*S*)-3-cyclopropyl-1-((4-(cyclopropylamino)-3,4-dioxo-1-((*S*)-2-oxopyrrolidin-3-yl)butan-2-yl)amino)-1-oxopropan-2-yl)-3-phenylpentanamide;
(2*S*)-*N*-(4-(azetidin-1-yl)-3,4-dioxo-1-((*S*)-2-oxopyrrolidin-3-yl)butan-2-yl)-2-((*E*)-3-(2,4-dichlorophenyl)acrylamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*E*)-3-(2,4-difluorophenyl)acrylamido)-4,4-dimethylpentanamide;
(2*S*)-2-((*E*)-3-(4-chlorophenyl)acrylamido)-*N*-(4-(cyclopropylamino)-3,4-dioxo-1-((*S*)-2-oxopyrrolidin-3-yl)butan-2-yl)-4,4-dimethylpentanamide;
(2*S*)-2-((*E*)-3-(2-chloro-4-fluorophenyl)acrylamido)-*N*-(4-(cyclopropylamino)-3,4-dioxo-1-((*S*)-2-oxopyrrolidin-3-yl)butan-2-yl)-4,4-dimethylpentanamide;
(2*S*)-*N*-(4-(cyclopropylamino)-3,4-dioxo-1-((*S*)-2-oxopyrrolidin-3-yl)butan-2-yl)-2-((*E*)-3-(4-fluorophenyl)acrylamido)-4,4-dimethylpentanamide;
(2*S*)-2-((*E*)-3-(4-chloro-3-fluorophenyl)acrylamido)-*N*-(4-(cyclopropylamino)-3,4-dioxo-1-((*S*)-2-oxopyrrolidin-3-yl)butan-2-yl)-4,4-dimethylpentanamide;
(2*S*)-2-((*E*)-3-(5-chloropyridin-2-yl)acrylamido)-*N*-(4-(cyclopropylamino)-3,4-dioxo-1-((*S*)-2-oxopyrrolidin-3-yl)butan-2-yl)-4,4-dimethylpentanamide;
(2*S*)-*N*-(4-(cyclopropylamino)-3,4-dioxo-1-((*S*)-2-oxopyrrolidin-3-yl)butan-2-yl)-2-((*E*)-3-(5-fluoropyridin-2-yl)acrylamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*E*)-3-(4-(difluoromethyl)phenyl)acrylamido)-4,4-dimethylpentanamide;
(1*R*,2*R*)-*N*-((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)-2-phenylcyclopropane-1-carboxamide;
(1*S*,2*S*)-*N*-((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)-2-phenylcyclopropane-1-carboxamide;
(2*S*)-*N*-(4-(cyclopropylamino)-3,4-dioxo-1-((*S*)-2-oxopyrrolidin-3-yl)butan-2-yl)-2-((*S*)-3-(2,4-difluorophenyl)pentanamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*R*)-3-(2,4-difluorophenyl)pentanamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*S*)-3-(2,4-dichlorophenyl)pentanamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*R*)-3-(2,4-dichlorophenyl)pentanamido)-4,4-dimethylpentanamide;
(2*S*)-2-((*S*)-3-(4-chloro-2-fluorophenyl)pentanamido)-*N*-(4-(cyclopropylamino)-3,4-dioxo-1-((S)-2-oxopyrrolidin-3-yl)butan-2-yl)-4,4-dimethylpentanamide;
(2*S*)-2-((*R*)-3-(4-chloro-2-fluorophenyl)pentanamido)-*N*-(4-(cyclopropylamino)-3,4-dioxo-1-((S)-2-oxopyrrolidin-3-yl)butan-2-yl)-4,4-dimethylpentanamide;
(2*S*)-2-((*S*)-3-(4-chlorophenyl)pentanamido)-*N*-(4-(cyclopropylamino)-3,4-dioxo-1-((S)-2-oxopyrrolidin-3-yl)butan-2-yl)-4,4-dimethylpentanamide;
(2*S*)-2-((*R*)-3-(4-chlorophenyl)pentanamido)-*N*-(4-(cyclopropylamino)-3,4-dioxo-1-((S)-2-oxopyrrolidin-3-yl)butan-2-yl)-4,4-dimethylpentanamide;
(2*S*)-*N*-(4-(cyclopropylamino)-3,4-dioxo-1-((*S*)-2-oxopyrrolidin-3-yl)butan-2-yl)-2-((*S*)-3-(4-fluorophenyl)pentanamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*R*)-3-(4-fluorophenyl)pentanamido)-4,4-dimethylpentanamide;
(2S)-*N*-(4-(azetidin-1-yl)-3,4-dioxo-1-((*S*)-2-oxopyrrolidin-3-yl)butan-2-yl)-2-((*E*)-3-(4-chloro-2-fluorophenyl)acrylamido)-4,4-dimethylpentanamide;
(2*S*)-*N*-(4-(cyclopropylamino)-3,4-dioxo-1-((*S*)-2-oxopyrrolidin-3-yl)butan-2-yl)-4,4-dimethyl-2-((R)-3-(2-(trifluoromethoxy)phenyl)pentanamido)pentanamide;
(2*S*)-*N*-(4-(cyclopropylamino)-3,4-dioxo-1-((*S*)-2-oxopyrrolidin-3-yl)butan-2-yl)-4,4-dimethyl-2-((S)-3-(2-(trifluoromethoxy)phenyl)pentanamido)pentanamide;
(2*S*)-*N*-(4-(cyclopropylamino)-3,4-dioxo-1-((*S*)-2-oxopyrrolidin-3-yl)butan-2-yl)-2-((*R*)-3-(2-(difluoromethoxy)phenyl)pentanamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*S*)-3-(2-(difluoromethoxy)phenyl)pentanamido)-4,4-dimethylpentanamide;
(2*S*)-2-((*E*)-3-(4-chloro-2-cyanophenyl)acrylamido)-*N*-(4-(cyclopropylamino)-3,4-dioxo-1-((S)-2-oxopyrrolidin-3-yl)butan-2-yl)-4,4-dimethylpentanamide;
(2*S*)-2-((*E*)-3-(4-chloro-3-cyanophenyl)acrylamido)-*N*-(4-(cyclopropylamino)-3,4-dioxo-1-((S)-2-oxopyrrolidin-3-yl)butan-2-yl)-4,4-dimethylpentanamide;
(2*S*)-2-((*E*)-3-(2-chloro-4-cyanophenyl)acrylamido)-*N*-(4-(cyclopropylamino)-3,4-dioxo-1-((S)-2-oxopyrrolidin-3-yl)butan-2-yl)-4,4-dimethylpentanamide;
(2*S*)-*N*-(4-(cyclopropylamino)-3,4-dioxo-1-((*S*)-2-oxopyrrolidin-3-yl)butan-2-yl)-4,4-dimethyl-2-((R)-3-(3-(trifluoromethoxy)phenyl)butanamido)pentanamide;
(2*S*)-*N*-(4-(cyclopropylamino)-3,4-dioxo-1-((*S*)-2-oxopyrrolidin-3-yl)butan-2-yl)-4,4-dimethyl-2-((*S*)-3-(3-(trifluoromethoxy)phenyl)butanamido)pentanamide;
(2*S*)-*N*-(4-(cyclopropylamino)-3,4-dioxo-1-((*S*)-2-oxopyrrolidin-3-yl)butan-2-yl)-2-((*R*)-3-(3-(difluoromethoxy)phenyl)butanamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*S*)-3-(3-(difluoromethoxy)phenyl)butanamido)-4,4-dimethylpentanamide;
(2*S*)-2-((*R*)-3-(2-chloro-4-(methylsulfonyl)phenyl)pentanamido)-*N*-(4-(cyclopropylamino)-3,4-dioxo-1-((*S*)-2-oxopyrrolidin-3-yl)butan-2-yl)-4,4-dimethylpentanamide;
(2*S*)-2-((*S*)-3-(2-chloro-4-(methylsulfonyl)phenyl)pentanamido)-*N*-(4-(cyclopropylamino)-3,4-dioxo-1-((*S*)-2-oxopyrrolidin-3-yl)butan-2-yl)-4,4-dimethylpentanamide;
3-chloro-4-((3*R*)-1-(((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)amino)-1-oxopentan-3-yl)phenyl methanesulfonate;
3-chloro-4-((3*S*)-1-(((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)amino)-1-oxopentan-3-yl)phenyl methanesulfonate;
(2*S*)-2-((*R*)-3-(2-chloro-4-(dimethylphosphoryl)phenyl)pentanamido)-*N*-(4-(cyclopropylamino)-3,4-dioxo-1-((*S*)-2-oxopyrrolidin-3-yl)butan-2-yl)-4,4-dimethylpentanamide;
(2*S*)-2-((*S*)-3-(2-chloro-4-(dimethylphosphoryl)phenyl)pentanamido)-*N*-(4-(cyclopropylamino)-3,4-dioxo-1-((*S*)-2-oxopyrrolidin-3-yl)butan-2-yl)-4,4-dimethylpentanamide;
(2*S*)-*N*-(4-(cyclopropylamino)-3,4-dioxo-1-((*S*)-2-oxopyrrolidin-3-yl)butan-2-yl)-4,4-dimethyl-2-((*E*)-3-(4-(pentafluoro-l6-sulfaneyl)phenyl)acrylamido)pentanamide;
(2*S*)-*N*-(4-(cyclopropylamino)-3,4-dioxo-1-((*S*)-2-oxopyrrolidin-3-yl)butan-2-yl)-2-((*R*)-3-(4-methoxyphenyl)pentanamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*S*)-3-(4-methoxyphenyl)pentanamido)-4,4-dimethylpentanamide;
(2*S*)-2-(3-(4-chloro-2-(trifluoromethoxy)phenyl)propanamido)-*N*-(4-(cyclopropylamino)-3,4-dioxo-1-((*S*)-2-oxopyrrolidin-3-yl)butan-2-yl)-4,4-dimethylpentanamide;
(2*S*)-2-(3-(4-chloro-2-(difluoromethoxy)phenyl)propanamido)-*N*-(4-(cyclopropylamino)-3,4-dioxo-1-((*S*)-2-oxopyrrolidin-3-yl)butan-2-yl)-4,4-dimethylpentanamide;
(2*S*)-2-(3-(4-chloro-2-cyanophenyl)propanamido)-*N*-(4-(cyclopropylamino)-3,4-dioxo-1-((*S*)-2-oxopyrrolidin-3-yl)butan-2-yl)-4,4-dimethylpentanamide;
(2*S*)-2-(3-(2-chloro-4-cyanophenyl)propanamido)-*N*-(4-(cyclopropylamino)-3,4-dioxo-1-((*S*)-2-oxopyrrolidin-3-yl)butan-2-yl)-4,4-dimethylpentanamide;
(2*S*)-2-(3-(4-chloro-3-(trifluoromethoxy)phenyl)propanamido)-*N*-(4-(cyclopropylamino)-3,4-dioxo-1-((*S*)-2-oxopyrrolidin-3-yl)butan-2-yl)-4,4-dimethylpentanamide;
(2*S*)-*N*-(1-cyano-2-((*S*)-2-oxopyrrolidin-3-yl)ethyl)-2-(3-(2,4-dichlorophenyl)propanamido)-4,4-dimethylpentanamide;
2,4-dichlorobenzyl ((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)carbamate;
4-chloro-2-fluorobenzyl ((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)carbamate;
(*S*)-1-(4-chloro-2-fluorophenyl)ethyl ((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)carbamate;
(*R*)-1-(4-chloro-2-fluorophenyl)ethyl ((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)carbamate;
(2*S*)-*N*-(4-(cyclopropylamino)-3,4-dioxo-1-((*S*)-2-oxopyrrolidin-3-yl)butan-2-yl)-2-((*R*)-3-(2,4-difluorophenyl)-4,4,4-trifluorobutanamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*S*)-3-(2,4-difluorophenyl)-4,4,4-trifluorobutanamido)-4,4-dimethylpentanamide;
(2*S*)-2-((*R*)-2-benzyl-3,3,3-trifluoropropanamido)-*N*-(4-(cyclopropylamino)-3,4-dioxo-1-((*S*)-2-oxopyrrolidin-3-yl)butan-2-yl)-4,4-dimethylpentanamide;
(2*S*)-2-((*S*)-2-benzyl-3,3,3-trifluoropropanamido)-*N*-(4-(cyclopropylamino)-3,4-dioxo-1-((*S*)-2-oxopyrrolidin-3-yl)butan-2-yl)-4,4-dimethylpentanamide;
(1*S*,2*S*)-2-(4-chlorophenyl)-*N*-((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)cyclopropane-1-carboxamide;
(2*S*)-*N*-(4-(cyclopropylamino)-3,4-dioxo-1-((*S*)-2-oxopyrrolidin-3-yl)butan-2-yl)-2-(2-(2,4-dichlorophenoxy)acetamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*S*)-2-(2,4-dichlorophenoxy)propanamido)-4,4-dimethylpentanamide;
(2*S*)-*N*-(4-(cyclopropylamino)-3,4-dioxo-1-((*S*)-2-oxopyrrolidin-3-yl)butan-2-yl)-2-((*R*)-2-(2,4-dichlorophenoxy)propanamido)-4,4-dimethylpentanamide;
or a salt thereof.

18. A pharmaceutical composition comprising a compound as defined in any one of claims 1 to 17 and a pharmaceutically acceptable excipient.

19. The compound or composition according to any one of claims 1 to 18 for use in the treatment of SARS-CoV-2 or in the treatment of disorders associated with SARS-CoV-2: Mpro.

## Patentansprüche

1. Verbindung der Formel (1'): 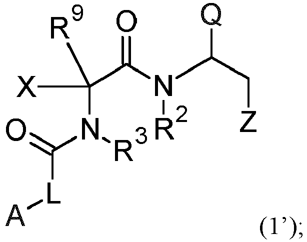 oder ein Salz davon, wobei:
A aus Folgenden ausgewählt ist: 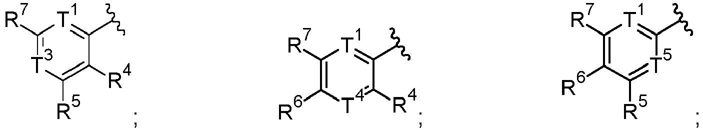 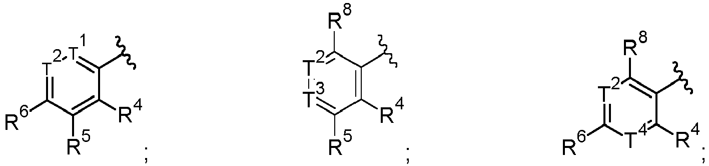
Q CN oder eine Gruppe der folgenden Formel ist: 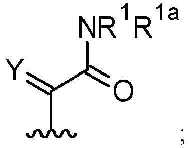
X eine gesättigte oder ungesättigte C₁₋₆-Kohlenwasserstoffgruppe ist, die optional mit 1 bis 6 Fluoratomen substituiert ist, oder X mit R⁹ verbunden ist, um einen C₃₋₆-Cycloalkylring zu bilden, der optional mit 1 bis 3 Fluoratomen oder 1 bis 3 C₁₋₃-Alkylgruppen substituiert ist;
Y O oder NOR¹⁶ ist;
T¹ CR⁸ oder N ist;
T² CR⁷ oder N ist;
T³ CR⁶ oder N ist;
T⁴ CR⁵ oder N ist;
T⁵ CR⁴ oder N ist;
Z ein 5- oder 6-gliedriger heterocyclischer Ring ist, der optional mit Oxo oder 1 bis 6 Fluoratomen substituiert ist, oder Z C₃₋₆-Cycloalkyl ist, das optional mit 1 bis 6 Fluoratomen substituiert ist oder Z -(CH₂)ₚCONHR¹³ ist oder Z -(CH₂)ₚCO₂R¹³ ist;
L -CR¹¹=CR¹²-, -CHR¹¹-CHR¹²- oder -O-CHR¹¹- ist;
R¹ und R^{1a} unabhängig H oder eine gesättigte C₁₋₆-Kohlenwasserstoffgruppe sind, die optional mit 1 bis 6 Fluoratomen substituiert ist, oder R¹ und R^{1a} miteinander verknüpft sind, um einen 3- bis 6-gliedrigen gesättigten Ring zu bilden, der optional ein zusätzliches Heteroatom enthält;
R² und R³ unabhängig H oder C₁₋₃-Alkyl sind, das optional mit 1 bis 6 Fluoratomen substituiert ist;
R⁴, R⁵, R⁶, R⁷ und R⁸ unabhängig Folgendes sind: H, Halogen, CN, CO₂R¹⁴, OR¹⁴, SO₂R¹⁴, SONHR¹⁴, OSO₂R¹⁴, PO(R¹⁴)₂, SF₅ oder C₁₋₃-Alkyl, das optional mit 1 bis 6 Fluoratomen substituiert ist;
R⁹ H ist oder mit X verbunden ist, um einen C₃₋₆-Cycloalkylring zu bilden, der optional mit 1 bis 3 Fluoratomen oder 1 bis 3 C₁₋₃-Alkylgruppen substituiert ist;
R¹¹ und R¹² unabhängig Folgendes sind: H, -(CH₂)ₘCO₂R¹⁵ oder C₁₋₃-Alkyl, das optional mit 1 bis 6 Fluoratomen substituiert ist; oder R¹¹ und R¹² verbunden sind, um einen Cyclopropylring zu bilden,
R¹³, R¹⁴ und R¹⁵ unabhängig H oder C₁₋₃-Alkyl sind, das optional mit 1 bis 6 Fluoratomen substituiert ist;
R¹⁶ H oder C₁₋₃-Alkyl ist, das optional mit 1 bis 6 Fluoratomen substituiert ist;
p und m unabhängig 0-3 sind,
wobei, wenn R¹ und R^{1a} nicht beide H sind, L Folgendes ist:
-CHR¹¹-CHR¹²-;
-CR¹¹=CR¹²-, wo R³, R⁴, R⁵, R⁶, R⁷ und R⁸ nicht alle H sind;
oder -O-CHR¹¹-, wo R³ nicht H ist oder wo zwei oder mehr von R⁴, R⁵, R⁶, R⁷ und R⁸ nicht H sind.

2. Verbindung nach Anspruch 1, die eine Verbindung der Formel (1c) ist: 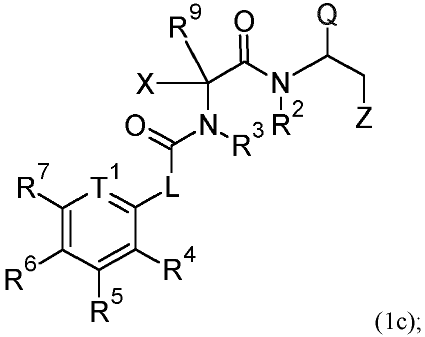 oder ein Salz davon.

3. Verbindung nach Anspruch 1, die eine Verbindung der Formel (1b) ist: 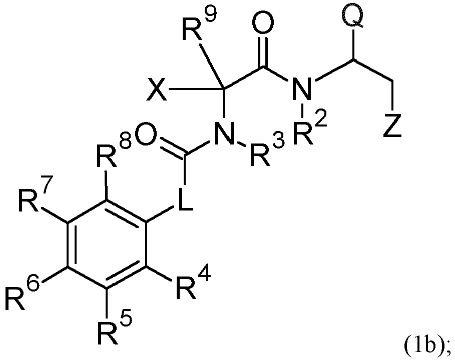 oder ein Salz davon, wobei:
Q CN oder eine Gruppe der folgenden Formel ist: 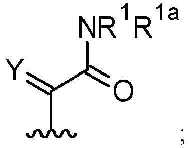
X eine gesättigte oder ungesättigte C₁₋₆-Kohlenwasserstoffgruppe ist, die optional mit 1 bis 6 Fluoratomen substituiert ist, oder X mit R⁹ verbunden ist, um einen C₃₋₆-Cycloalkylring zu bilden, der optional mit 1 bis 3 Fluoratomen oder 1 bis 3 C₁₋₃-Alkylgruppen substituiert ist;
Y O oder NOR¹⁶ ist;
Z ein 5- oder 6-gliedriger heterocyclischer Ring ist, der optional mit Oxo oder 1 bis 6 Fluoratomen substituiert ist, oder Z C₃₋₆-Cycloalkyl ist, das optional mit 1 bis 6 Fluoratomen substituiert ist oder Z -(CH₂)ₚCONHR¹³ ist oder Z -(CH₂)ₚCO₂R¹³ ist;
L -CR¹¹=CR¹²-, -CHR¹¹-CHR¹²- oder -O-CHR¹¹- ist;
R¹ und R^{1a} unabhängig H oder eine gesättigte C₁₋₆-Kohlenwasserstoffgruppe sind, die optional mit 1 bis 6 Fluoratomen substituiert ist, oder R¹ und R^{1a} miteinander verknüpft sind, um einen 3- bis 6-gliedrigen gesättigten Ring zu bilden, der optional ein zusätzliches Heteroatom enthält;
R² und R³ unabhängig H oder C₁₋₃-Alkyl sind, das optional mit 1 bis 6 Fluoratomen substituiert ist;
R⁴, R⁵, R⁶, R⁷ und R⁸ unabhängig H, Halogen, CN, CO₂R¹⁴ oder C₁₋₃-Alkyl sind, das optional mit 1 bis 6 Fluoratomen substituiert ist;
R⁹ H ist oder mit X verbunden ist, um einen C₃₋₆-Cycloalkylring zu bilden, der optional mit 1 bis 3 Fluoratomen oder 1 bis 3 C₁₋₃-Alkylgruppen substituiert ist;
R¹¹ und R¹² unabhängig H, -(CH₂)ₘCO₂R¹⁵ oder C₁₋₃-Alkyl sind, das optional mit 1 bis 6 Fluoratomen substituiert ist;
R¹³, R¹⁴ und R¹⁵ unabhängig H oder C₁₋₃-Alkyl sind, das optional mit 1 bis 6 Fluoratomen substituiert ist;
R¹⁶ H oder C₁₋₃-Alkyl ist, das optional mit 1 bis 6 Fluoratomen substituiert ist;
p und m unabhängig 0-3 sind,
wobei, wenn R¹ und R^{1a} nicht beide H sind, L Folgendes ist:
-CHR¹¹-CHR¹²-;
-CR¹¹=CR¹²-, wo R³, R⁴, R⁵, R⁶, R⁷ und R⁸ nicht alle H sind,
oder -O-CHR¹¹-, wo R³ nicht H ist oder wo zwei oder mehr von R⁴, R⁵, R⁶, R⁷ und R⁸ nicht H sind,
bevorzugt wobei
(a) Q 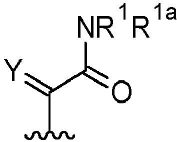 ist; und
(b) R^{1a} H ist.

4. Verbindung nach Anspruch 1, die eine Verbindung der Formel (1ai) oder Formel (1aii) ist: 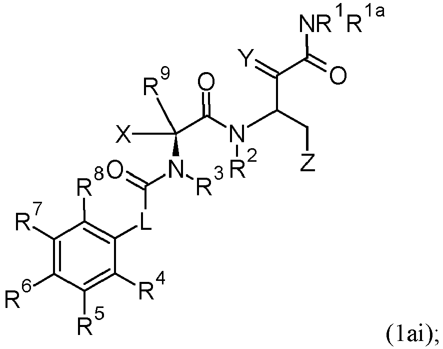 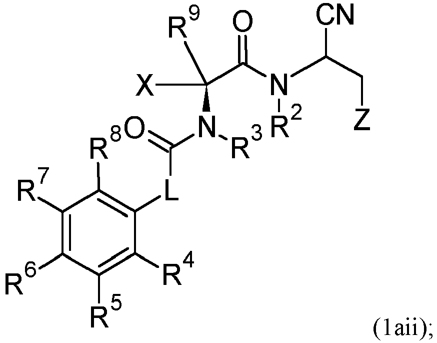 oder ein Salz davon.

5. Verbindung nach einem der Ansprüche 1 bis 4, wobei Y O ist.

6. Verbindung nach einem der Ansprüche 1 bis 3, wobei R¹ und R^{1a} unabhängig H sind oder aus der Gruppe ausgewählt sind, die aus Folgenden besteht: 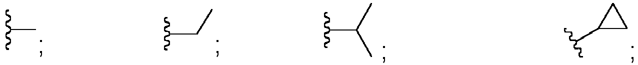 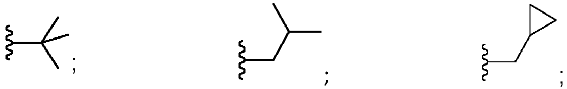 oder wobei R¹ und R^{1a} verbunden sind, um einen Ring zu bilden, sodass die Gruppe NR¹R^{1a} Folgendes ist:

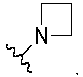

7. Verbindung nach einem der Ansprüche 1 bis 6, wobei R² H ist.

8. Verbindung nach einem der Ansprüche 1 bis 7, wobei X aus der Gruppe ausgewählt ist, die aus Folgenden besteht:

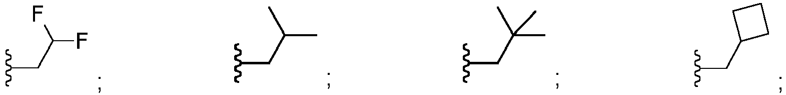

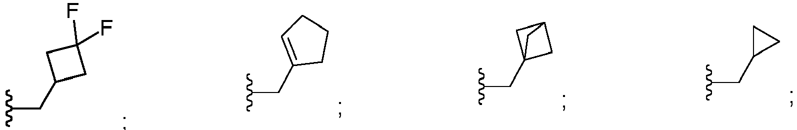

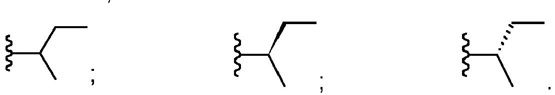

9. Verbindung nach Anspruch 1, die eine Verbindung der Formel (3bi), (3bii), (3ci) oder (3cii) ist: 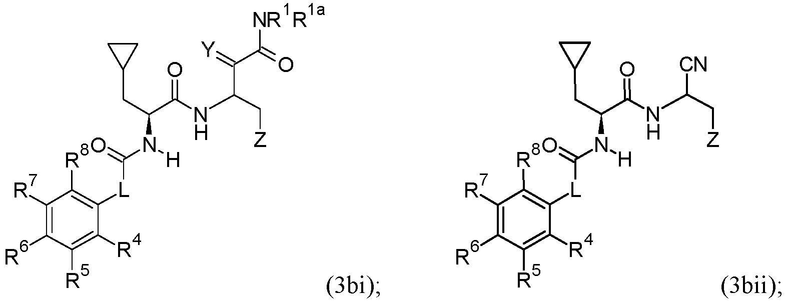 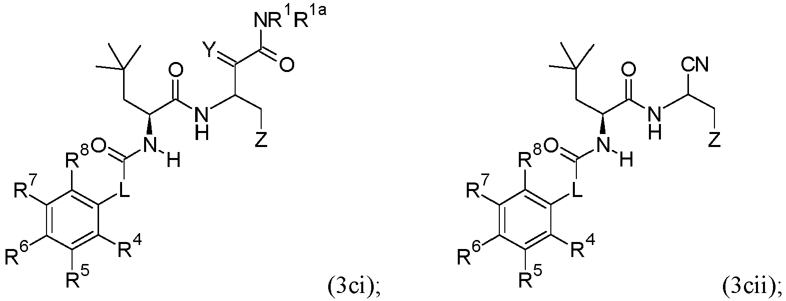 und Salze davon, wobei
Y O oder NOH ist;
Z ein 5- oder 6-gliedriger heterocyclischer Ring ist, der optional mit Oxo oder 1 bis 6 Fluoratomen substituiert ist, oder Z C₃₋₆-Cycloalkyl ist, das optional mit 1 bis 6 Fluoratomen substituiert ist;
L -CH=CH-, -CH₂-CH₂- oder -O-CH₂- ist;
R¹ und R^{1a} unabhängig H oder eine gesättigte C₁₋₄-Kohlenwasserstoffgruppe sind, die optional mit 1 bis 6 Fluoratomen substituiert ist, oder R¹ und R^{1a} miteinander verknüpft sind, um einen 3- bis 6-gliedrigen gesättigten Ring zu bilden, der optional ein zusätzliches Heteroatom enthält;
R⁴, R⁵, R⁶, R⁷ und R⁸ unabhängig H oder Halogen sind.

10. Verbindung nach einem der Ansprüche 1 bis 9, wobei Z aus der Gruppe ausgewählt ist, die aus Folgenden besteht:

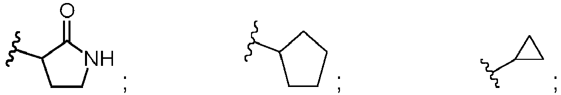

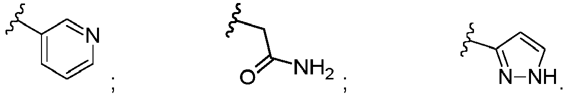

11. Verbindung nach Anspruch 1, die eine Verbindung der Formel (4ai) oder (4aii) ist: 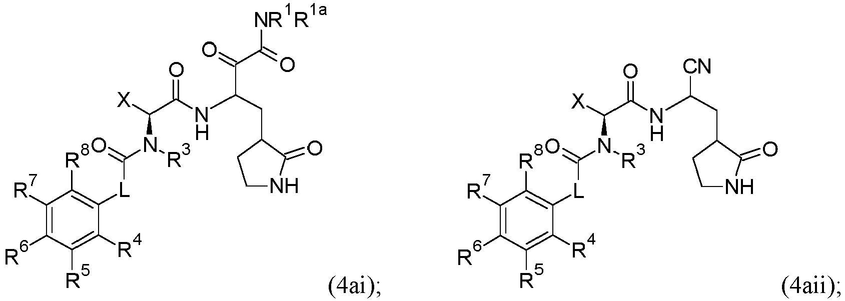 oder ein Salz davon.

12. Verbindung nach Anspruch 1, wobei
R¹ und R^{1a} beide H sind und L -CR¹¹=CR¹²- oder -O-CHR¹¹- ist; oder
R¹ and R^{1a} nicht beide H sind und
(i) L -CR¹¹=CR¹²- ist, wo R³, R⁴, R⁵, R⁶, R⁷ und R⁸ nicht alle H sind, oder
(ii) L -O-CHR¹¹- ist, wo R³ nicht H ist oder wo zwei oder mehr von R⁴, R⁵, R⁶, R⁷ und R⁸ nicht H sind.

13. Verbindung nach Anspruch 1, wobei L Folgendes ist: -CH=CH-, -CH₂CH₂-, - CH₂-CH(CH₂CH₃)-, -CH₂-CH(CH₂CO₂H)-, -OCH₂-, -CH₂-CH(CH₃)-, -CH₂-CH(CF₃)-, -OCH(CH₃)- oder

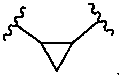

14. Verbindung nach einem der Ansprüche 1 bis 13, wobei R³ H, Methyl oder CH₂CF₃ ist.

15. Verbindung nach Anspruch 1, die eine Verbindung der Formel (9b) ist: 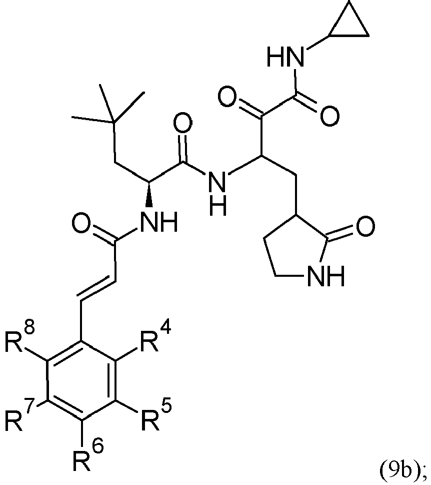 oder ein Salz davon.

16. Verbindung nach einem der Ansprüche 1 bis 15, wobei R⁴, R⁵, R⁶, R⁷ und R⁸ unabhängig aus Folgenden ausgewählt sind: H, CN, Cl, F, CF₂H, OCH₃, OCF₃, OCF₂H, SO₂CH₃, OSO₂CH₃, PO(CH₃)₂, SF₅ und CO₂H; bevorzugt wobei R⁴ F ist, R⁵ H ist, R⁶ Cl ist und R⁷ und R⁸ H sind.

17. Verbindung nach Anspruch 1, die aus der Gruppe ausgewählt ist, die aus Folgenden besteht:
3-((*S*)-2-((*E*)-3-(4-Chlor-2-fluorphenyl)acrylamido)-3-cyclopropylpropanamido)-2-oxo-4-((*S*)-2-oxopyrrolidin-3-yl)butanamid;
3-((*S*)-2-Cinnamamido-3-cyclopropylpropanamido)-2-oxo-4-((*S*)-2-oxopyrrolidin-3-yl)butanamid;
Benzyl-((2*S*)-1-((4-amino-3,4-dioxo-1-((*S*)-2-oxopyrrolidin-3-yl)butan-2-yl)amino)-3-cyclopropyl-1-oxopropan-2-yl)carbamat;
3-((*S*)-2-((*E*)-3-(4-Chlor-2-fluorphenyl)acrylamido)-3-cyclopropylpropanamido)-2-(hydroxyimino)-4-((*S*)-2-oxopyrrolidin-3-yl)butanamid;
*N*-(*tert*-Butyl)-3-((*S*)-2-((*E*)-3-(4-chlor-2-fluorphenyl)acrylamido)-3-cyclopropylpropanamido)-2-oxo-4-((*S*)-2-oxopyrrolidin-3-yl)butanamid;
3-((*S*)-2-((*E*)-3-(4-Chlor-2-fluorphenyl)acrylamido)-3-cyclopropylpropanamido)-4-cyclopropyl-2-oxobutanamid;
3-((*S*)-2-((*E*)-3-(4-Chlor-2-fluorphenyl)acrylamido)-3-cyclopropylpropanamido)-4-cyclopentyl-2-oxobutanamid;
3-((*S*)-3-Cyclopropyl-2-(3-phenylpropanamido)propanamido)-2-oxo-4-((*S*)-2-oxopyrrolidin-3-yl)butanamid;
(2*S*)-*N*-(1-Cyano-2-(*S*)-2-oxopyrrolidin-3-yl)ethyl)-2-(*E*)-3-(2,4-dichlorphenyl)acrylamido)-4,4-dimethylpentanamid;
(2*S*)-*N*-(4-(Cyclopropylamino)-3,4-dioxo-1-(*S*)-2-oxopyrrolidin-3-yl)butan-2-yl)-2-((*E*)-3-(2,4-dichlorphenyl)acrylamido)-4,4-dimethylpentanamid;
(2*S*)-2-(*E*)-3-(4-Chlor-2-fluorphenyl)acrylamido)-*N*-(4-(cyclopropylamino)-3,4-dioxo-1-((*S*)-2-oxopyrrolidin-3-yl)butan-2-yl)-4,4-dimethylpentanamid;
(2*S*)-*N*-(4-(Cyclopropylamino)-3,4-dioxo-1-((*S*)-2-oxopyrrolidin-3-yl)butan-2-yl)-4,4-dimethyl-2-((*S*)-3-phenylbutanamido)pentanamid;
(2*S*)-*N*-(4-(Cyclopropylamino)-3,4-dioxo-1-((*S*)-2-oxopyrrolidin-3-yl)butan-2-yl)-4,4-dimethyl-2-((*R*)-3-phenylbutanamido)pentanamid;
(2*S*)-*N*-(4-(Cyclopropylamino)-3,4-dioxo-1-((*S*)-2-oxopyrrolidin-3-yl)butan-2-yl)-4,4-dimethyl-2-((*S*)-3-phenylpentanamido)pentanamid;
(2*S*)-*N*-(4-(Cyclopropylamino)-3,4-dioxo-1-((*S*)-2-oxopyrrolidin-3-yl)butan-2-yl)-4,4-dimethyl-2-((*R*)-3-phenylpentanamido)pentanamid;
(2*S*)-*N*-(4-(Cyclopropylamino)-3,4-dioxo-((*S*)-2-oxopyrrolidin-3-yl)butan-2-yl)-4,4-dimethyl-2-((*S*)-4,4,4-trifluor-3-phenylbutanamido)pentanamid;
(2*S*)-*N*-(4-(Cyclopropylamino)-3,4-dioxo-1-(*S*)-2-oxopyrrolidin-3-yl)butan-2-yl)-4,4-dimethyl-2-((*R*)-4,4,4-trifluor-3-phenylbutanamido)pentanamid;
(2*S*)-*N*-(4-Amino-3,4-dioxo-1-((*S*)-2-oxopyrrolidin-3-yl)butan-2-yl)-2-((*E*)-3-(4-chlor-2-fluorphenyl)acrylamido)-4-methylpentanamid;
3-((*S*)-2-((*E*)-3-(4-Chlor-2-fluorphenyl)acrylamido)-3-cyclopropylpropanamido)-*N-*ethyl-2-oxo-4-((*S*)-2-oxopyrrolidin-3-yl)butanamid;
3-((*S*)-3-Cyclopropyl-2-((*E*)-3-(2,4-dichlorphenyl)acrylamido)propanamido)-2-oxo-4-((*S*)-2-oxopyrrolidin-3-yl)butanamid;
3-((*S*)-2-((*E*)-3-(4-Chlor-2-fluorphenyl)acrylamido)-3-cyclopropylpropanamido)-*N-*cyclopropyl-2-oxo-4-((*S*)-2-oxopyrrolidin-3 -yl)butanamid;
(2*S*)-*N*-(4-Amino-3,4-dioxo-1-((*S*)-2-oxopyrrolidin-3-yl)butan-2-yl)-2-((*E*)-3-(4-chlor-2-fluorphenyl)acrylamido)-4,4-dimethylpentanamid;
(2*S*)-2-((*E*)-3-(4-Chlor-2-fluorphenyl)acrylamido)-*N*-(4-(ethylamino)-3,4-dioxo-1-((*S*)-2-oxopyrrolidin-3-yl)butan-2-yl)-4,4-dimethylpentanamid;
3-((*S*)-3-Cyclopropyl-2-((*E*)-3-(2,4-dichlorphenyl)acrylamido)propanamido)-*N*-ethyl-2-oxo-4-((*S*)-2-oxopyrrolidin-3-yl)butanamid;
*N*-Cyclopropyl-3-((*S*)-3-cyclopropyl-2-((*E*)-3-(2,4-dichlorphenyl)acrylamido)propanamido)-2-oxo-4-((*S*)-2-oxopyrrolidin-3-yl)butanamid;
(2*S*)-2-((*E*)-3-(2,4-Dichlorphenyl)acrylamido)-*N*-(4-(ethylamino)-3,4-dioxo-1-((*S*)-2-oxopyrrolidin-3-yl)butan-2-yl)-4,4-dimethylpentanamid;
(2*S*)-*N*-(4-Amino-3,4-dioxo-1-((*S*)-2-oxopyrrolidin-3-yl)butan-2-yl)-2-((*E*)-3-(2,4-dichlorphenyl)acrylamido)-4,4-dimethylpentanamid;
3-((*S*)-2-((*E*)-3-(4-Chlor-2-fluorphenyl)acrylamido)-3-cyclopropylpropanamido)-*N-*cyclopropyl-2-oxo-4-((*S*)-2-oxopyrrolidin-3-yl)butanamid;
Benzyl-((2*S*)-3-cyclopropyl-1-((4-(cyclopropylamino)-3,4-dioxo-1-((*S*)-2-oxopyrrolidin-3-yl)butan-2-yl)amino)-1-oxopropan-2-yl)carbamat;
*N*-Cyclopropyl-3-((*S*)-3-cyclopropyl-2-(3-(2,4-dichlorphenyl)propanamido)propanamido)-2-oxo-4-((*S*)-2-oxopyrrolidin-3-yl)butanamid;
3-((*S*)-2-((*E*)-3-(4-Chlor-2-fluorphenyl)acrylamido)-3-cyclobutylpropanamido)-*N-*cyclopropyl-2-oxo-4-((*S*)-2-oxopyrrolidin-3-yl)butanamid;
(3*S*)-*N*-((2*S*)-3-Cyclopropyl-1-((4-(cyclopropylamino)-3,4-dioxo-1-((*S*)-2-oxopyrrolidin-3-yl)butan-2-yl)amino)-1-oxopropan-2-yl)-3-phenylpentanamid;
(3*R*)-*N*-((25)-3-Cyclopropyl-1-((4-(cyclopropylamino)-3,4-dioxo-1-((*S*)-2-oxopyrrolidin-3-yl)butan-2-yl)amino)-1-oxopropan-2-yl)-3-phenylpentanamid;
(2*S*)-*N*-(4-(Azetidin-1-yl)-3,4-dioxo-1-((*S*)-2-oxopyrrolidin-3-yl)butan-2-yl)-2-((*E*)-3-(2,4-dichlorphenyl)acrylamido)-4,4-dimethylpentanamid;
(2*S*)-*N*-(4-(Cyclopropylamino)-3,4-dioxo-1-((*S*)-2-oxopyrrolidin-3-yl)butan-2-yl)-2-((*E*)-3-(2,4-difluorphenyl)acrylamido)-4,4-dimethylpentanamid;
(2*S*)-2-((*E*)-3-(4-Chlorphenyl)acrylamido)-*N*-(4-(cyclopropylamino)-3,4-dioxo-1-((*S*)-2-oxopyrrolidin-3-yl)butan-2-yl)-4,4-dimethylpentanamid;
(2*S*)-2-((*E*)-3-(2-Chlor-4-fluorphenyl)acrylamido)-*N*-(4-(cyclopropylamino)-3,4-dioxo-1-((*S*)-2-oxopyrrolidin-3-yl)butan-2-yl)-4,4-dimethylpentanamid;
(2*S*)-*N*-(4-(Cyclopropylamino)-3,4-di oxo-1-((*S*)-2-oxopyrrolidin-3-yl)butan-2-yl)-2-((*E*)-3-(4-fluorphenyl)acrylamido)-4,4-dimethylpentanamid;
(2*S*)-2-((*E*)-3-(4-Chlor-3-fluorphenyl)acrylamido)-*N*-(4-(cyclopropylamino)-3,4-dioxo-1-((*S*)-2-oxopyrrolidin-3-yl)butan-2-yl)-4,4-dimethylpentanamid;
(2*S*)-2-((*E*)-3-(5-Chlorpyridin-2-yl)acrylamido)-*N*-(4-(cyclopropylamino)-3,4-dioxo-1-((*S*)-2-oxopyrrolidin-3-yl)butan-2-yl)-4,4-dimethylpentanamid;
(2*S*)-*N*-(4-(Cyclopropylamino)-3,4-dioxo-1-((*S*)-2-oxopyrrolidin-3-yl)butan-2-yl)-2-((*E*)-3-(5-fluorpyridin-2-yl)acrylamido)-4,4-dimethylpentanamid;
(2*S*)-*N*-(4-(Cyclopropylamino)-3,4-dioxo-1-((*S*)-2-oxopyrrolidin-3-yl)butan-2-yl)-2-((*E*)-3-(4-(difluormethyl)phenyl)acrylamido)-4,4-dimethylpentanamid;
(1*R*,2*R*)-*N*-((2*S*)-1-((4-(Cyclopropylamino)-3,4-dioxo-1-((*S*)-2-oxopyrrolidin-3-yl)butan-2-yl)amino)-4,4-dimethyl-1-oxopentan-2-yl)-2-phenylcyclopropan-1-carboxamid;
(1*S*,2*S*)-*N*-((2*S*)-1-((4-(Cyclopropylamino)-3,4-dioxo-1-((*S*)-2-oxopyrrolidin-3-yl)butan-2-yl)amino)-4,4-dimethyl-1-oxopentan-2-yl)-2-phenylcyclopropan-1-carboxamid;
(2*S*)-*N*-(4-(Cyclopropylamino)-3,4-dioxo-1-((*S*)-2-oxopyrrolidin-3-yl)butan-2-yl)-2-((*S*)-3-(2,4-difluorphenyl)pentanamido)-4,4-dimethylpentanamid;
(2*S*)-*N*-(4-(Cyclopropylamino)-3,4-dioxo-1-((*S*)-2-oxopyrrolidin-3-yl)butan-2-yl)-2-((*R*)-3-(2,4-difluorphenyl)pentanamido)-4,4-dimethylpentanamid;
(2*S*)-*N*-(4-(Cyclopropylamino)-3,4-dioxo-1-((*S*)-2-oxopyrrolidin-3-yl)butan-2-yl)-2-((*S*)-3-(2,4-dichlorphenyl)pentanamido)-4,4-dimethylpentanamid;
(2*S*)-*N*-(4-(Cyclopropylamino)-3,4-dioxo-1-((*S*)-2-oxopyrrolidin-3-yl)butan-2-yl)-2-((*R*)-3-(2,4-dichlorphenyl)pentanamido)-4,4-dimethylpentanamid;
(2*S*)-2-((*S*)-3-(4-Chlor-2-fluorphenyl)pentanamido)-*N*-(4-(cyclopropylamino)-3,4-dioxo-1-((*S*)-2-oxopyrrolidin-3-yl)butan-2-yl)-4,4-dimethylpentanamid,
(2*S*)-2-((*R*)-3-(4-Chlor-2-fluorphenyl)pentanamido)-*N*-(4-(cyclopropylamino)-3,4-dioxo-1-((*S*)-2-oxopyrrolidin-3-yl)butan-2-yl)-4,4-dimethylpentanamid;
(2*S*)-2-((*S*)-3-(4-Chlorphenyl)pentanamido)-*N*-(4-(cyclopropylamino)-3,4-dioxo-1-((*S*)-2-oxopyrrolidin-3-yl)butan-2-yl)-4,4-dimethylpentanamid;
(2*S*)-2-((*R*)-3-(4-Chlorphenyl)pentanamido)-*N*-(4-(cyclopropylamino)-3,4-dioxo-1-((*S*)-2-oxopyrrolidin-3-yl)butan-2-yl)-4,4-dimethylpentanamid;
(2*S*)-*N*-(4-(Cyclopropylamino)-3,4-dioxo-1-((*S*)-2-oxopyrrolidin-3-yl)butan-2-yl)-2-((*S*)-3-(4-fluorphenyl)pentanamido)-4,4-dimethylpentanamid;
(2*S*)-*N*-(4-(Cyclopropylamino)-3,4-dioxo-1-((*S*)-2-oxopyrrolidin-3-yl)butan-2-yl)-2-((*R*)-3-(4-fluorphenyl)pentanamido)-4,4-dimethylpentanamid;
(2*S*)-*N*-(4-(Azetidin-1-yl)-3,4-dioxo-1-((*S*)-2-oxopyrrolidin-3-yl)butan-2-yl)-2-((*E*)-3-(4-chlor-2-fluorphenyl)acrylamido)-4,4-dimethylpentanamid;
(2*S*)-*N*-(4-(Cyclopropylamino)-3,4-dioxo-1-((*S*)-2-oxopyrrolidin-3-yl)butan-2-yl)-4,4-dimethyl-2-((*R*)-3-(2-(trifluormethoxy)phenyl)pentanamido)pentanamid;
(2*S*)-*N*-(4-(Cyclopropylamino)-3,4-dioxo-1-((*S*)-2-oxopyrrolidin-3-yl)butan-2-yl)-4,4-dimethyl-2-((*S*)-3-(2-(trifluonnethoxy)phenyl)pentanamido)pentanamid;
(2*S*)-*N*-(4-(Cyclopropylamino)-3,4-dioxo-1-((*S*)-2-oxopyrrolidin-3-yl)butan-2-yl)-2-((*R*)-3-(2-(difluormethoxy)phenyl)pentanamido)-4,4-dimethylpentanamid;
(2*S*)-*N*-(4-(Cyclopropylamino)-3,4-dioxo-1-((*S*)-2-oxopyrrolidin-3-yl)butan-2-yl)-2-((*S*)-3-(2-(difluormethoxy)phenyl)pentanamido)-4,4-dimethylpentanamid;
(2*S*)-2-((*E*)-3-(4-Chlor-2-cyanophenyl)acrylamido)-*N*-(4-(cyclopropylamino)-3,4-dioxo-1-((*S*)-2-oxopyrrolidin-3-yl)butan-2-yl)-4,4-dimethylpentanamid;
(2*S*)-2-((*E*)-3-(4-Chlor-3-cyanophenyl)acrylamido)-*N*-(4-(cyclopropylamino)-3,4-dioxo-1-((*S*)-2-oxopyrrolidin-3-yl)butan-2-yl)-4,4-dimethylpentanamid;
(2*S*)-2-((*E*)-3-(2-Chlor-4-cyanophenyl)acrylamido)-*N*-(4-(cyclopropylamino)-3,4-dioxo-1-((*S*)-2-oxopyrrolidin-3-yl)butan-2-yl)-4,4-dimethylpentanamid;
(2*S*)-*N*-(4-(Cyc1opropylamino)-3,4-dioxo-1-((*S*)-2-oxopyrrolidin-3-yl)butan-2-yl)-4,4-dimethyl-2-((*R*)-3-(3-(trifluormethoxy)phenyl)butanamido)pentanamid;
(2*S*)-*N*-(4-(Cyclopropylamino)-3,4-dioxo-1-((*S*)-2-oxopyrrolidin-3-yl)butan-2-yl)-4,4-dimethyl-2-((*S*)-3-(3-(trifluonnethoxy)phenyl)butanamido)pentanamid;
(2*S*)-*N*-(4-(Cyclopropylamino)-3,4-dioxo-1-((*S*)-2-oxopyrrolidin-3-yl)butan-2-yl)-2-((*R*)-3-(3-(difluormethoxy)phenyl)butanamido)-4,4-dimethylpentanamid;
(2*S*)-*N*-(4-(Cyclopropylamino)-3,4-dioxo-1-((*S*)-2-oxopyrrolidin-3-yl)butan-2-yl)-2-((*S*)-3-(3-(difluormethoxy)phenyl)butanamido)-4,4-dimethylpentanamid;
(2*S*)-2-((*R*)-3-(2-Chlor-4-(methylsulfonyl)phenyl)pentanamido)-*N*-(4-(cyclopropylamino)-3,4-dioxo-1-((*S*)-2-oxopyrrolidin-3-yl)butan-2-yl)-4,4-dimethylpentanamid;
(2*S*)-2-((*S*)-3-(2-Chlor-4-(methylsulfonyl)phenyl)pentanamido)-*N*-(4-(cyclopropylamino)-3,4-dioxo-1-((*S*)-2-oxopyrrolidin-3-yl)butan-2-yl)-4,4-dimethylpentanamid;
3-Chlor-4-((3*R*)-1-(((2*S*)-1-((4-(cyclopropylamino)-3,4-dioxo-1-((*S*)-2-oxopyrrolidin-3- yl)butan-2-yl)amino)-4,4-dimethyl-1-oxopentan-2-yl)amino)-1-oxopentan-3-yl)phenylmethansulfonat;
3-Chlor-4-((3*S*)-1-(((2*S*)-1-((4-(cyclopropylamino)-3,4-dioxo-1-((*S*)-2-oxopyrrolidin-3- yl)butan-2-yl)amino)-4,4-dimethyl-1-oxopentan-2-yl)amino)-1-oxopentan-3-yl)phenylmethansulfonat;
(2*S*)-2-((*R*)-3-(2-Chlor-4-(dimethylphosphoryl)phenyl)pentanamido)-*N*-(4-(cyclopropylamino)-3,4-dioxo-1-((*S*)-2-oxopyrrolidin-3-yl)butan-2-yl)-4,4-dimethylpentanamid;
(2*S*)-2-((*S*)-3-(2-Chlor-4-(dimethylphosphoryl)phenyl)pentanamido)-*N*-(4-(cyclopropylamino)-3,4-dioxo-1-((*S*)-2-oxopyrrolidin-3-yl)butan-2-yl)-4,4-dimethylpentanamid;
(2*S*)-*N*-(4-(Cyclopropylamino)-3,4-dioxo-1-((S)-2-oxopyrrolidin-3-yl)butan-2-yl)-4,4-dimethyl-2-((*E*)-3-(4-(pentafluor-I6-sulfanyl)phenyl)acrylamido)pentanamid,
(2*S*)-*N*-(4-(Cyclopropylamino)-3,4-dioxo-1-((*S*)-2-oxopyrrolidin-3-yl)butan-2-yl)-2-((*R*)-3-(4-methoxyphenyl)pentanamido)-4,4-dimethylpentanamid;
(2*S*)-*N*-(4-(Cyclopropylamino)-3,4-dioxo-1-((*S*)-2-oxopyrrolidin-3-yl)butan-2-yl)-2-((*S*)-3-(4-methoxyphenyl)pentanamido)-4,4-dimethylpentanamid;
(2*S*)-2-(3-(4-Chlor-2-(tiifluormethoxy)phenyl)propanamido)-*N*-(4-(cyclopropylamino)-3,4-dioxo-1-((*S*)-2-oxopyrrolidin-3-yl)butan-2-yl)-4,4-dimethylpentanamid;
(2*S*)-2-(3-(4-Chlor-2-(difluormethoxy)phenyl)propanamido)-*N*-(4-(cyclopropylamino)-3,4-dioxo-1-((*S*)-2-oxopyrrolidin-3-yl)butan-2-yl)-4,4-dimethylpentanamid;
(2*S*)-2-(3-(4-Chlor-2-cyanophenyl)propanamido)-*N*-(4-(cyclopropylamino)-3,4-dioxo-1-((*S*)-2-oxopyrrolidin-3-yl)butan-2-yl)-4,4-dimethylpentanamid;
(2*S*)-2-(3-(2-Chlor-4-cyanophenyl)propanamido)-*N*-(4-(cyclopropylamino)-3,4-dioxo-1-((*S*)-2-oxopyrrolidin-3-yl)butan-2-yl)-4,4-dimethylpentanamid;
(2*S*)-2-(3-(4-Chlor-3-(trifluormethoxy)phenyl)propanamido)-*N*-(4-(cyc1opropylamino)-3,4-dioxo-1-((*S*)-2-oxopyrrolidin-3-yl)butan-2-yl)-4,4-dimethylpentanamid;
(2*S*)-*N*-(1-Cyano-2-((*S*)-2-oxopyrrolidin-3-yl)ethyl)-2-(3-(2,4-dichlorphenyl)propanamido)-4,4-dimethylpentanamid;
2,4-Dichlorbenzyl-((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)carbamat;
4-Chlor-2-fluorbenzyl-((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)carbamat;
(*S*)-1-(4-Chlor-2-fluorphenyl)ethyl-((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)carbamat;
(*R*)-1-(4-Chlor-2-fluorphenyl)ethyl-((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)carbamat;
(2*S*)-*N*-(4-(Cyclopropylamino)-3,4-dioxo-1-((*S*)-2-oxopyrrolidin-3-yl)butan-2-yl)-2-((*R*)-3-(2,4-difluorphenyl)-4,4,4-trifluorbutanamido)-4,4-dimethylpentanamid;
(2*S*)-*N*-(4-(Cyclopropylamino)-3,4-dioxo-1-((*S*)-2-oxopyrrolidin-3-yl)butan-2-yl)-2-((*S*)-3-(2,4-difluorphenyl)-4,4,4-trifluorbutanamido)-4,4-dimethylpentanamid;
(2*S*)-2-((*R*)-2-Benzyl-3,3,3-trifluorpropanamido)-*N*-(4-(cyclopropylamino)-3,4-dioxo-1-((*S*)-2-oxopyrrolidin-3-yl)butan-2-yl)-4,4-dimethy-1-pentanamid;
(2*S*)-2-((*S*)-2-Benzyl-3,3,3-trifluorpropanamido)-*N*-(4-(cyclopropylamino)-3,4-dioxo-1-((*S*)-2-oxopyrrolidin-3-yl)butan-2-yl)-4,4-dimethylpentanamid;
(1*S*,2*S*)-2-(4-Chlorphenyl)-*N*-((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)cyclopropan-1-carboxamid;
(2*S*)-*N*-(4-(Cyclopropylamino)-3,4-dioxo-1-((*S*)-2-oxopyrrolidin-3-yl)butan-2-yl)-2-(2-(2,4-dichlorphenoxy)acetamido)-4,4-dimethylpentanamid;
(2*S*)-*N*-(4-(Cyclopropylamino)-3,4-dioxo-1-(*S*)-2-oxopyrrolidin-3-yl)butan-2-yl)-2-((*S*)-2-(2,4-dichlorphenoxy)propanamido)-4,4-dimethylpentanamid;
(2*S*)-*N*-(4-(Cyclopropylamino)-3,4-dioxo-1-((*S*)-2-oxopyrrolidin-3-yl)butan-2-yl)-2-((*R*)-2-(2,4-dichlorphenoxy)propanamido)-4,4-dimethylpentanamid;
oder ein Salz davon.

18. Pharmazeutische Zusammensetzung, die eine Verbindung, wie in einem der Ansprüche 1 bis 17 definiert, und einen pharmazeutisch verträglichen Hilfsstoff umfasst.

19. Verbindung oder Zusammensetzung nach einem der Ansprüche 1 bis 18 für die Verwendung bei der Behandlung von SARS-CoV-2 oder bei der Behandlung von Erkrankungen, die mit SARS-CoV-2: Mpro verbunden sind.

## Revendications

1. Composé de formule (1') : 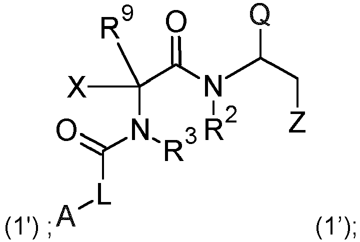 ou un de ses sels ;
A est sélectionné parmi 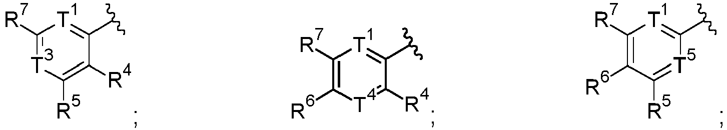 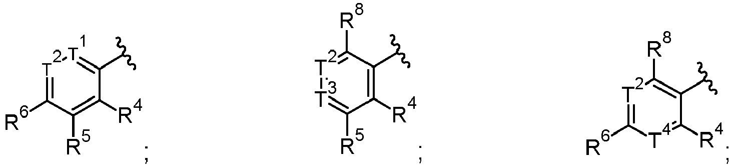
Q représente CN ou un groupe de la formule 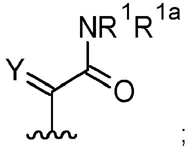
X représente un groupe hydrocarboné en C₁₋₆ saturé ou insaturé éventuellement substitué portant 1 à 6 atomes de fluor ou X est relié à R⁹ pour former un cycle cycloalkyle en C₃₋₆ qui est éventuellement substitué par 1 à 3 atomes de fluor ou 1 à 3 groupes alkyle en C₁₋₃ ;
Y représente O ou NOR¹⁶ ;
T¹ représente CR⁸ ou N ;
T² représente CR⁷ ou N ;
T³ est CR⁶ ou N ;
T⁴ représente CR⁵ ou N ;
T⁵ représente CR⁴ ou N ;
Z représente un cycle hétérocyclique à 5 ou 6 chaînons éventuellement substitué par oxo ou 1 à 6 atomes de fluor, ou Z représente un cycloalkyle en C₃₋₆ éventuellement substitué par 1 à 6 atomes de fluor, ou Z représente - (CH₂)pCONHR¹³ ou Z représente -(CH₂)ₚCO₂R¹³ ;
L représente -CR¹¹ = CR¹²-, -CHR¹¹-CHR¹²- ou -O-CHR¹¹⁻ ;
R¹ et R^{1a} représentent indépendamment H ou un groupe hydrocarboné saturé en C₁₋₆ éventuellement substitué par 1 à 6 atomes de fluor, ou R¹ et R^{1a} sont liés pour former un cycle saturé à 3 à 6 chaînons contenant éventuellement un hétéroatome supplémentaire ;
R² et R³ représentent indépendamment H ou un alkyle en C₁₋₃ éventuellement substitué par 1 à 6 atomes de fluor ;
R⁴, R⁵, R⁶, R⁷ et R⁸ représentent indépendamment H, halo, CN, CO₂R¹⁴, OR¹⁴, SO₂R¹⁴, SONHR¹⁴, OSO₂R¹⁴, PO(R¹⁴)₂, SF₅ ou un alkyle en C₁₋₃ éventuellement substitué par 1 à 6 atomes de fluor ;
R⁹ représente H ou est lié à X pour former un cycle cycloalkyle en C₃₋₆ qui est éventuellement substitué par 1 à 3 atomes de fluor ou 1 à 3 groupes alkyle en C1-3 ;
R¹¹ et R¹² représentent indépendamment H, -(CH₂)ₘCO₂R¹⁵ ou un groupe alkyle en C₁₋₃ éventuellement substitué par 1 à 6 atomes de fluor ; ou R¹¹ et R¹² sont liés pour former un cycle cyclopropyle ;
R¹³, R¹⁴ et R¹⁵ représentent indépendamment H ou un alkyle en C₁₋₃ éventuellement substitué par 1 à 6 atomes de fluor ;
R¹⁶ représente H ou un alkyle en C₁₋₃ éventuellement substitué par 1 à 6 atomes de fluor ;
p et m représentent indépendamment 0-3 ;
dans lequel, lorsque R¹ et R^{1a} ne représentent pas tous deux H, L correspond à :
-CHR¹¹-CHR¹²⁻ ;
-CR¹¹= CR¹²⁻ où R³, R⁴, R⁵, R⁶, R⁷ et R⁸ ne représentent pas tous H ;
ou -O-CHR¹¹- où R³ ne représente pas H, ou deux ou plus de R⁴, R⁵, R⁶, R⁷ et R⁸ ne représentent pas H.

2. Composé selon la revendication 1, qui est un composé de formule (1c) : 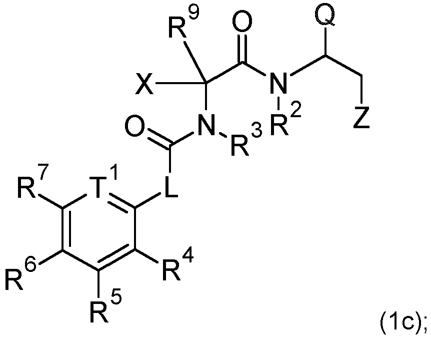 ou un de ses sels.

3. Composé selon la revendication 1, qui est un composé de formule (1b) : 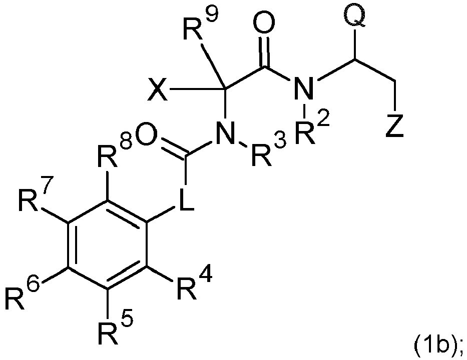 ou un de ses sels, dans lequel ;
Q représente CN ou un groupe de formule : 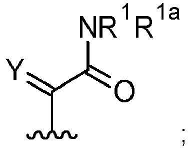
X représente un groupe hydrocarboné saturé ou insaturé en C₁₋₆ éventuellement substitué par 1 à 6 atomes de fluor ou X est lié à R⁹ pour former un cycle cycloalkyle en C₃₋₆ éventuellement substitué par 1 à 3 atomes de fluor ou 1 à 3 groupes alkyle en C₁₋₃ ;
Y représente O ou NOR¹⁶ ;
Z représente un cycle hétérocyclique à 5 ou 6 chaînons, éventuellement substitué par oxo ou par 1 à 6 atomes de fluor, ou Z représente un cycloalkyle en C₃₋₆, éventuellement substitué par 1 à 6 atomes de fluor, ou Z représente - (CH₂)ₚCONHR¹³, ou Z représente -(CH₂)ₚCO₂R¹³ ;
L représente -CR¹¹ CR^{12-,} -CHR¹¹⁻CHR¹²⁻ ou -O-CHR¹¹⁻;
R¹ et R^{1a} représentent indépendamment H ou un groupe hydrocarboné saturé en C₁₋₆ éventuellement substitué par 1 à 6 atomes de fluor, ou R¹ et R^{1a} sont liés conjointement pour former un cyclesaturé à 3 à 6 chaînons contenant éventuellement un hétéroatome supplémentaire ;
R² et R³ représentent indépendamment H ou un alkyle en C₁₋₃ éventuellement substitué par 1 à 6 atomes de fluor ;
R⁴, R⁵, R⁶, R⁷ et R⁸ représentent indépendamment H, halo, CO₂R¹⁴ ou alkyle en C₁₋₃ éventuellement substitué par 1 à 6 atomes de fluor ;
R⁹ représente H ou est lié à X pour former un cycle cycloalkyle en C₃₋₆, éventuellement substitué par 1 à 3 atomes de fluor ou 1 à 3 groupes alkyles en C₁₋₃;
R¹¹ et R¹² représentent indépendamment H, -(CH₂)ₘCO₂R¹⁵ ou un alkyle en C₁₋₃ éventuellement substitué par 1 à 6 atomes de fluor ;
R¹³, R¹⁴ et R¹⁵ représentent indépendamment H ou un alkyle en C₁₋₃ éventuellement substitué par 1 à 6 atomes de fluor ;
R¹⁶ représente H ou un alkyle en C₁₋₃ éventuellement substitué par 1 à 6 atomes de fluor ;
p et m représentent indépendamment 0 à 3 ;
où lorsque R¹ et R^{1a} ne représentent pas tous deux H, L signifie : - CHR¹¹-CHR¹²-;
-CR¹¹ = CR¹²- où R³, R⁴, R⁵, R⁶, R⁷ et R⁸ ne représentent pas tous H;
ou -O-CHR¹¹- où R³ ne représente pas H ou deux ou plusieurs parmi R⁴, R⁵, R⁶, R⁷ and R⁸ ne représentent pas H ;
préférablement lorsque
(a) Q est 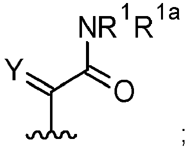 et
(b) R^{1a} est H.

4. Composé selon la revendication 1, qui est un composé de la formule (1ai) ou de la formule (1aii) : 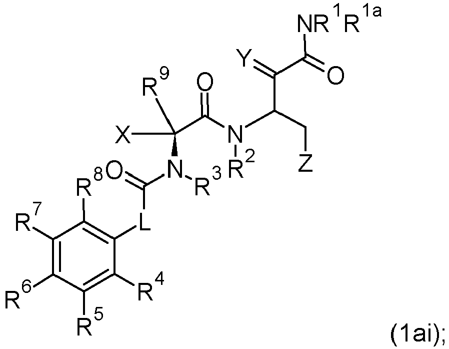 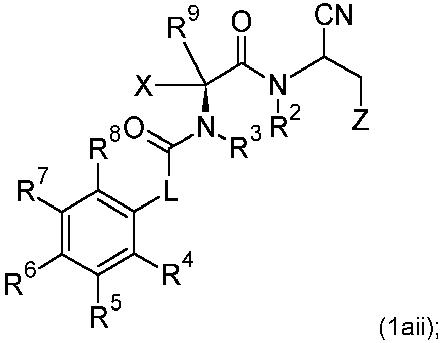 ou un de ses sels.

5. Composé selon l'une des revendications 1 à 4, dans lequel Y représente O.

6. Composé selon l'une des revendications 1 à 3, dans lequel R¹ et R^{1a} représentent indépendamment H ou sont sélectionnés dans le groupe consistant en : 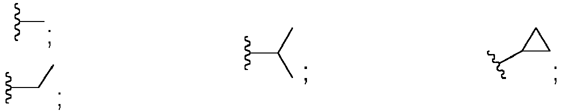 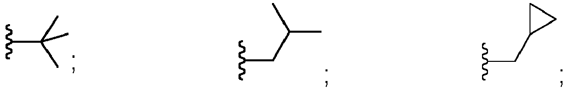 ou dans lequel R¹ et R^{1a} sont liés pour former un cycle de telle sorte que le groupe NR¹R^{1a} représente :
N

7. Composé selon l'une des revendications 1 à 6, dans lequel R² s'exprime en H.

8. Composé selon l'une des revendications 1 à 7, dans lequel X est sélectionné dans le groupe constitué de :

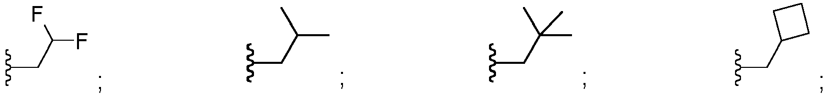

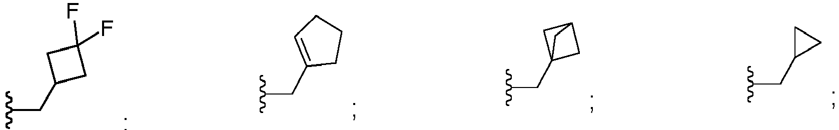

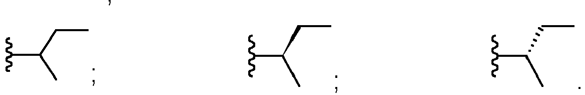

9. Composé selon la revendication 1, qui est un composé de formule (3bi), (3bii), (3ci) ou (3cii) : 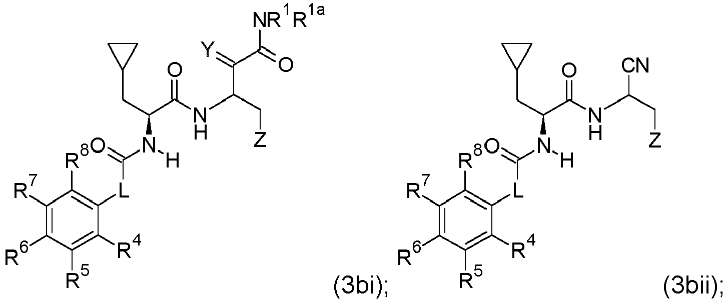 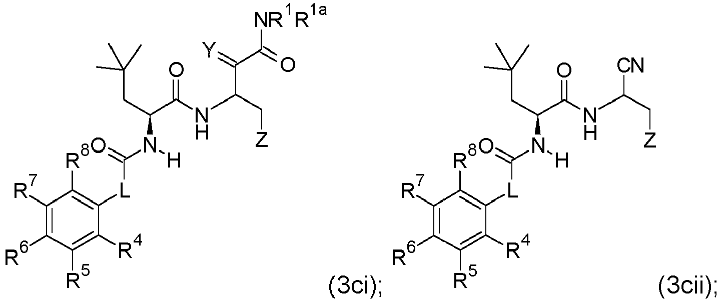 et leurs sels, dans lesquels
Y représente O ou NOH ;
Z représente un cycle hétérocyclique à 5 ou 6 chaînons, éventuellement substitué par oxo ou par 1 à 6 atomes de fluor, ou Z représente un cycloalkyle en C₃₋₆, éventuellement substitué par 1 à 6 atomes de fluor ;
L désigne -CH = CH-, -CH₂-CH₂- ou -O-CH₂-;
R¹ and R^{1a} représentent indépendamment H ou un groupe d'hydrocarboné en C₁₋₄ éventuellement substitué par 1 à 6 atomes de fluor, ou R¹ et R^{1a} sont liés conjointement pour former un cycle saturé à 3 à 6 chaînons contenant éventuellement un hétéroatome supplémentaire ;
R⁴, R⁵, R⁶, R⁷ et R⁸ représentent indépendamment H ou halo.

10. Composé selon l'une quelconque des revendications 1 à 9, dans lequel Z est sélectionné dans le groupe constitué de :

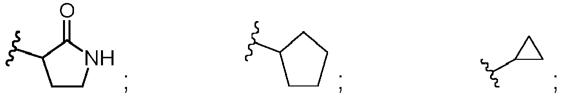

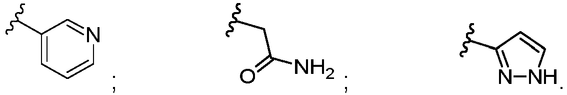

11. Composé selon la revendication 1, qui est un composé de formule (4ai) ou (4aii) : 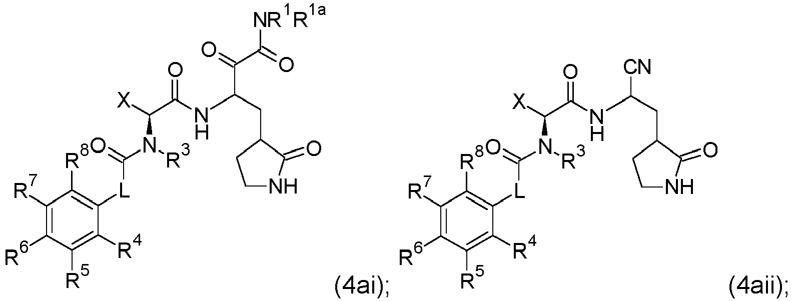 ou un de ses sels.

12. Composé selon la revendication 1, dans lequel
R¹ et R^{1a} représentent tous deux H et L représente -CR¹¹ = CR¹²- ou -O-CHR¹¹⁻; ou
R¹ et R^{1a} ne désignent pas tous deux H et
(i) L représente -CR¹¹ = CR¹²⁻ où R³, R⁴, R⁵, R⁶, R⁷ et R⁸ ne désignent pas tous H ou
(ii) L représente -O-CHR¹¹⁻ où R³ ne désigne pas H, ou deux ou plusieurs de R⁴, R⁵, R⁶, R⁷ et R⁸ ne représentent pas H.

13. Composé selon la revendication 1, dans lequel L représente -CH=CH-, - CH₂CH₂-, -CH₂-CH(CH₂CH₃)-, -CH₂-CH(CH₂CO₂H)-, -OCH₂-, -CH₂-CH(CH₃)-, -CH₂-CH(CF₃)-, -OCH(CH₃)-, ou

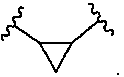

14. Composé selon l'une quelconque des revendications 1 à 13, dans lequel R³ désigne H, méthyle ou CH₂CF₃.

15. Composé selon la revendication 1, qui est un composé de formule (9b) : 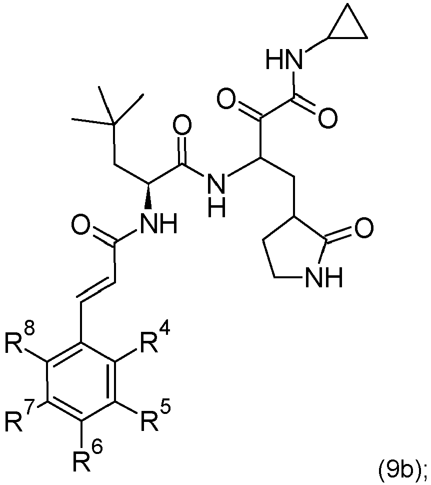 ou un de ses sels.

16. Composé selon l'une quelconque des revendications 1 à 15, dans lequel R⁴, R⁵, R⁶, R⁷ et R⁸ sont indépendamment sélectionnés parmi H, CN, Cl, F, CF₂H, OCH₃, OCF₃, OCF₂H, SO₂CH₃, OSO₂CH₃, PO(CH₃)₂, SF₅ et CO₂H; préférablement dans lequel R⁴ représente F, R⁵ représente H, R⁶ désigne Cl et R⁷ et R⁸ représentent H.

17. Composé selon la revendication 1 qui est sélectionné dans le groupe constitué de :
butanamide de 3-((*S*)-2-((*E*)-3-(4-chloro-2-fluorophényl)acrylamido)-3-cyclopropylpropanamido)-2-oxo-4-((*S*)-2-oxopyrrolidin-3-yl) ;
butanamide de 3-((*S*)-2-cinnamamido-3-cyclopropylpropanamido)-2-oxo-4-((*S*)-2-oxopyrrolidin-3-yl) ;
carbamate benzylique de ((2*S*)-1-((4-amino-3,4-dioxo-1-((*S*)-2-oxopyrrolidin-3-yl)butan-2-yl)amino)-3-cyclopropyl-1-oxopropan-2-yl) ;
butanam ide de 3-((*S*)-2-((*E*)-3-(4-chloro-2-fluorophényl)acrylamido)-3 cyclopropylpropanamido)-2-(hydroxyimino)-4-((*S*)-2-oxopyrrolidin-3-yl) ;
butanam ide *de N*-(*tert*-butyl)-3-((*S*)-2-((*E*)-3-(4-chloro-2-fluorophényl)acrylamido)-3-cyclopropylpropanamido)-2-oxo-4-((*S*)-2-oxopyrrolidin-3-yl) ;
oxobutanamide de 3-((*S*)-2-((*E*)-3-(4-chloro-2-fluorophényl)acrylamido)-3-cyclopropylpropanamido)-4-cyclopropyl-2- ;
oxobutanamide de 3-((*S*)-2-((*E*)-3-(4-chloro-2-fluorophenyl)acrylamido)-3-cyclopropylpropanamido)-4-cyclopentyl-2- ;
butanam ide de 3-((*S*)-3-cyclopropyl-2-(3-phénylpropanamido)propanamido)-2-oxo-4-((*S*)-2-oxopyrrolidin-3-yl) ;
diméthylpentanamide de (2*S*)-*N*-(1-cyano-2-((*S*)-2-oxopyrrolidin-3-yl)éthyl)-2-((*E*)-3-(2,4-dichlorophényl)acrylamido)-4,4 ;
diméthylpentanamide de (2*S*)-*N*-(4-(cyclopropylamino)-3,4-dioxo-1-((*S*)-2-oxopyrrolidin-3-yl)butan-2-yl)-2-((*E*)-3-(2,4-dichlorophényl)acrylamido)-4,4 ;
diméthylpentanamide de (2*S*)-2-((*E*)-3-(4-chloro-2-fluorophényl)acrylamido)-pentanamide de *N*-(4-(cyclopropylamino)-3,4-dioxo-1-((*S*)-2-oxopyrrolidin-3-yl)butan-2-yl)-4,4 ;
(pentanamide de 2*S*)-*N*-(4-(cyclopropylamino)-3,4-dioxo-1-((*S*)-2-oxopyrrolidin-3-yl)butan-2-yl)-4,4-diméthyl-2-((*S*)-3-phénylbutanamido);
pentanamide de (2*S*)-*N*-(4-(cyclopropylamino)-3,4-dioxo-1-((*S*)-2-oxopyrrolidin-3-yl)butan-2-yl)-4,4-diméthyl-2-((*R*)-3-phénylbutanamido) ;
pentanamide de (2*S*)-*N*-(4-(cyclopropylamino)-3,4-dioxo-1-((*S*)-2-oxopyrrolidin-3-yl)butan-2-yl)-4,4-diméthyl-2-((*S*)-3-phénylpentanamido) ;
pentanamide de (2*S*)-*N*-(4-(cyclopropylamino)-3,4-dioxo-1-((*S*)-2-oxopyrrolidin-3-yl)butan-2-yl)-4,4-diméthyl-2-((*R*)-3-phénylpentanamido) ;
pentanamide de (2*S*)-*N*-(4-(cyclopropylamino)-3,4-dioxo-1-((*S*)-2-oxopyrrolidin-3-yl)butan-2-yl)-4,4-diméthyl-2-((*S*)-4,4,4-trifluoro-3-phénylbutanamido) ;
pentanamide de (2*S*)-*N*-(4-(cyclopropylamino)-3,4-dioxo-1-((*S*)-2-oxopyrrolidin-3-yl)butan-2-yl)-4,4-diméthyl-2-((*R*)-4,4,4-trifluoro-3-phénylbutanamido) ;
méthylpentanamide de (2*S*)-*N*-(4-amino-3,4-dioxo-1-((*S*)-2-oxopyrrolidin-3-yl)butan-2-yl)-2-((*E*)-3-(4-chloro-2-fluorophényl)acrylamido)-4 ;
3-((*S*)-2-((*E*)-3-(4-chloro-2-fluorophényl)acrylamido)-3-butanamide de cyclopropylpropanamido)-*N*-éthyl-2-oxo-4-((*S*)-2-oxopyrrolidin-3-yl) ;
butanam ide de 3-((*S*)-3-cyclopropyl-2-((*E*)-3-(2,4-dichlorophényl)acrylamido)propanamido)-2-oxo-4-((*S*)-2-oxopyrrolidin-3-yl) ;
3-((*S*)-2-((*E*)-3-(4-chloro-2-fluorophényl)acrylamido)-3-cyclopropylpropanamido)-*N*-cyclopropyl-2-oxo-4-((*S*)-2-oxopyrrolidin-3-yl);
diméthylpentanamide de (2*S*)-*N*-(4-amino-3,4-dioxo-1-((*S*)-2-oxopyrrolidin-3-yl)butan-2-yl)-2-((*E*)-3-(4-chloro-2-fluorophényl)acrylamido)-4,4 ;
diméthylpentanamide de (2*S*)-2-((*E*)-3-(4-chloro-2-fluorophényl)acrylamido)-*N*-(4-(éthylamino)-3,4-dioxo-1-((*S*)-2-oxopyrrolidin-3-yl)butan-2-yl)-4,4 ;
butanamide de 3-((*S*)-3-cyclopropyl-2-((*E*)-3-(2,4-dichlorophényl)acrylamido)propanamido)-*N*-éthyl-2-oxo-4-((*S*)-2-oxopyrrolidin-3-yl) ;
butanamide *de N*-cyclopropyl-3-((*S*)-3-cyclopropyl-2-((*E*)-3-(2,4-dichlorophenyl)acrylamido)propanamido)-2-oxo-4-((*S*)-2-oxopyrrolidin-3-yl);
diméthylpentanamide de (2*S*)-2-((*E*)-3-(2,4-dichlorophényl)acrylamido)-*N*-(4-(éthylamino)-3,4-dioxo-1-((*S*)-2-oxopyrrolidin-3-yl)butan-2-yl)-4,4 ;
diméthylpentanamide de (2*S*)-*N*-(4-amino-3,4-dioxo-1-((*S*)-2-oxopyrrolidin-3-yl)butan-2-yl)-2-((*E*)-3-(2,4-dichlorophényl)acrylamido)-4,4 ;
butanamide de 3-((*S*)-2-((*E*)-3-(4-chloro-2-fluorophényl)acrylamido)-3-cyclopropylpropanamido)-*N*-cyclopropyl-2-oxo-4-((*S*)-2-oxopyrrolidin-3-yl) ;
carbamatebenzylique de ((2*S*)-3-cyclopropyl-1-((4-(cyclopropylamino)-3,4-dioxo-1-((*S*)-2-oxopyrrolidin-3-yl)butan-2-yl)amino)-1-oxopropan-2-yl) ;
butanamide *de N*-cyclopropyl-3-((S)-3-cyclopropyl-2-(3-(2,4-dichlorophényl)propanamido)propanamido)-2-oxo-4-((*S*)-2-oxopyrrolidin-3-yl) ;
butanamide de 3-((*S*)-2-((*E*)-3-(4-chloro-2-fluorophényl)acrylamido)-3-cyclobutylpropanamido)-*N*-cyclopropyl-2-oxo-4-((*S*)-2-oxopyrrolidin-3-yl) ;
phénylpentanamide de (3*S*)-*N*-((2S)-3-cyclopropyl-1-((4-(cyclopropylamino)-3,4-dioxo-1-((*S*)-2-oxopyrrolidin-3-yl)butan-2-yl)amino)-1-oxopropan-2-yl)-3 ;
phénylpentanamide de (3*R*)-*N*-((2*S*)-3-cyclopropyl-1-((4-(cyclopropylamino)-3,4-dioxo-1-((*S*)-2-oxopyrrolidin-3-yl)butan-2-yl)amino)-1-oxopropan-2-yl)-3 ;
(diméthylpentanamide de 2*S*)-*N*-(4-(azétidin-1-yl)-3,4-dioxo-1-((*S*)-2-oxopyrrolidin-3-yl)butan-2-yl)-2-((*E*)-3-(2,4-dichlorophényl)acrylamido)-4,4 ;
diméthylpentanamide de (2*S*)-*N*-(4-(cyclopropylamino)-3,4-dioxo-1-((*S*)-2-oxopyrrolidin-3-yl)butan-2-yl)-2-((*E*)-3-(2,4-difluorophényl)acrylamido)-4,4 ;
diméthylpentanamide de (2*S*)-2-((*E*)-3-(4-chlorophényl)acrylamido)-*N*-(4-(cyclopropylamino)-3,4-dioxo-1-((*S*)-2-oxopyrrolidin-3-yl)butan-2-yl)-4,4 ;
diméthylpentanamide de (2*S*)-2-((*E*)-3-(2-chloro-4-fluorophényl)acrylamido)-*N*-(4-(cyclopropylamino)-3,4-dioxo-1-((*S*)-2-oxopyrrolidin-3-yl)butan-2-yl)-4,4 ;
diméthylpentanamide de (2*S*)-*N*-(4-(cyclopropylamino)-3,4-dioxo-1-((*S*)-2-oxopyrrolidin-3-yl)butan-2-yl)-2-((*E*)-3-(4-fluorophényl)acrylamido)-4,4 ;
diméthylpentanamide de (2*S*)-2-((*E*)-3-(4-chloro-3-fluorophényl)acrylamido)-*N*-(4-(cyclopropylamino)-3,4-dioxo-1-((*S*)-2-oxopyrrolidin-3-yl)butan-2-yl)-4,4 ;
diméthylpentanamide de (2*S*)-2-((*E*)-3-(5-chloropyridin-2-yl)acrylamido)-*N*-(4-(cyclopropylamino)-3,4-dioxo-1-((*S*)-2-oxopyrrolidin-3-yl)butan-2-yl)-4,4 ;
diméthylpentanamide de (2*S*)-*N*-(4-(cyclopropylamino)-3,4-dioxo-1-((*S*)-2-oxopyrrolidin-3-yl)butan-2-yl)-2-((*E*)-3-(5-fluoropyridin-2-yl)acrylamido)-4,4 ;
diméthylpentanamide de (2*S*)-*N*-(4-(cyclopropylamino)-3,4-dioxo-1-((*S*)-2-oxopyrrolidin-3-yl)butan-2-yl)-2-((*E*)-3-(4-(difluorométhyl)phényl)acrylamido)-4,4 ;
carboxamide de (1*R*,2*R*)-*N*-((2*S*)-1-((4-(cyclopropylamino)-3,4-dioxo-1-((*S*)-2-oxopyrrolidin-3-yl)butan-2-yl)amino)-4,4-diméthyl-1-oxopentan-2-yl)-2-phénylcyclopropane-1 ;
carboxamide de (1*S*,2*S*)-*N*-((2S)-1-((4-(cyclopropylamino)-3,4-dioxo-1-((*S*)-2-oxopyrrolidin-3-yl)butan-2-yl)amino)-4,4-diméthyl-1-oxopentan-2-yl)-2-phénylcyclopropane-1 ;
diméthylpentanamide de (2*S*)-*N*-(4-(cyclopropylamino)-3,4-dioxo-1-((*S*)-2-oxopyrrolidin-3-yl)butan-2-yl)-2-((*S*)-3-(2,4-difluorophényl)pentanamido)-4,4 ;
diméthylpentanamide de (2*S*)-*N*-(4-(cyclopropylamino)-3,4-dioxo-1-((*S*)-2-oxopyrrolidin-3-yl)butan-2-yl)-2-((*R*)-3-(2,4-difluorophényl)pentanamido)-4,4 ;
diméthylpentanamide de (2*S*)-*N*-(4-(cyclopropylamino)-3,4-dioxo-1-((*S*)-2-oxopyrrolidin-3-yl)butan-2-yl)-2-((*S*)-3-(2,4-dichlorophényl)pentanamido)-4,4 ;
diméthylpentanamide de (2*S*)-*N*-(4-(cyclopropylamino)-3,4-dioxo-1-((*S*)-2-oxopyrrolidin-3-yl)butan-2-yl)-2-((*R*)-3-(2,4-dichlorophényl)pentanamido)-4,4 ;
diméthylpentanamide de (2S)-2-((*S*)-3-(4-chloro-2-fluorophenyl)pentanamido)-*N*-(4-(cyclopropylamino)-3,4-dioxo-1-((*S*)-2-oxopyrrolidin-3-yl)butan-2-yl)-4,4 ;
diméthylpentanamide de (2*S*)-2-((*R*)-3-(4-chloro-2-fluorophényl)pentanamido)-*N*-(4-(cyclopropylamino)-3,4-dioxo-1-((*S*)-2-oxopyrrolidin-3-yl)butan-2-yl)-4,4 ;
diméthylpentanamide de (2*S*)-2-((*S*)-3-(4-chlorophényl)pentanamido)-*N*-(4-(cyclopropylamino)-3,4-dioxo-1-((*S*)-2-oxopyrrolidin-3-yl)butan-2-yl)-4,4 ;
diméthylpentanamide de (2*S*)-2-((*R*)-3-(4-chlorophényl)pentanamido)-*N*-(4-(cyclopropylamino)-3,4-dioxo-1-((*S*)-2-oxopyrrolidin-3-yl)butan-2-yl)-4,4 ;
diméthylpentanamide de (2*S*)-*N*-(4-(cyclopropylamino)-3,4-dioxo-1-((*S*)-2-oxopyrrolidin-3-yl)butan-2-yl)-2-((*S*)-3-(4-fluorophényl)pentanamido)-4,4 ;
diméthylpentanamide de (2*S*)-*N*-(4-(cyclopropylamino)-3,4-dioxo-1-((*S*)-2-oxopyrrolidin-3-yl)butan-2-yl)-2-((*R*)-3-(4-fluorophényl)pentanamido)-4,4 ;
diméthylpentanamide de (2*S*)-*N*-(4-(azétidin-1-yl)-3,4-dioxo-1-((*S*)-2-oxopyrrolidin-3-yl)butan-2-yl)-2-((*E*)-3-(4-chloro-2-fluorophényl)acrylamido)-4,4 ;
pentanamide de (2*S*)-*N*-(4-(cyclopropylamino)-3,4-dioxo-1-((*S*)-2-oxopyrrolidin-3-yl)butan-2-yl)-4,4-diméthyl-2-((*R*)-3-(2-(trifluorométhoxy)phényl)pentanamido);
pentanamide de (2*S*)-*N*-(4-(cyclopropylamino)-3,4-dioxo-1-((*S*)-2-oxopyrrolidin-3-yl)butan-2-yl)-4,4-diméthyl-2-((*S*)-3-(2-(trifluorométhoxy)phényl)pentanamido) ;
diméthylpentanamide de (2*S*)-*N*-(4-(cyclopropylamino)-3,4-dioxo-1-((*S*)-2-oxopyrrolidin-3-yl)butan-2-yl)-2-((*R*)-3-(2-(difluorométhoxy)phényl)pentanamido)-4,4 ;
diméthylpentanamide de (2*S*)-*N*-(4-(cyclopropylamino)-3,4-dioxo-1-((*S*)-2-oxopyrrolidin-3-yl)butan-2-yl)-2-((*S*)-3-(2-(difluorométhoxy)phényl)pentanamido)-4,4 ;
diméthylpentanamide de (2*S*)-2-((*E*)-3-(4-chloro-2-cyanophényl)acrylamido)-*N*-(4-(cyclopropylamino)-3,4-dioxo-1-((*S*)-2-oxopyrrolidin-3-yl)butan-2-yl)-4,4 ;
diméthylpentanamide de (2*S*)-2-((*E*)-3-(4-chloro-3-cyanophényl)acrylamido)-*N*-(4-(cyclopropylamino)-3,4-dioxo-1-((*S*)-2-oxopyrrolidin-3-yl)butan-2-yl)-4,4 ;
diméthylpentanamide de (2*S*)-2-((*E*)-3-(2-chloro-4-cyanophényl)acrylamido)-*N*-(4-(cyclopropylamino)-3,4-dioxo-1-((*S*)-2-oxopyrrolidin-3-yl)butan-2-yl)-4,4 ;
pentanamide de (2*S*)-*N*-(4-(cyclopropylamino)-3,4-dioxo-1-((*S*)-2-oxopyrrolidin-3-yl)butan-2-yl)-4,4-diméthyl-2-((*R*)-3-(3-(trifluorométhoxy)phényl)butanamido) ;
pentanamide de (2*S*)-*N*-(4-(cyclopropylamino)-3,4-dioxo-1-((*S*)-2-oxopyrrolidin-3-yl)butan-2-yl)-4,4-diméthyl-2-((*S*)-3-(3-(trifluorométhoxy)phényl)butanamido) ;
diméthylpentanamide de (2*S*)-*N*-(4-(cyclopropylamino)-3,4-dioxo-1-((*S*)-2-oxopyrrolidin-3-yl)butan-2-yl)-2-((*R*)-3-((difluorométhoxy)phényl)butanamido)-4,4 ;
diméthylpentanamide de (2*S*)-*N*-(4-(cyclopropylamino)-3,4-dioxo-1-((*S*)-2-oxopyrrolidin-3-yl)butan-2-yl)-2-((*S*)-3-)2-diméthylpentanamide de -((*S*)-3-(3-(difluorométhoxy)phényl)butanamido)-4,4 ;
(2*S*)-2-((*R*)-3-(2-chloro-4-(methylsulfonyl)phényl)pentanamido)-*N*-(4-(cyclopropylamino)-3,4-dioxo-1-((*S*)-2-oxopyrrolidin-3-yl)butan-2-yl)-4,4 ;
diméthylpentanamide de (2*S*)-2-((*S*)-3-(2-chloro-4-(méthylsulfonyl)phényl)pentanamido)-*N*-(4-(cyclopropylamino)-3,4-dioxo-1-((*S*)-2-oxopyrrolidin-3-yl)butan-2-yl)-4,4 ;
méthanesulfonate de 3-chloro-4-((3*R*)-1-(((2*S*)-1-((4-(cyclopropylamino)-3,4-dioxo-1-((*S*)-2-oxopyrrolidin-3-yl)butan-2-yl)amino)-4,4-dimethyl-1-oxopentan-2-yl)amino)-1-oxopentan-3-yl)phényle ;
méthanesulfonate de 3-chloro-4-((3*S*)-1-(((2*S*)-1-((4-(cyclopropylamino)-3,4-dioxo-1-((*S*)-2-oxopyrrolidin-3-yl)butan-2-yl)amino)-4,4-diméthyl-1-oxopentan-2-yl)amino)-1-oxopentan-3-yl)phényle ;
diméthylpentanamide de (2*S*)-2-((*R*)-3-(2-chloro-4-(diméthylphosphoryl)phényl)pentanamido)-*N*-(4-(cyclopropylamino)-3,4-dioxo-1-((*S*)-2-oxopyrrolidin-3-yl)butan-2-yl)-4,4 ;
diméthylpentanamide de (2*S*)-2-((*S*)-3-(2-chloro-4-(diméthylphosphoryl)phényl)pentanamido)-*N*-(4-(cyclopropylamino)-3,4-dioxo-1-((*S*)-2-oxopyrrolidin-3-yl)butan-2-yl)-4,4 ;
pentanamide de (2*S*)-*N*-(4-(cyclopropylamino)-3,4-dioxo-1-((*S*)-2-oxopyrrolidin-3-yl)butan-2-yl)-4,4-diméthyl-2-((*E*)-3-(4-(pentafluoro-l6-sulfaneyl)phényl)acrylamido) ;
diméthylpentanamide de (2*S*)-*N*-(4-(cyclopropylamino)-3,4-dioxo-1-((*S*)-2-oxopyrrolidin-3-yl)butan-2-yl)-2-((*R*)-3-(4-méthoxyphényl)pentanamido)-4,4 ;
diméthylpentanamide de (2*S*)-*N*-(4-(cyclopropylamino)-3,4-dioxo-1-((*S*)-2-oxopyrrolidin-3-yl)butan-2-yl)-2-((*S*)-3-(4-méthoxyphényl)pentanamido)-4,4 ;
diméthylpentanamide de (2*S*)-2-(3-(4-chloro-2-(trifluorométhoxy)phényl)propanamido)-*N*-(4-(cyclopropylamino)-3,4-dioxo-1-((*S*)-2-oxopyrrolidin-3-yl)butan-2-yl)-4,4 ;
diméthylpentanamide de (2*S*)-2-(3-(4-chloro-2-(difluorométhoxy)phényl)propanamido)-*N*-(4-(cyclopropylamino)-3,4-dioxo-1-((*S*)-2-oxopyrrolidin-3-yl)butan-2-yl)-4,4 ;
diméthylpentanamidede(2*S*)-2-(3-(4-chloro-2-cyanophényl)propanamido)-*N-*(4-(cyclopropylamino)-3,4-dioxo-1-((*S*)-2-oxopyrrolidin-3-yl)butan-2-yl)-4,4-diméthylpentanamide;
diméthylpentanamide de (2*S*)-2-(3-(2-chloro-4-cyanophényl)propanamido)-*N-*(4-(cyclopropylamino)-3,4-dioxo-1-((*S*)-2-oxopyrrolidin-3-yl)butan-2-yl)-4,4 ;
diméthylpentanamide de (2*S*)-2-(3-(4-chloro-3-(trifluorométhoxy)phényl)propanamido)-*N*-(4-(cyclopropylamino)-3,4-dioxo-1-((*S*)-2-oxopyrrolidin-3-yl)butan-2-yl)-4,4 ;
diméthylpentanamide de (2*S*)-*N*-(1-cyano-2-((*S*)-2-oxopyrrolidin-3-yl)éthyl)-2-(3-(2,4-dichlorophényl)propanamido)-4,4 ;
carbamate de 2,4-dichlorobenzyl ((2*S*)-1-((4-(cyclopropylamino)-3,4-dioxo-1-((*S*)-2-oxopyrrolidin-3-yl)butan-2-yl)amino)-4,4-diméthyl-1-oxopentan-2-yl) ;
carbamate de 4-chloro-2-fluorobenzyl ((2*S*)-1-((4-(cyclopropylamino)-3,4-dioxo-1-((*S*)-2-oxopyrrolidin-3-yl)butan-2-yl)amino)-4,4-diméthyl-1-oxopentan-2-yl) ;
carbamate de (*S*)-1-(4-chloro-2-fluorophényl)éthyl ((2*S*)-1-((4-(cyclopropylamino)-3,4-dioxo-1-((*S*)-2-oxopyrrolidin-3-yl)butan-2-yl)amino)-4,4-diméthyl-1-oxopentan-2-yl) ;
carbamate de (*R*)-1-(4-chloro-2-fluorophényl)éthyl ((2*S*)-1-((4-(cyclopropylamino)-3,4-dioxo-1-((*S*)-2-oxopyrrolidin-3-yl)butan-2-yl)amino)-4,4-diméthyl-1-oxopentan-2-yl) ;
diméthylpentanamide de (2*S*)-*N*-(4-(cyclopropylamino)-3,4-dioxo-1-((*S*)-2-oxopyrrolidin-3-yl)butan-2-yl)-2-((*R*)-3-(2,4-difluorophényl)-4,4,4-trifluorobutanamido)-4,4 ;
(diméthylpentanamide de 2*S*)-*N*-(4-(cyclopropylamino)-3,4-dioxo-1-((*S*)-2-oxopyrrolidin-3-yl)butan-2-yl)-2-((*S*)-3-(2,4-difluorophényl)-4,4,4-trifluorobutanamido)-4,4 ;
diméthylpentanamide de (2*S*)-2-((*R*)-2-benzyl-3,3,3-trifluoropropanamido)-*N-*(4-(cyclopropylamino)-3,4-dioxo-1-((*S*)-2-oxopyrrolidin-3-yl)butan-2-yl)-4,4 ;
diméthylpentanamide de 2*S*)-2-((*S*)-2-benzyl-3,3,3-trifluoropropanamido)-*N-*(4-(cyclopropylamino)-3,4-dioxo-1-((*S*)-2-oxopyrrolidin-3-yl)butan-2-yl)-4,4 ;
carboxamide de (1*S*,2*S*)-2-(4-chlorophényl)-*N*-((2*S*)-1-((4-(cyclopropylamino)-3,4-dioxo-1-((*S*)-2-oxopyrrolidin-3-yl)butan-2-yl)amino)-4,4-diméthyl-1- oxopentan-2-yl)cyclopropane-1 ;
diméthylpentanamide de (2*S*)-*N*-(4-(cyclopropylamino)-3,4-dioxo-1-((*S*)-2-oxopyrrolidin-3-yl)butan-2-yl)-2-(2-(2,4-dichlorophenoxy)acétamido)-4,4 ;
diméthylpentanamide de (2*S*)-*N*-(4-(cyclopropylamino)-3,4-dioxo-1-((*S*)-2-oxopyrrolidin-3-yl)butan-2-yl)-2-((*S*)-2-(2,4-dichlorophénoxy)propanam ido)-4,4
diméthylpentanamide de (2*S*)-*N*-(4-(cyclopropylamino)-3,4-dioxo-1-((*S*)-2-oxopyrrolidin-3-yl)butan-2-yl)-2-((*R*)-2-(2,4-dichlorophénoxy)propanamido)-4,4
ou un de leur sels.

18. Composition pharmaceutique comprenant un composé tel que défini dans l'une quelconque des revendications 1 à 17 et un excipient pharmaceutiquement acceptable.

19. Composé ou composition selon l'une des revendications 1 à 18 pour utilisation dans le traitement du SARS-CoV-2 ou dans le traitement des troubles associés au SARS-CoV-2 : Mpro.
